(19) **Europäisches Patentamt**
**European Patent Office**
**Office européen des brevets**

(11) **EP 2 393 514 B1**

(12) **EUROPEAN PATENT SPECIFICATION**

(45) Date of publication and mention
of the grant of the patent:
**20.12.2017  Bulletin 2017/51**

(51) Int Cl.:
*A61K 39/395* (2006.01)     *C07H 21/04* (2006.01)
*C12N 15/113* (2010.01)

(21) Application number: **10739211.0**

(86) International application number:
**PCT/US2010/023426**

(22) Date of filing: **06.02.2010**

(87) International publication number:
**WO 2010/091324 (12.08.2010 Gazette 2010/32)**

(54) **DUAL INHIBITION OF IMMUNOPHILIN/CYCLOPHILIN AND EMMPRIN IMMUNOGLOBULIN RECEPTOR SUPERFAMILY MEMBERS**

IMMUNOPHILIN/CYCLOPHILIN-DOPPELHEMMUNG UND MITGLIEDER DER EMMPRIN-IMMUNOGLOBULIN-REZEPTORSUPERFAMILIE

DOUBLE INHIBITION DE MEMBRES DE LA FAMILLE DES IMMUNOPHILINES/CYCLOPHILINES ET DE MEMBRES DE LA SUPERFAMILLE DES RÉCEPTEURS D'IMMUNOGLOBULINES EMMPRIN

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.02.2009  US 150660 P**

(43) Date of publication of application:
**14.12.2011  Bulletin 2011/50**

(73) Proprietor: **Sichuan Huiyu Pharmaceutical Co., Ltd.**
**NaJiang Sichuan province (CN)**

(72) Inventors:
• **EDWARDS, Carl, K., III**
**Commerce, CO 80022 (US)**
• **RENGASAMY, Asokan**
**Aurora, CO 80026 (US)**
• **FUJITA, Mayumi**
**Englewood, CO 80111 (US)**
• **EISENMESSER, Elan, Z.**
**Denver, CO 80206 (US)**
• **JONSCHER, Karen, R.**
**Centennial, CO 80112 (US)**
• **NORRIS, David, A.**
**Greenwood Village, CO 80111 (US)**
• **LI, Li**
**Centennial, CO 80015 (US)**

(74) Representative: **Hanna Moore + Curley**
**Garryard House**
**25/26 Earlsfort Terrace**
**Dublin 2, D02 PX51 (IE)**

(56) References cited:
**WO-A2-2007/046893     US-A1- 2008 241 145**
**US-A1- 2009 258 024**

• JONSCHER K R ET AL: "Determining the roles of FKBP52 and CD147-EMMPRIN in stimulated human T cells leading to macrophage activation and proinflammatory cytokine production", JOURNAL OF INVESTIGATIVE DERMATOLOGY, vol. 129, no. Suppl. 1, April 2009 (2009-04), page S117, XP002699827, & 69TH ANNUAL MEETING OF THE SOCIETY-OF-INVESTIGATIVE-DERMATOLOGY; MONTREAL, CANADA; MAY 06 -09, 2009 ISSN: 0022-202X
• JESSE M. DAMSKER ET AL: "Targeting the chemotactic function of CD147 reduces collagen-induced arthritis", IMMUNOLOGY, vol. 126, no. 1, 1 January 2009 (2009-01-01), pages 55-62, XP55068991, ISSN: 0019-2805, DOI: 10.1111/j.1365-2567.2008.02877.x
• WILLIAM GWINN ET AL: "Novel approach to inhibit asthma-mediated lung inflammation using anti-CD147 intervention.", THE JOURNAL OF IMMUNOLOGY, vol. 177, no. 7, 1 October 2006 (2006-10-01), pages 4870-4879, XP055068995, ISSN: 0022-1767

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- DATABASE BIOSIS [Online] BIOSCIENCES INFORMATION SERVICE, PHILADELPHIA, PA, US; December 2008 (2008-12), CHANG JING ET AL: "Study on psoriatic peripheral blood T-lymphocytes: effects of CD147-targeting small interfering RNA on the expression of CD147 and on cell proliferation and activation", XP002699828, Database accession no. PREV200900160528 & CHANG JING ET AL: "Study on psoriatic peripheral blood T-lymphocytes: effects of CD147-targeting small interfering RNA on the expression of CD147 and on cell proliferation and activation", ZHONGHUA PIFUKE ZAZHI, vol. 41, no. 12, December 2008 (2008-12), pages 783-786, ISSN: 0412-4030
- K. Arora ET AL: "Extracellular Cyclophilins Contribute to the Regulation of Inflammatory Responses", THE JOURNAL OF IMMUNOLOGY, vol. 175, no. 1, 21 June 2005 (2005-06-21) , pages 517-522, XP055296429, US ISSN: 0022-1767, DOI: 10.4049/jimmunol.175.1.517
- Vyacheslav Yurchenko ET AL: "Dealing with the family: CD147 interactions with cyclophilins", Immunology, vol. 117, no. 3, 1 March 2006 (2006-03-01) , pages 301-309, XP055296432, GB ISSN: 0019-2805, DOI: 10.1111/j.1365-2567.2005.02316.x
- J. M. Damsker ET AL: "Preferential chemotaxis of activated human CD4+ T cells by extracellular cyclophilin A", Journal of Leukocyte Biology, vol. 82, no. 3, 26 June 2007 (2007-06-26), pages 613-618, XP055068993, ISSN: 0741-5400, DOI: 10.1189/jlb.0506317
- RAILSON J E ET AL: "Heat Shock Protein-56 is Induced by Cardiotrophin-1 and Mediates its Hypertrophic Effect", JOURNAL OF MOLECULAR AND CELLULAR CARDIOLOGY, ACADEMIC PRESS, GB, vol. 33, no. 6, 1 June 2001 (2001-06-01), pages 1209-1221, XP027228309, ISSN: 0022-2828 [retrieved on 2001-06-01]
- Zhi-Guang Fu ET AL: "A novel small-molecule compound targeting CD147 inhibits the motility and invasion of hepatocellular carcinoma cells", Oncotarget, 23 February 2016 (2016-02-23), page 9429, XP055328689, United States Retrieved from the Internet: URL:https://www.ncbi.nlm.nih.gov/pmc/articles/PMC4891050/pdf/oncotarget-07-9429.pdf
- NAIHSUAN GUY ET AL: "Therapeutic Targeting of the FKBP52 Co-Chaperone in Steroid Hormone Receptor-Regulated Physiology and Disease", CURRENT MOLECULAR PHARMACOLOGY, vol. 9, no. 2, 7 December 2015 (2015-12-07), pages 109-125, XP055328698, NL ISSN: 1874-4672, DOI: 10.2174/1874467208666150519114115

**Description**

**FIELD OF THE INVENTION**

The present invention relates to compositions for modulating an adaptive immune response without significantly affecting an innate immunity response in a subject by inhibiting, independently or in combination, an Immunophilin/Cyclophilin Family Member and/or an EMMPRIN Immunoglobulin Receptor Superfamily Member. The present invention also relates to compositions for treating a variety of clinical conditions by inhibiting, independently orin combination, an Immunophilin/Cyclophilin Family Member and/or an EMMPRIN Immunoglobulin Receptor Superfamily Member.

**BACKGROUND OF THE INVENTION**

A wide variety of clinical conditions and diseases are mediated by acute and chronic inflammatory processes, regulated by the immune system. The immune system is designed to protect the host from infectious insults and tumor cells that may arise during a lifetime of exposure to a variety of invaders, and to eliminate foreign, potentially toxic chemicals. In a broad sense, there are two major effector pathways that the immune system uses to protect the host: innate and adaptive immunity. Both innate and adaptive immune responses stimulate inflammatory response to injuries and invasion by foreign invaders through common effector molecules, particularly the proinflammatory cytokines $TNF\alpha$ and IL-1$\beta$. Therefore, both types of immunity can contribute to the pathophysiology of inflammatory diseases.

Innate immunity is designed to respond rapidly, but is limited in its scope of protection and does not develop into a long-lasting memory. An innate immune response is characterized by responses that involve acute inflammation (mediated by cytokine release by injured or infected cells), the complement system, and non-specific leukocytes including phagocytes (macrophages, neutrophils, dendritic cells) and natural killer cells. Adaptive immunity takes longer to develop, occurring after the primary exposure to a pathogen, vaccination, or experimental immunization. However, adaptive immune responses are specific (i.e., have the ability to respond to a wide range of different types of potentially dangerous insults to the host), and have the ability to develop long lasting "immunologic memory". This ability results in a pool of memory T cells that can be rapidly mobilized, activated, expanded, and differentiated into mature peripheral effector cells to mediate a protective immune response against most, if not all, foreign invaders (i.e., bacteria, viruses, and parasites). Both the innate and adaptive immunity are highly interactive and play critical roles in initiating inflammation, amplifying an immune response, shaping the nature of the immune response, eliminating the pathogen, and terminating the immune effector mechanisms and the associated inflammation at an appropriate time. Both adaptive and innate immunity share common effector molecules, particularly cytokines and chemokines. These mediators are critical for cell-cell interactions, activation and migration of effector cells, and the generation of an effective immune response. Conditions such as inflammatory and autoimmune diseases result from overly-active innate or adaptive immune responses (or both).

Examples of conditions that are mediated by chronic inflammation are Autoimmune diseases such as Rheumatoid Arthritis (RA), Chronic Plaque Psoriasis (CPPs), and Psoriatic Arthritis (PsA). These chronic inflammatory diseases are characterized by hyperproliferation of fibroblasts and synoviocytes (in RA) and keratinocytes (in CPPs/PsA) and the infiltration of activated T-cells (particularly TH22 cells), M$\Phi$, and other immune cells into lesional skin and/or joints. Proinflammatory cytokines, including Tumor Necrosis Factor-$\alpha$ ($TNF\alpha$), Interleukin-1$\beta$ (IL-1$\beta$), and Interleukin-32$\gamma$ (IL-32$\gamma$) are produced in these patients, and induce and maintain chronic inflammation. Up-regulation of these factors by activated immune system cells including M$\Phi$ drives the pathogenesis of these disorders. It has been shown in clinically relevant animal models that T cells play a key role in M$\Phi$ activation and the pathogenesis of autoimmune diseases. In addition to autoimmune diseases, chronic inflammation also plays a role in many cancers and cardiovascular diseases.

Psoriasis, which affects approximately 2-3 percent of the world's population and the cost of treatment in the US alone exceeds $3 billion each year. Activated T cells and cytokines are important in the pathophysiology of this relatively common immune disease. Cutaneous and systemic overexpression of several proinflammatory cytokines, particularly $TNF\alpha$, is considered to be responsible for initiation, maintenance and recurrence of skin lesions. Introduction of $TNF\alpha$ inhibitors has proved to be an effective treatment for inflammation in autoimmune and rheumatic diseases including rheumatoid arthritis, psoriasis, psoriatic arthritis, ulcerative colitis, and ankylosing spondylitis. However, $TNF\alpha$ inhibitors have many serious drawbacks. $TNF\alpha$ inhibitor treatment does not cure the disease. For those who experience relief from symptoms, that relief is often only temporary. Further, the side effects caused by $TNF\alpha$ inhibitors include an increased risk of infection and other maladies such as tuberculosis, gram positive infections, lymphomas; and lupus-like and demyelinating diseases. The increased risk of serious infections in patients treated with $TNF\alpha$ inhibitors is due to the inhibitor's suppression of both the adaptive and innate immune systems, which leaves the patient defenseless against infection.

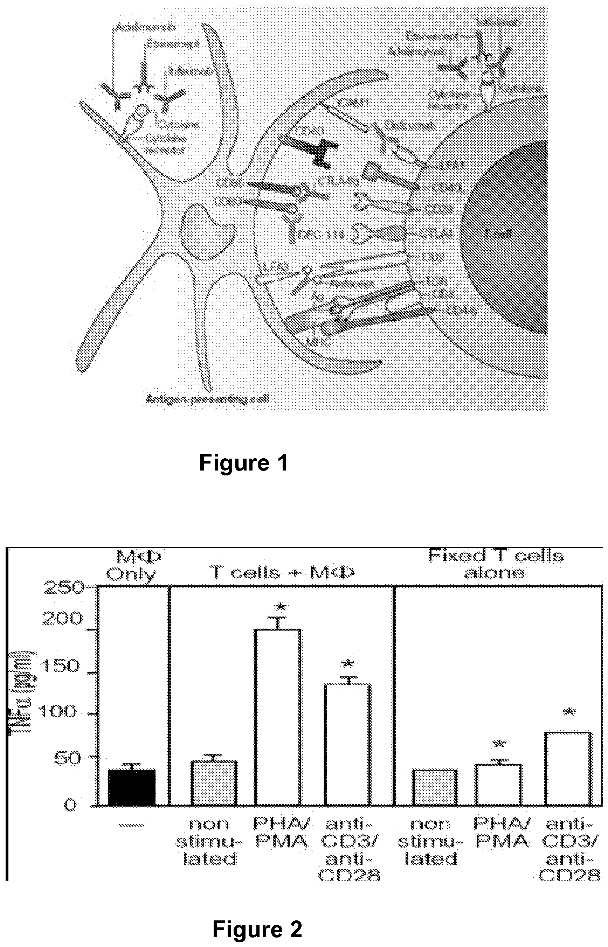

Biologics specifically targeted toward psoriasis and psoriatic arthritis have emerged as promising new treatment options. These treatments are "targeted" and work by blocking signaling of activated T cells. Amevive® (alefacept), the first biologic agent approved for treating psoriasis, binds to CD2 receptors on T cells, disrupting the interaction with LFA-3 in the immune synapse, triggering apoptosis of pathogenic T cells. Raptiva® (efalizumab) binds to LFA-1, blocking ICAM-1 binding of T cells and antigen presenting cells, thus preventing T cell activation and trafficking into the dermis and epidermis. Treatments such as etanercept, infliximab and adalimumab are biologics designed to bind TNFα, inhibiting inflammatory responses, cell filtration and keratinocyte proliferation. However, these biologic treatments are not effective in all patients, suggesting that alternative T cell pathogenesis pathways and/or T cell co-stimulation molecules may exist. Figure 1 shows an example of an IS and the site of action for current treatments.

Damsker et al., Immunology, Vol. 126, No. 1, pp. 55-62 (2009) describe that the targehting of the chemotactic function of CD147 reduces collagen-induced arthritis.

Gwinn et al., The Journal of Immunology, Vol. 177, No. 7, pp. 4870-4879 (2006) describe a novel approach to inhgibit asthma-mediated lung inflammation using anti-CD147 intervention.

Jonscher et al., The Journal of Investigative Dermatology, Vol. 129, Suppl. 1, p. S117 (2009) concerns the determination of the roles of FKBP52 and CD147-EMMPRIN in stimulated human T cells leading to macrophage activation and pro-inflammatory cytokine production.

The lack of effective treatments for conditions involving chronic inflammation, has given rise to a need for treatments that can reduce chronic inflammation without depriving the patient of both adaptive and innate immune defenses.

## SUMMARY

In one embodiment, the current disclosure is directed to a composition that simultaneously inhibits activity and/or expression of FKBP52 and CD147 for use in a method for inhibiting pro-inflammatory cytokine production in a human having a chronic inflammatory disease, said method comprising administering an effective amount of a said composition, wherein the composition comprises

(i) one or more siRNA molecules that silence expression of FKBP52 and CD147,
(ii) one or more antibodies or functional fragments thereof that inhibit the activity and/or expression of FKBP52 and CD147.

In some embodiments, the composition may comprise two or more siRNA molecules that silence CD147 and FKBP52 expression. In other embodiments, the composition comprises two or more antibodies or functional fragments thereof that inhibit CD147 and FKBP52 activity. In some embodiments, the antibody may be a bispecific antibody.

The disclosure is related to a method for selectively inhibiting an adaptive immune response without significantly affecting an innate immunity response in a subject, the method comprising inhibiting the activity and/or expression of CD147 and FKBP52 proteins in the subject.

In some embodiments, the activity and/or expression of CD147 and FKBP52 may be inhibited by administering siRNA molecules to silence CD147 and FKBP52 expression or by administering two or more antibodies or functional antibody fragments. In some embodiments, the antibody may be a bispecific antibody.

## BRIEF DESCRIPTION OF THE DRAWINGS

Figure 1 is a schematic diagram of an immune synapse showing known co-stimulatory molecules and associated treatments currently available.

Figure 2 is a graph showing that nearly twice as much TNFα was produced in the when activated T cells are cultured with MΦ in comparison with T cell control cultures alone

Figure 3 is a graph that showed that TNFα and IL-1β were barely induced in PHA/PMA stimulated Molt4 and Jurkat cells, while not induced at all in resting primary human T cells, unstimulated Hut-78, H9, Molt4 or Jurkat cells. TNFα and IL-β were induced in Hut-78 and H9 cells.

Figure 4 is a schematic diagram of FKBP52 (Figure 4A), full length cellular EMMPRIN (Figure 4B), soluble EMMPRIN ligand (Figure 4C), and soluble EMMPRIN receptor (Figure 4D). Potential inhibitors and binding sites shown.

Figure 5 is a schematic diagram of how a bispecific antibody (bsAb) could act to recognize (Figure 5A) or block (Figure 5B) FKBP52 and CD147 interactions on the surface of human CD4+ T cells.

**Figure 6** is a schematic diagram of how a panel of mammalian cell derived bispecific, high affinity antibodies to FKBP52 and CD147 can be prepared.

**Figure 7** is a schematic diagram of CD147 Ig domains and the immunophilins/cyclophilins that can associate with it.

**Figure 8** illustrates that recombinant CD147 is active and can be studied in solution. A) CD147[22-269] stimulates secretion of both TNF$\alpha$ and IL-1$\beta$ in THP-1 macrophages. THP-1 cells were initially induced for 24 hrs with PMA, producing adherent macrophages that were further stimulated with buffer, LPS, or 100ug/ml recombinant protein. ELISA detection kits quantified cytokines. B) 15N-HSQC spectra are shown for two constructs that also contain the two proposed cyclophilin target sites, Pro180 and Pro211.

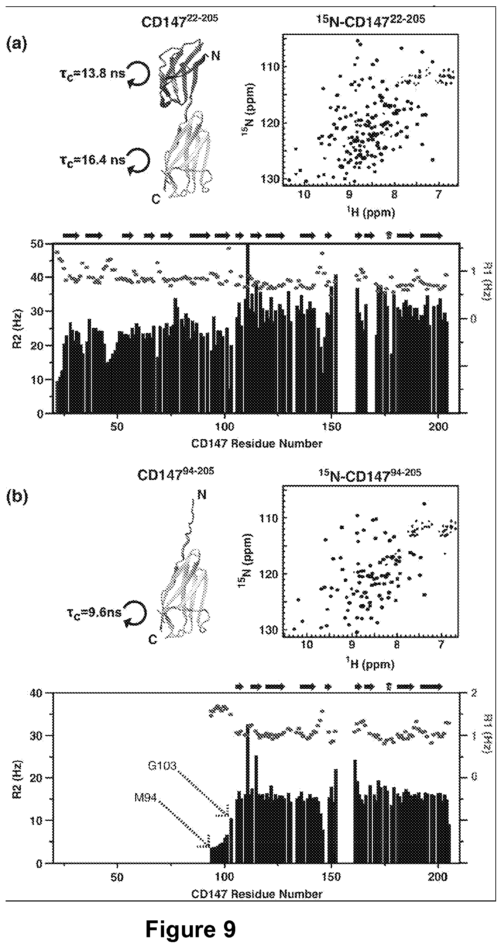

**Figure 9** shows the results of NMR solution studies that reveal that the CD147 Ig-like domains do not self-associate or interact with each other. The extracellular regions of the two major CD147 isoforms, (a) isoform 2 (CD147 [22-205]) and (b) isoform 3 (CD147[94-205]), were characterized using NMR solution studies. The particular regions under study are shown using the X-ray crystal structure (Protein Data Bank accession code: 3B5H) with the corresponding 15N-HSQC spectra and NMR relaxation data. Specifically, amide R1 (dots) and R2 (black bars) relaxation rates were obtained for both CD147 constructs and used to calculate the correlation times. The extracted correlation times are shown adjacent to each Ig-like domain, Ig1 and Ig2. Secondary-structure propensities are shown on top of each plot as calculated from our chemical shift assignments with the program TALOS. (43) All data were collected at 25°C and 900 MHz.

**Figure 10** shows that CypA and CypB target CD147 through their active sites. 15N-TROSY-HSQC spectra of free 0.5 mM 15N-labeled A) CypA and B) CypB. Free enzymes (black) with 0.5 mM CD147[94-214]. Thus, both of these enzymes specifically recognize CD147. Titrations (left panels) were conducted at 25 °C at 900 MHz and only residues exhibiting 15N or 1H shifts >0.1, 0.02 ppm, respectively, are shown mapped onto the structures in dark (right panels).

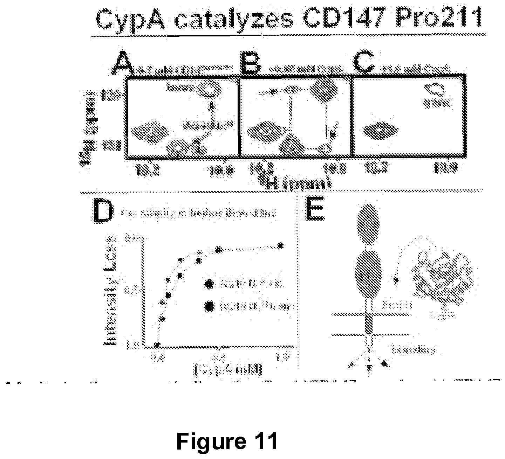

**Figure 11** illustrates monitoring of the enzymatically active CypA/CD147 complex. A) CD147 residues 206-214 exist in two conformations in the absence of a cyclophilin ligand that is dictated by Pro211 slow isomerization. B) The addition of substoichiometric concentrations of CypA results in new peaks, called "exchange peaks" (black arrows), indicating CypA enhances the rate of catalysis. C) The *cis* resonances diminish in intensity (line broaden) faster than the *trans* resonances. D) Quantitatively, the faster loss of the *cis* resonance intensities suggests the affinity of CypA for this conformation of CD147 is higher than the *trans.* E) Cartoon depiction of CypA targeting CD147 Pro211 that alters the *cis:trans* equilibrium leading to intracellular signaling.

**Figure 12** illustrates the characterization of CypA/CD147 binding and CypA-mediated catalysis of CD147 in solution. A) A short peptide encompassing the cyclophilin-target site of CD147 P211, called CD147[10mer], has been used to estimate the affinity of this complex. CypA residues exhibited chemical shift changes give $K_D$ ~5 mM. Binding isotherms are shown for W121[HE] and A101[NH]. B) ZZ-exchange for W121[NE] using [CypA]=0.05 mM, [CD14794-214]=0.5 mM of both auto and exchange peaks.

**Figure 13** are graphs showing that the proinflammatory cytokine TNF$\alpha$ is upregulated in T cells taken from the blood of psoriasis patients as compared with those obtained from the blood of healthy donors.

**Figure 14** is a 2D gel electrophoresis of H9 T cells. H9 cells were stimulated with PMA/Ionomycin and separated by 2D gel electrophoresis. Spot number 13, identified as FKBP52 using tandem mass spectrometry and database searching, was increased in expression by at least 3 fold over the matching spot in unstimulated cells.

**Figure 15** is a tandem mass spectrum illustrating the relative absorbance for spot number 13 (FKBP52) from the 2D gel electrophorsis results of Figure 14.

**Figure 16** is a Western blot showing the upregulation of FKBP52 in CD3+ cells. Lanes 1,2: H9 cells, unstimulated (U) and stimulated with PMA/ionomycin (S). Lanes 3,4: CD3+ T cells, U and S. Lanes 5,6: Jurkat cells, U and S. Lane 7: + Control.

**Figure 17** is a Western blot demonstrating effective knockdown of FKBP52 by siRNA in CD3+ cells. Lanes 1,2: Unstimulated (U) and stimulated (S) H9 cells. Lanes 3,4: FKBP52 siRNA transfected H9 cells, U and S. Lanes 5,6: CD3+ T cells, U and S. Lanes 7,8: FKBP siRNA transfected CD3+ T cells, U and S.

**Figure 18** is a graph illustrating that FKBP52 did not have a significant inhibitory effect on TNFα levels, and an inset graph with 4-1 BB as a positive control.

**Figure 19** is a FACS analysis that shows that CD147 cell surface expression is upregulated in stimulated T cells and is induced by T cell Mφ contact.

**Figure 20** is a graph illustrating the results of FACS analysis of Figure 19.

**Figure 21** illustrates NMR data for $^{15}$N-labeled CD147 at 0.5 mM (darker lines) that was titrated with 0.5 mM unlabeled FKBP52. Resonances of CD147 corresponding to the receptor's membrane proximal region, residues 207-234, exhibit NMR chemical shift changes, indicating a weak association.

**Figure 22** is graph illustrating a significant decrease in TNFα production as a result of dual siRNA knockdown of CD147 and FKBP52.

**Figure 23** is a graph illustrating insignificant change in cytokine production (TNFα) as a result of siRNA knockdown of CD147 ("TCISM #1).

**Figure 24** is a graph illustrating a moderate decrease (approximately 45-50%) in cytokine production (IL-32γ) as a result of siRNA knockdown of CD147 ("TCISM #1") in CD3+ cells.

**Figure 25** is is a graph illustrating insignificant change in cytokine production (TNFα) as a result of siRNA knockdown of FKBP52 ("TCISM #2) in CD3+ cells.

**Figure 26** is a graph illustrating a moderate decrease in cytokine production (IL-32γ) as a result of siRNA knockdown of FKBP52 ("TCISM #2) in CD3+ cells.

**Figure 27** is a graph illustrating a highly significant (**$P < 0.001$) decrease in cytokine production (TNFα) as a result of siRNA knockdown of CD147 ("TCISM #1") and FKBP52 ("TCISM #2) in CD3+ cells.

**Figure 28** is a graph illustrating a highly significant (**$P < 0.001$) decrease in cytokine production (IL-32γ) as a result of siRNA knockdown of CD147 ("TCISM #1") and FKBP52 ("TCISM #2) in CD3+ cells.

**Figure 29** is a graph illustrating a highly significant (**$P < 0.001$, significance is between matching *, **, and ***) decrease in cytokine production (TNFα) as a result of siRNA knockdown of CD147 ("TCISM #1") and FKBP52 ("TCISM #2") in Th22 cells.

**Figure 30** is a graph illustrating a highly significant (**$P < 0.001$, significance is between matching *, **, and ***) decrease in cytokine production (IL-32γ) as a result of siRNA knockdown of CD147 ("TCISM #1") and FKBP52 ("TCISM #2") in Th22 cells.

**Figure 31** is a graph illustrating a highly significant (**$P < 0.001$, significance is between matching *, **, and ***) decrease in cytokine production (TNFα) as a result of mAb blocking of CD147 ("TCISM #1") and FKBP52 ("TCISM #2") in Th22 cells.

**Figure 32** is a graph illustrating a highly significant (**$P < 0.001$, significance is between matching *, **; and ***) decrease in cytokine production (IL-32γ) as a result of mAb blocking of CD147 ("TCISM #1") and FKBP52 ("TCISM #2") in Th22 cells.

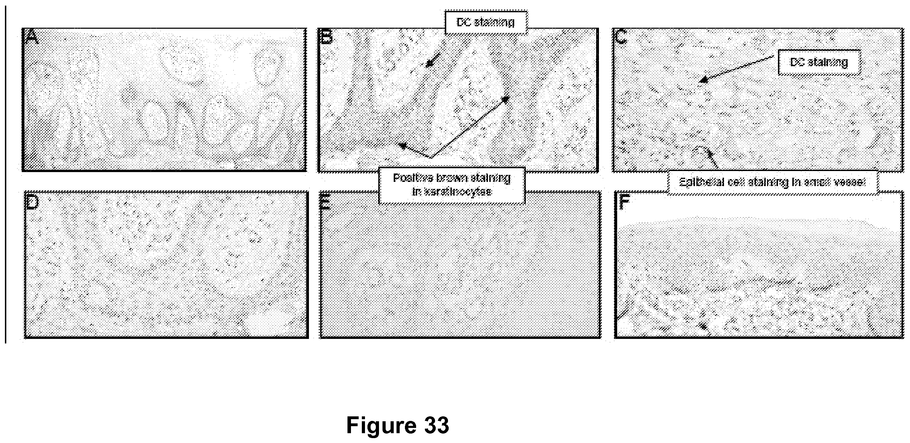

**Figure 33** shows immunohistochemical expression of human IL-32γ in lesional psoriatic skin. Human psoriatic skin biopsies (N=15) (A to C) and normal skin (N=13) (F) were stained with a murine anti-human IL-32γ Mab. In psoriatic skin, there is extensive cytoplasmic granular staining present in basal layer keratinocytes along with strong nuclear staining indifferent subpopulations of dendritic cells. A control antibody showed no positive staining (D). An IL-32γ competitive antagonist peptide blocked the staining of the IL-32γ Mab, demonstrating the specificity of IL-32γ protein expression in the psoriatic skin biopsies (E). Weakly nuclear and cytoplasmic IL-32γ staining was apparent in normal skin keratinocytes (F). Representative data are shown from a total of three separate experiments (with triplicate staining) where the assessor was blinded to the identity of each slide viewed.

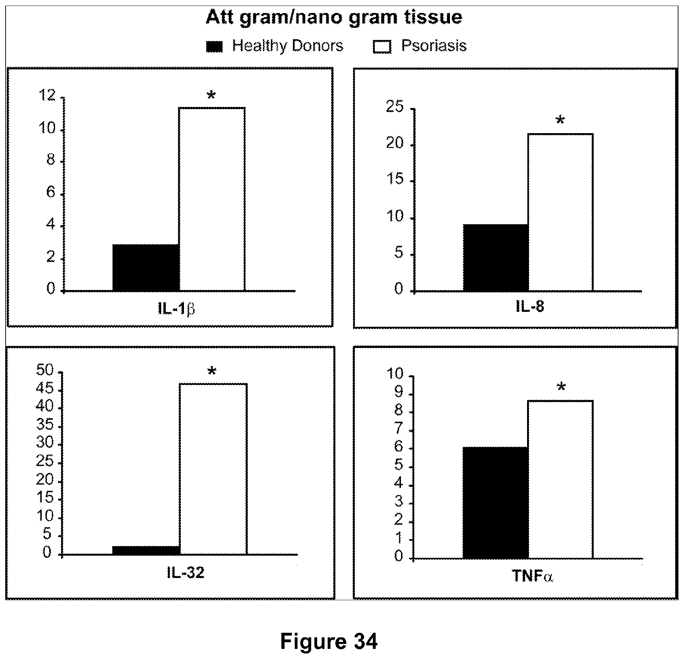

**Figure 34** shows IL-32 and other proinflammatory cytokines are expressed in lesional psoriatic skin biopsy samples. Proinflammatory cytokine gene expression analysis was conducted using quantitative "real-time" polymerase chain reaction (qRT-PCR) analysis in lesional psoriatic skin formalin-fixed paraffin embedded (FFPE) biopsy samples (N=6). Healthy human donor skin biopsy samples (N=6) were used as negative controls. Human monocyte-derived Mφ cultures were used as positive control tissue (data not shown). The quantities of the target cytokine, assessed with consensus sequence PCR probes, were measured in triplicate. The qRT-PCR results indicated IL-32γ, IL-1β, TNFα and IL-8 expression was significantly (*) different (P < 0.01 by ANOVAs) than similar cytokine expression observed in the healthy control skin tissue. The results are representative of a single experiment where a total of three separate experiments were performed. Each bar represents the mean +/- SD.

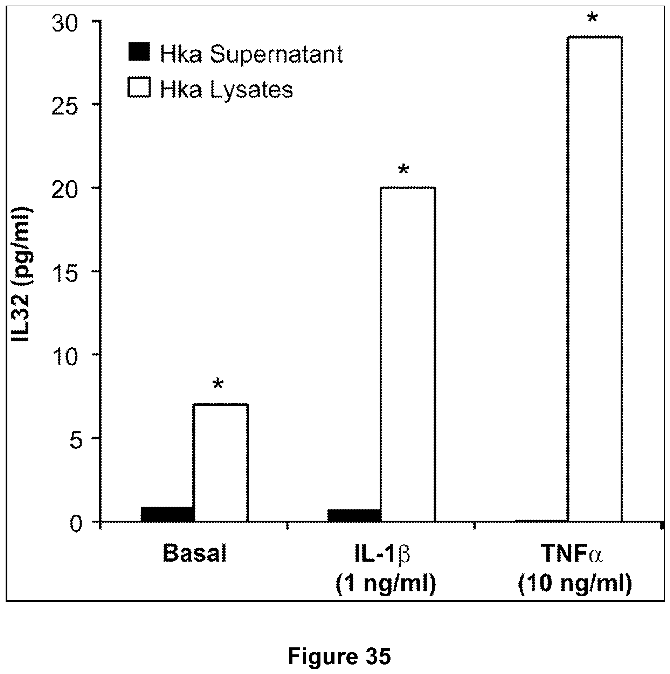

**Figure 35** illustrates Electrochemiluminescence (ECL) analysis of IL-32γ protein production by human primary skin keratinocytes. ECL analysis of human primary keratinocytes (Hka) cell culture fluid (shown as black) and lysates (shown as white) indicates that IL32γ is present essentially as a cytoplasmic cytokine. IL1β (R&D Systems) and TNFα (R&D Systems) potently induces significant (*; P < 0.001 by ANOVA) amounts of cytoplasmic IL32γ protein in Hka cultures. Samples were measured in duplicate; results are representative of a single experiment where four separate experiments using different passages of primary skin Hka cell cultures were conducted. IL32γ did not induce either Hka tissue culture fluid IL32γ or Hka lysate IL32γ when measured by ECL (data not shown). Each bar represents the mean ± SD.

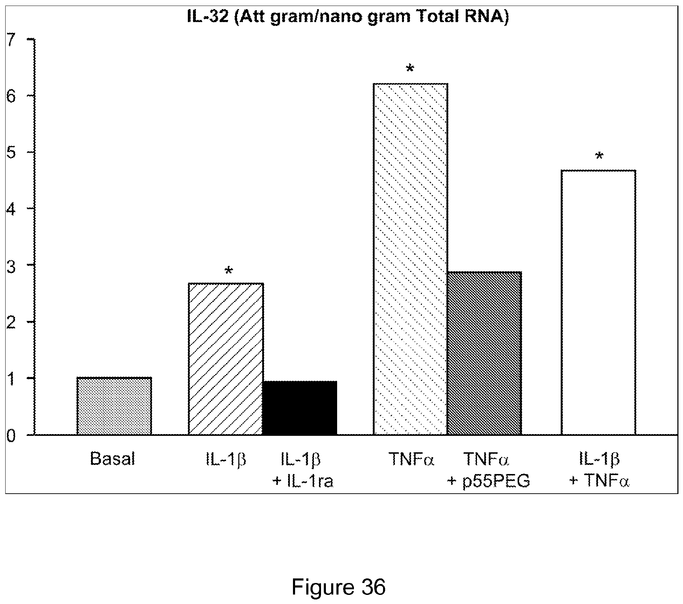

**Figure 36** illustrates that proinflammatory cytokines upregulate IL-32γ gene expression in human primary skin keratinocyte cultures. Human primary skin keratinocytes (Hka) were stimulated with either IL1β (1 ng/ml), TNFα (10ng/ml), or both cytokines. IL32γ gene expression was detected using qRT-PCR. IL32γ gene expression was significantly (*; P < 0.001 by ANOVA) increased after cytokine stimulation. These responses were reversed *in vitro* by either IL1ra (Kineret; Amgen Thousand Oaks, CA) (*Cohen et al. 2003*) or p55-PEG (Pegsunercept; Amgen Thousand Oaks, CA) (*Edwards, 1999*). The quantities of target IL32γ, assessed with IL32 consensus sequence IL32 probes, were measured in triplicate. The results are from a single representative experiment (mean +/- SD) where three separate experiments were conducted.

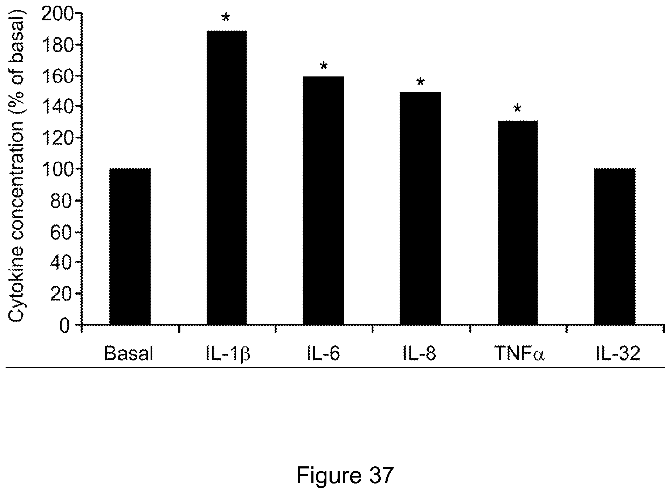

**Figure 37** illustrates that recombinant human IL32α (rhuIL32α) induces proinflammatory cytokines in primary human skin keratinocyte cultures. Optimal protein levels of rhuIL-32α (10 pg/ml; R&D Systems) significantly (*; P < 0.05 by ANOVA) induces various proinflammatory cytokines in human primary skin keratinocytes (Hka). Cytokine protein in Hka cell culture fluid and lysates (data not shown) was measured by ECL. This preparation of endotoxin free rhuIL32α did not induce increased levels of Hka cell culture fluid or lysates IL32γ. Samples were measured in triplicate and data shown are from a single representative experiment where four separate experiments were conducted with different human donor Hka cultures. Each bar represents the mean +/-SD.

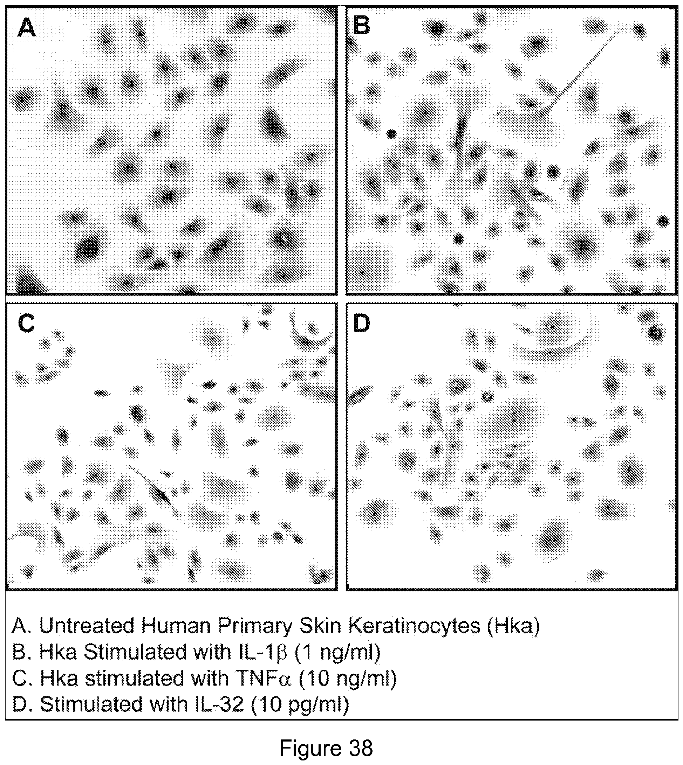

**Figure 38** illustrates that recombinant human IL32γ alters the phenotypic appearance of human primary skin keratinocytes. Human primary skin keratinocytes (Hka) were treated with optimal activating levels of recombinant human cytokines, including IL32γ, and stained by hematoxylin and eosin (H&E) staining 12, 24, and 48 hours later. The Hka cultures at 48hr post-treatment are shown (200X by confocal microscope analysis). Individual Hka cell cultures treated with noted recombinant cytokine appeared spread out and to contain numerous pseudopodia with a darkly stained nucleus. Representative data shown from a single experiment where a total of three separate experiments (with triplicate staining) were conducted. The assessor was blinded to the identity of each slide viewed.

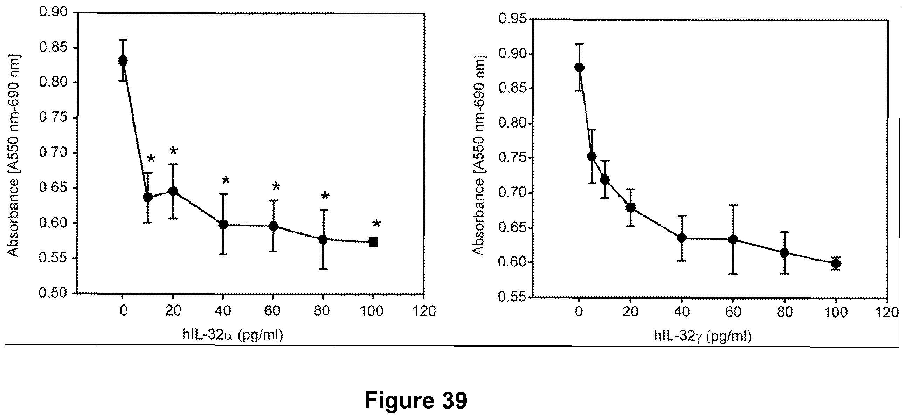

**Figure 39** illustrates that recombinant IL32α or IL32γ inhibits human primary skin keratinocyte proliferation. Human primary skin keratinocyte cells (Hka) were treated *in vitro* with increasing amounts of either rhuIL-32α or rhuIL32γ, and proliferation was measured by the MTT assay 12, 24, and 48 hours later. The data represent a mean ± SD of six in individual replicates. Data are representative from a single experiment where three separate experiments were performed using different human donor Hka cells. Each data point (*) is significantly different (P < 0.001 by ANOVA) when compared to Hka untreated cells.

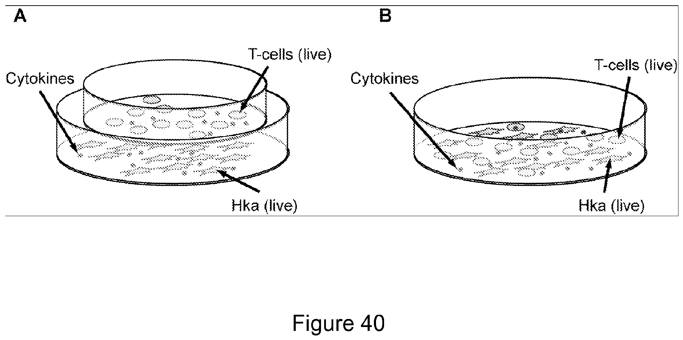

**Figure 40** illustrates an indirect and direct cell-cell contact bioassay methodology. In the indirect cell-cell contact *in vitro* assay (shown in A), non-activated or activated live human CD3[+], H9 or Th22 T-cells are placed in sterile plastic *trans* well inserts before being placed on top of primary human skin keratinocytes (Hka). Proinflammatory cytokine gene expression is measured by qRT-PCR, and protein production, measured by ECL, are subsequently measured in collected *trans* well T-cells or Hka cell culture cell culture fluids (2 ml) and lysates 12, 24, and 48 hr post-addition of transwell. In the direct cell-cell contact assay (shown in B), non-activated or activated live human CD3[+], H9 or Th22 T-cells are placed directly on top of live Hka cells. After 12, 24, or 48hr, non-adherent T-cells and cell culture fluid (2 ml) is collected, centrifuged, whereby T-cells are separated and either frozen at -80° or T cell total RNA is prepared for qRT-PCR gene expression analysis. Adherent Hka cells are carefully washed 3X with warm tissue culture media, scraped using a rubber policeman, collected, centrifuged, and aliquot off, and are then either frozen at -80° or Hka total RNA is prepared for qRT-PCR gene expression analysis. Cell culture fluid and Hka cell lysates (obtained by using triton X 100) can be measured for proinflammatory cytokine levels using ECL. The addition of Triton X 100 does not interfere with ECL analysis.

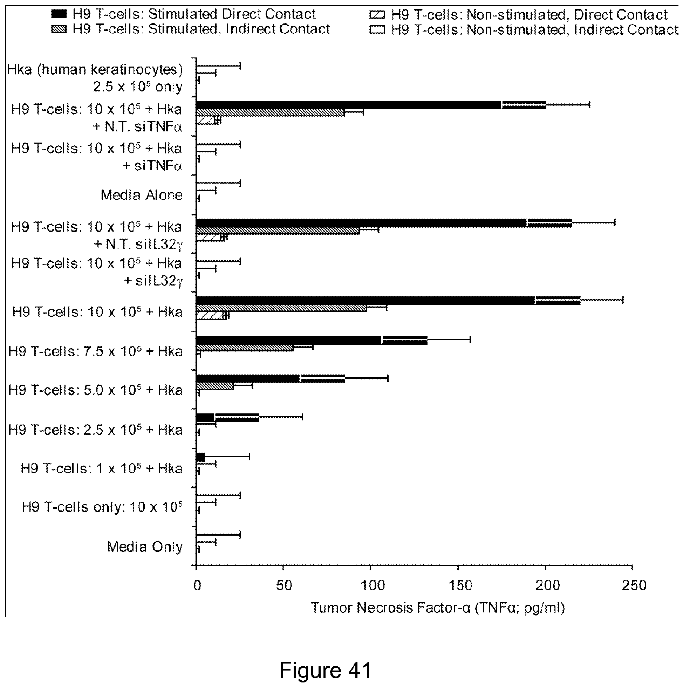

**Figure 41** is a bar graph illustrating the production of Hka derived TNF$\alpha$ after direct cell-cell contact with H9 T cells. TNF$\alpha$ (measured by ELISA) was assessed from Hka supernatants after direct cell-cell contact for +48 hrs with non-activated or PMA/ionomycin-activated human H9 T cells, in a dose-related fashion. (A). Hka (2.5 x 105 total cells only); (B). Hka (2.5 x 105 total cells) directly contacted with non-activated H9 T cells (5 x 105 total H9); (C). Hka (2.5 x 105 total Hka) directly contacted with activated H9 T cells (10 x 105 total H9). Measurements were assessed in triplicate. Each bar represents mean $\pm$ SD (N=3 replicates) where *$P$ < 0.05 versus Hka only, and where *#$P$ < 0.001 versus Hka only. Data shown are representative from a single experiment where a total of four separate experiments with similar results were performed.

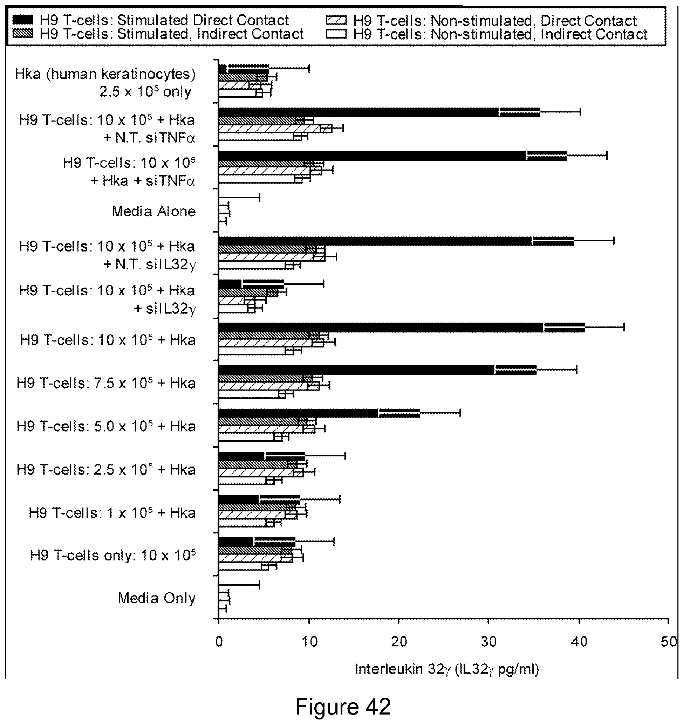

**Figure 42** is a bar graph illustrating production of Hka lysate-derived IL32$\gamma$ after direct cell-cell contact with H9 T cells. IL-32$\gamma$ (measured by ECL) was assessed from Hka lysates after direct cell-cell contact for +48 hrs with non-activated or PMA/ionomycin-activated human H9 T cells, in a dose-related fashion. (A). Hka (2.5 x 105 total cells only); (B). Hka (2.5 x 105 total cells) directly contacted with non-activated H9 T cells (5 x 105 total H9); (C). Hka (2.5 x 105 total Hka) directly contacted with activated H9 T cells (10 x 105 total H9). Measurements were assessed in triplicate. Each bar represents mean $\pm$ SD (N=3 replicates) where *$P$ < 0.05 versus Hka only, and where *#$P$ < 0.001 versus Hka only. Data shown are representative from a single experiment where a total of four separate experiments with similar results were performed.

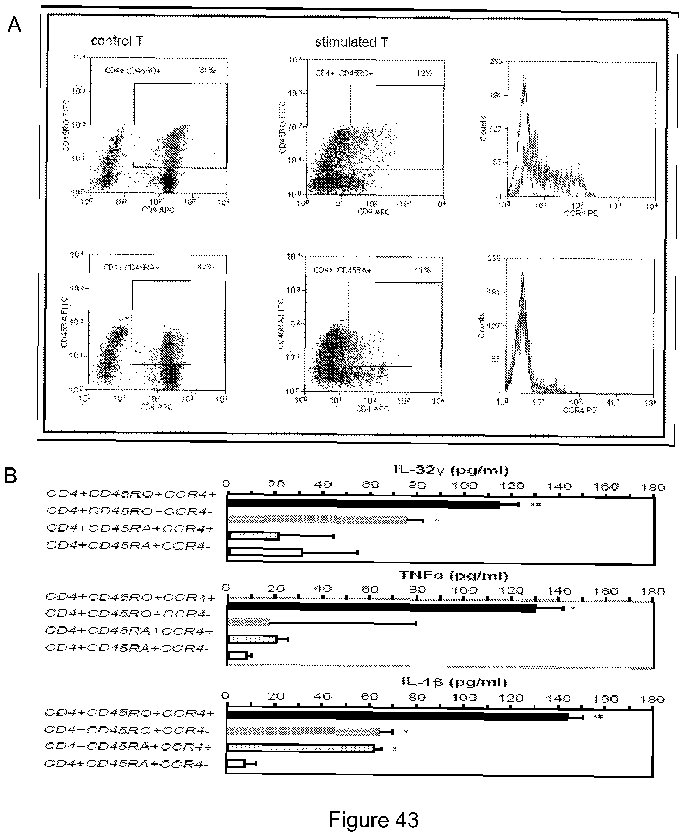

**Figure 43** illustrates the production of Hka lysate-derived IL-32$\gamma$, TNF$\alpha$, and IL1$\beta$ after direct cell-cell contact with activated human memory effector T cell populations. A) PMA/ionomycin-activated CD3[+] T cells, obtained from healthy human donor leukopacs (Belle-Bonfils Blood Center, UCD Hospital, Denver, CO), were further purified using Memory CD4[+], CD45RA[+] and CD45RO[+] T cell isolation kits (Miltenyi Biotec). Cells were stained with CD45RO-FITC, CD45RA-FITC, CD4-APC, and CCR4-PE (all obtained from Beckman Coulter) for 20 minutes at room temperature. Cells were then washed in PBS followed by fixation in 2% paraformaldehyde. Flow cytometry was performed on a FC500 (Beckman Coulter, Hialeah, FL). 5000 events were collected for each sample. Data shown are representative from a single experiment where a total of four separate experiments were performed using 4 separate leukopacs. 1% paraformaldehyde-fixed T cells added to the Hka cells induced Hka lysate-derived IL-32$\gamma$ mRNA and protein (data not shown). B). PMA/ionomycin-activated human Th22 memory effector T cell populations (CD4[+]CD45RO[+]CCR4[+], CD4[+]CD45RO[+]CCR4[-], CD4[+]CD45RA[+]CCR4[+], CD4[+]CD45RA[+]CCR4[-][10 x 10⁵ total T cells, purified as outlined above]) were contacted with Hka after direct cell-cell contact for [+]48hrs. Hka lysates were measured for either IL32$\gamma$ (by ECL), TNF$\alpha$, or IL1$\beta$ (by ELISA; R&D Systems). A total of 6 individual measurements were assessed for each cell population. Each bar represents mean $\pm$ SEM where (*) $P$ < 0.05 (ANOVA), and where *# $P$ < 0.001 (ANOVA). A total of three separate experiments using separate healthy human donors were completed with similar results.

**Figure 44** is a schematic diagram showing the inflammatory process between keratinocytes and immune cells.

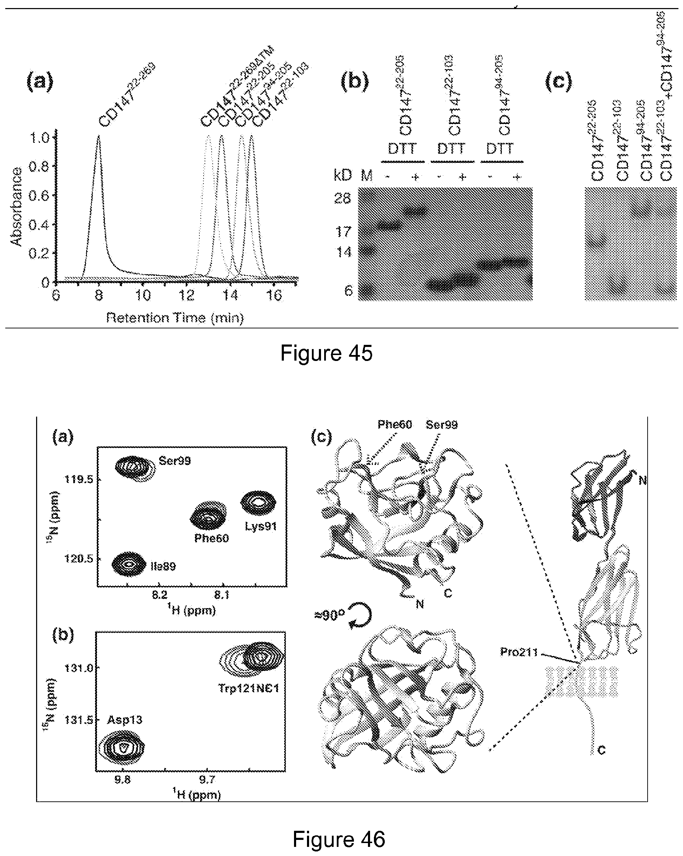

**Figure 45** shows that purified CD147 constructs exist as single monomeric species in solution in the absence of the TM region. (a) Size-exclusion chromatography of a subset of the constructs analyzed in this study and their retention volume upon loading 100 $\mu$l of 1 mg/ml of each sample onto an analytical Superose-12 column (column volume, 24 ml). Only constructs that contain the full TM domain, such as the full-length protein CD147[22-269], induce the formation of large oligomers, as shown by their elution at the void volume (-8 ml). The estimated molecular weights of CD147[94-205] and CD147[22-103]elute near the predicted molecular weights of a monomer, while both CD147[22-269]$\Delta$™ and CD147[22-205] elute higher due to their flexible regions and the flexible nature of the linker region between the two Ig-like domains. (b) Denaturing gel of CD147 constructs containing both domains (CD147[22-205]), Ig1 (CD147[22-103]), and Ig2 (CD147[94-205]) in the absence and presence of DTT. Reduction of the single disulfide bond within each domain leads to slower migratory behavior. (c) Native gel of the same constructs confirms that all constructs run as a single species and that Ig1 and Ig2 do not interact (right lane).

**Figure 46** shows that CypA specifically engages CD147 through its active site. (a) $^{15}$N-TROSY-HSQC spectra of 0.5 mM free 15NCypA (black) and in the presence of 1 mMCD147[22-214] (red) indicate that only the chemical environment of CypA active site residues such as Ser99 and Phe60 are perturbed by the interaction. (b)Within these same spectra, 15N-CypATrp121[Nε1] that is located in the short 3,10-$\alpha$-helix adjacent to the active site also exhibits chemical shift perturbation in the presence of CD147[22-214]. (c) Two views of the CypA residues that exhibit chemical shift perturbations upon addition of CD147[22-214] are shown, along with a cartoon extension of the Ig-like domains that contain the targeted Pro211. Residues are colored (red) onto our previously determined X-ray crystal structure of CypA (Protein Data Bank accession code: 1ZKT) if their respective chemical shift differences were greater than 0.06 ppm. Titrations were conducted at 25°C and at 900 MHz.

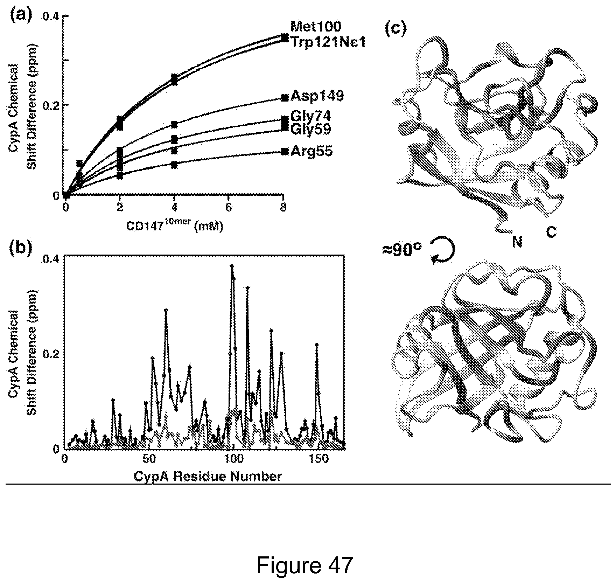

**Figure 47** illustrates the quantification of the binding interaction between CypA and CD147. (a) Binding isotherms are shown for both [15N]CypA active-site amides and the single indole of the active-site Trp121 upon addition of a peptide that corresponds to CD147 residues 205-214, the CD147[10mer]. A Kd of 4.2±0.2 mM was extracted from these fits. (b) Chemical shift changes of 15N-CypA are compared upon titration of CD147[22-214] (1 mM, red) and the CD14710mer peptide (8 mM, black). (c) CypA chemical shift changes with the CD147[10mer] peptide are relegated to the active site as they are with the larger CD147 constructs. All chemical shift changes were calculated as in Figure 46. Experiments were collected at 25°C and at 900 MHz.

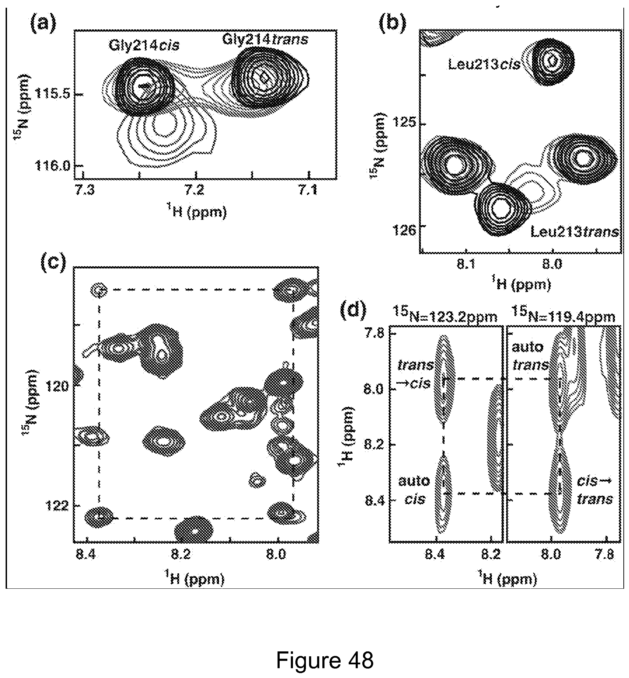

**Figure 48** shows that CD147 is a substrate of CypA. (a) 15N-TROSY-HSQC spectra of 0.5 mM free 15N-CD147[94-214] (black) and in the presence of both 100 $\mu$M CypA (blue) and 1 mM CypA (red) show that upon titration of the enzyme, CD147 Gly214 amide *cis* and *trans* resonances converge. Thus, relative to the CypA-bound chemical shift difference between the CD147 Gly214 *cis* and *trans* conformations, the CypA induced conformational exchange rate is fast. (b) Several of the CypA targeted CD147 residues, such as CD147 Leu213, exhibit severe linebroadening in the presence of substoichiometric concentrations of the enzyme and disappear under stoichiometric concentrations. Thus, relative to the CypA-bound chemical shift differences, the CypA-induced conformational exchange is on the intermediate timescale. (c) Using 0.5 mM 15N-CD147[94-214], ZZ-exchange cross peaks are observed for residues 207-214 in the presence of catalytic concentrations of CypA. Shown here is the corresponding spectrum of the CD147 Phe212 amide with 0.05 mM CypA and a mixing time of 0 ms (dark blue) and 300 ms (red). (d) 3D-NOESY-HSQC of 0.5 mM [15N]-CD147[94-214] and 0.01 mM CypA utilizing a 150-ms mixing time also identifies exchange peaks for CD147 residues 207-214 as shown for Phe212. In the absence of CypA, no such cross peaks are observed for either ZZ-exchange or within 3D-NOESY-HSQC experiments, indicating a much slower uncatalyzed exchange between CD147 *cis* and *trans* conformations (data not shown). All experiments were conducted at 25°C. Titrations were acquired at 900 MHz, and both ZZ-exchange and 3DNOESY-experiments were acquired at 720 MHz.

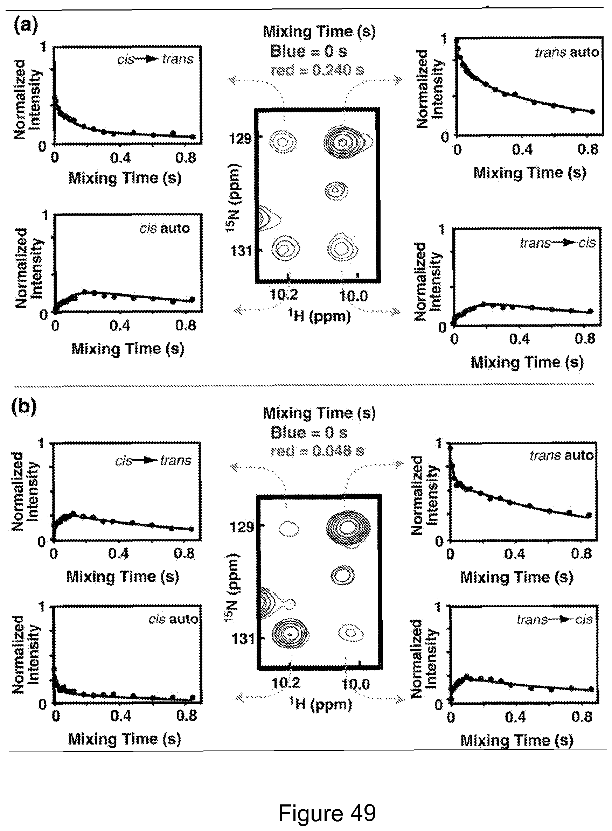

**Figure 49** is Quantifying the catalytic rate of exchange for CypA-mediated isomerization of CD147. The apparent catalytic exchange rates in Table 1 were derived from ZZ-exchange experiments using [CD147[94-214]]=0.5 mM and either (a) [CypA]=0.01 mM or (b) [CypA]=0.05 mM. The ZZ-exchange data along with the corresponding fits and two sample spectra from each experiment for CD147 Trp210[Nε1] are shown. Intensities for each of the four resonances were fit simultaneously to Eqs. (1a), (1b), (1c), and (1d). Intensities were normalized to that of the highest intensity, that is, the *trans* resonance, at no mixing time.

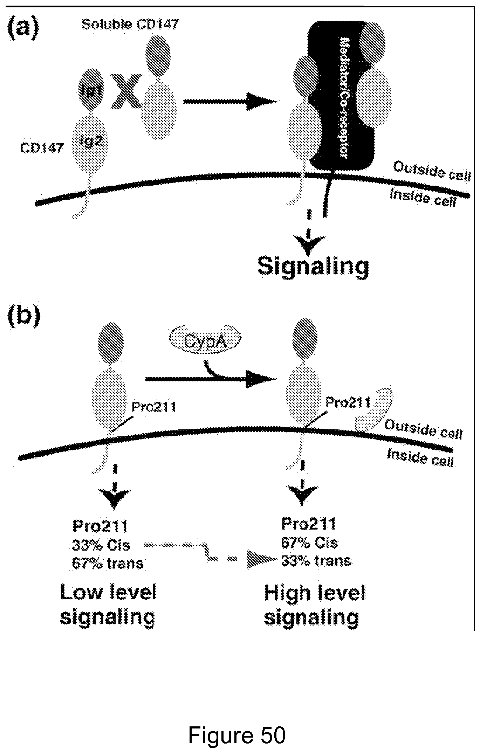

**Figure 50** is a schematic diagram of both the cyclophilin-independent and cyclophilin-dependent mechanisms of CD147 activity. (a) Based on our findings here that have shown that the extracellular region of CD147 does not self-associate, CD147 homophilic interactions are likely mediated by other molecules that potentially include TM proteins (i.e., co-receptors). (b) The direct observation of CypA mediated isomerization of Pro211 suggests a mechanism whereby CypA may alter the inherent *cis:trans* equilibrium of CD147 Pro211. The inherent *cis:trans* ratio of CD147 Pro211 was calculated from the relative peak intensities of the amides of residues 207-214 within [15N]HSQC spectra, and the *cis:trans* ratio in the active complex is based on previous studies with a model peptide substrate.(55)

**Figure 51** illustrates that extracellular EMMPRIN is a potent stimulator of MMP/cytokine secretion. A) The recombinant EMMPRIN ectodomain targets unknown cellular proteins that will be identified here. B) ELISA-based assays measure secretion of TNF-$\alpha$ and IL-1$\beta$ from THP-1 cells in a dose dependent manner. C) MMP/cytokine array data provide a broad means of identifying/comparing secretion due to 5 $\mu$g recombinant EMMPRIN from U937 cells.

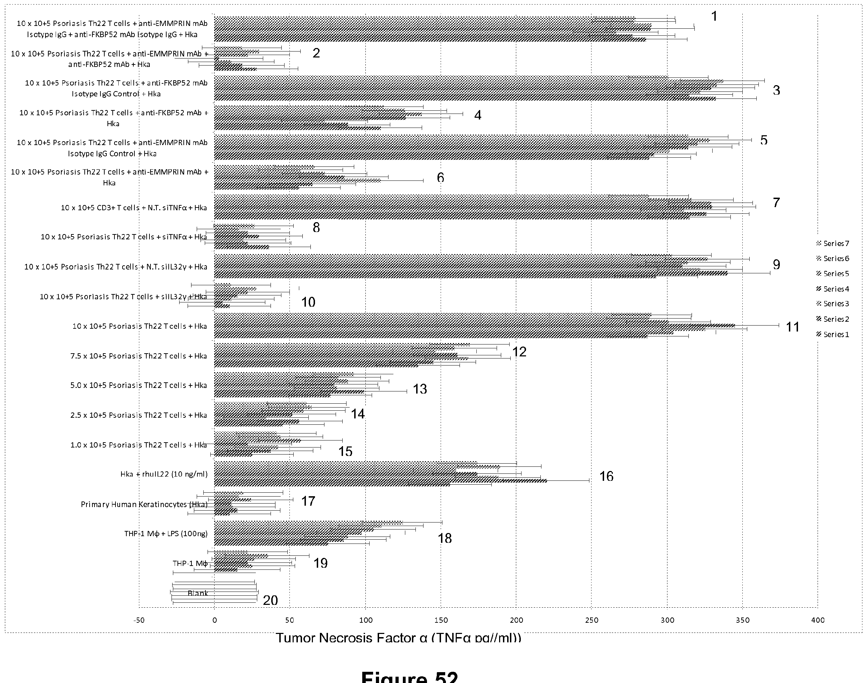

**Figure 52** is a graph showing significant reversal of TNF$\alpha$ production in primary human keratinocytes induced by Psoriatic Th22 Autoreactive T cells by anti-EMMPRIN monoclonal antibodies, anti-FKBP52 monoclonal antibodies, or dual monoclonal antibodies to block FKBP52 and EMMPRIN. The following groups were analyzed, which correspond to their numbers on the graph: (1) 10 x 10+5 Psoriasis Th22 T cells + anti-EMMPRIN mAb Isotype IgG + anti-FKBP52 mAb Isotype IgG + Hka; (2) 10 x 10+5 Psoriasis Th22 T cells + anti-EMMPRIN mAb + anti-FKBP52 mAb + Hka; (3) 10 x 10+5 Psoriasis Th22 T cells + anti-FKBP52 mAb Isotype IgG Control + Hka; (4) 10 x 10+5 Psoriasis Th22 T cells + anti-FKBP52 mAb + Hka; (5) 10 x 10+5 Psoriasis Th22 T cells + anti-EMMPRIN mAb Isotype IgG Control + Hka; (6) 10 x 10+5 Psoriasis Th22 T cells + anti-EMMPRIN mAb + Hka; (7) 10 x 10+5 CD3+ T cells + N.T. siTNFα + Hka; (8) 10 x 10+5 Psoriasis Th22 T cells + siTNFα + Hka; (9) 10 x 10+5 PsoriasisTh22 T cells + N.T. silL32γ + Hka; (10) 10 x 10+5 Psoriasis Th22 T cells + silL32γ + Hka; (11) 10x 10+5 Psoriasis Th22 T cells + Hka; (12) 7.5 x 10+5 Psoriasis Th22 T cells + Hka; (13) 5.0 x 10+5 Psoriasis Th22 T cells + Hka; (14) 2.5 x 10+5 Psoriasis Th22 T cells + Hka; (15) 1.0 x 10+5 Psoriasis Th22 T cells + Hka; (16) Hka + rhuIL22 (10 ng/ml); (17) Primary Human Keratinocytes (Hka); (18) THP-1 Mφ + LPS (100ng); (19) THP-1 Mφ; and (20) Blank.

## DETAILED DESCRIPTION

Methods for inhibiting a chronic inflammatory response and compositions to carry out such methods are **described** herein. The methods are directed toward selectively inhibiting an adaptive immune response, without significantly affecting an innate immune response. The methods for inhibiting a chronic inflammatory response are directed toward inhibiting proinflammatory cytokine production.

One approach that may be used to selectively target the adaptive immune system is to focus on activated T cell communication with macrophages (Mφ). When an activated T cell interacts with a Mφ, a series of proinflammatory cytokines are released in response to cell-to-cell contact at the immune synapse (IS) through one or more costimulatory molecules. Several proinflammatory cytokines are implicated in chronic inflammation and include, but are not limited to IL-1α, IL-1β, IL-6, LIF, IFN-γ, OSM, CNTF, TGF-β, GM-CSF, IL-11, IL-12, IL:18, IL-8, IL-32γ and TNFα. One or more of these proinflammatory cytokines can be measured to determine whether an immune response is activated or suppressed.

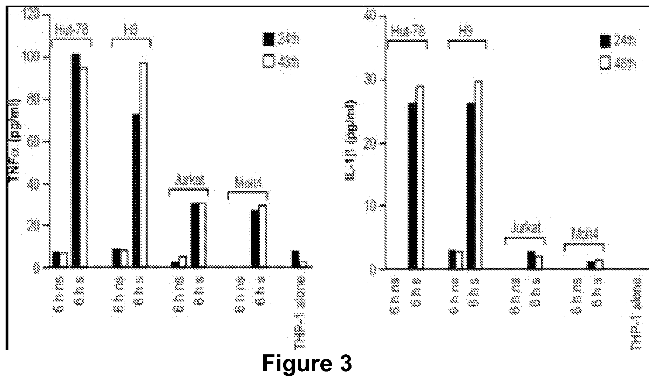

Production of the cytokines TNFα, IL-1, and IL-32γ are strongly induced in human primary Mφ and THP-1 Mφ by direct contact with primary human CD3+ T cells, human T cell lymphoma HUT-78 cells or human T cell H9 cells that are stimulated with PHA/PMA, PMA/ionomycin, αCD3/aCD28 or IL-12/IL-18 (see, e.g., Figure 2). However, cytokine production was barely induced in stimulated Molt4m, Jurkat and Raji human T-cells and was non-induced in resting primary human T cells, unstimulated HUT-78, H9, Molt4, Jurkat or Raji cells (see, e.g., Figure 3). Consequently, that these pathways appear to be mediated via direct cell-cell contact through an immune synapse (IS) mechanism in chronic inflammatory diseases such as psoriasis. These pathways appear to be mediated by T cell costimulatory molecules that are upregulated on or near the surface of the T cells during activation. Since these molecules are upregulated only or mostly only during T cell activation, these molecules represent a promising target for selectively inhibiting the adaptive immune system while leaving the innate immune system intact.

Activated T-cells express numerous membrane-bound costimulatory proteins that are instrumental to cell-cell contact signaling cascades leading to proinflammatory cytokine induction. These molecules are referred to herein as T cell Cytokine Inducing Surface Molecules (TCISMs). TCISMs provide a link between T cell activation and Mφ-driven cytokine upregulation during an adaptive immune response. Compositions that selectively inhibit the adaptive immune system may include TCISM inhibitors. Examples of TCISMs include, but are not limited to, CD40 Ligand (CD40L) members of the FKBP Multigene Family (e.g., FKBP52), cyclophilins (e.g., cyclophilin A) and CD147 (also known as Extracellular matrix metalloproteinase inducer, or EMMPRIN). Additional examples of TCISMs can be found in International Application No. PCT/US2008/065992, filed June 5, 2008.

CD147, or "EMMPRIN" is part of the EMMPRIN Immunoglobulin Receptor Superfamily, is a type-I transmembrane glycoprotein expressed in nearly every cell type including fibroblasts, platelets, and T-cells and tumor cells where its biological roles include the regulation of multiple protein families. (1-3) For example, CD147 stimulates the secretion of numerous matrix metalloproteinases (MMPs) that are responsible for the reconstruction of the extracellular matrix (4) and CD147 has been shown to stimulate secretion of several pro-inflammatory cytokines. (2, 4) CD147 is overexpressed in inflammatory disorders such as rheumatoid arthritis (RA) where CD147-mediated MMP secretion leads to the destruction of joint tissue. (12,13)

Although CD147 is well-known for its ability to stimulate the secretion of MMPs, its role as a TCISM was not previously known (see Example 1 below). Further, it was previously thought that CD147 acts as its own receptor, whereby either membrane-bound or soluble CD147 participates in homophilic interactions with membrane-bound CD147 to stimulate intracellular signaling (14,15). However, many CD147 protein interactions have emerged, suggesting that CD147-mediated signaling may be orchestrated by other proteins. Such protein interactions may include integrins, syndecan-1, and the recently identified enzyme ligands of CD147: the cyclophilin class of peptidyl-prolyl isomerases. (16-18)

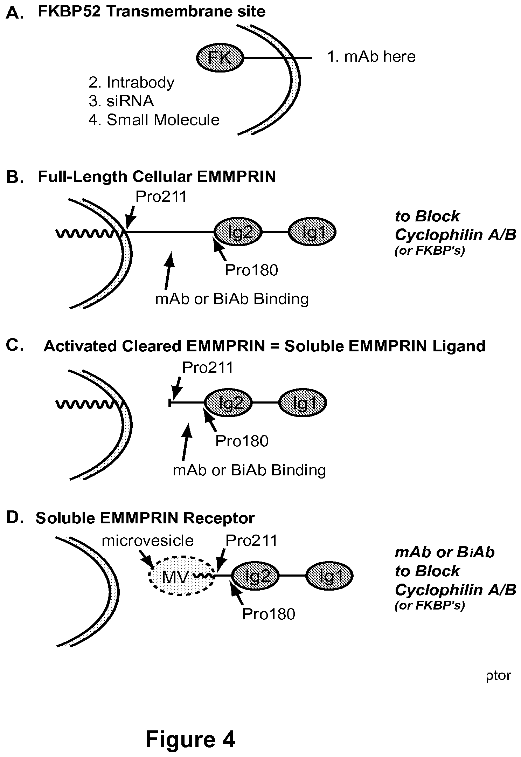

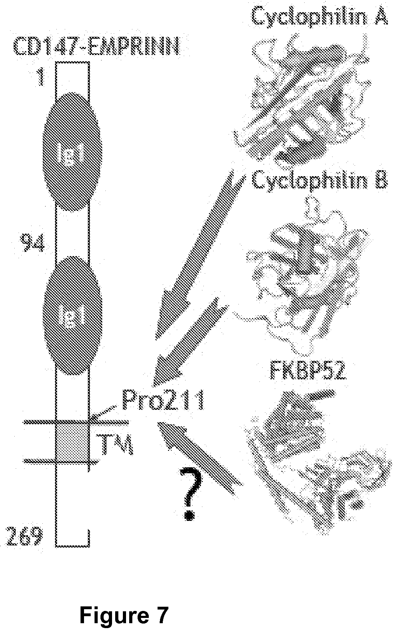

The primary transcript of CD147 comprises two immunoglobulin-like (Ig-like) domain, called EMMPRIN Ig1-Ig2. As discussed herein, CD147 can stimulate proinflammatory cytokine release, and may associate with members of the immunophilin/cyclophilin superfamily, FKBP52, cyclophilin A (CypA) and cyclophilin B (CypB) (Figure 7). EMMPRIN is expressed in several different forms, including full length EMMPRIN, which is attached to the cell membrane, and two soluble forms: microvesicle-associated soluble EMMPRIN Receptor, and a modified, soluble EMMPRIN Ligand (see Figure4).

FK506-binding protein 52 (FKBP52) (also designated as FKBP59, p59, or Hsp56}, coded by the *FKBP4* gene, is a member of the FKBP family of immunophilins. FKBPs are characterized by their ability to catalyze the *cis-trans* isomerization of *cis*-peptidyl-prolyl bonds, as well as by their strong affinity to the immunosuppressive drug FK-506. FKBP52 displays a modular organization with the immunophilin character of the protein attributed to the NH2-terminal part of the protein.

Cyclophilins are members of the immunophilin superfamily, bind to CD147, and are ubiquitously expressed enzymes that catalyze isomerization of peptidyl-prolyl bonds and regulate a diverse array of protein functions. For example, analogous to methylation and phosphorylation, cyclophilin-mediated isomerization is thought to be the underlying regulatory phenomena of several signal transduction pathways. (19) Similar to their CD147 receptor, cyclophilins are highly expressed in multiple cancers and inflammatory disorders such as RA. (20-21) It was within the synovial fluid of RA patients that the prototypical cyclophilin family member, cyclophilin A (CypA), was first identified as an extracellular protein. (22) Later studies showed that CD147 functions as a signaling receptor for extracellular CypA as well as another cyclophilin, cyclophilin B (CypB). (23, 24) Extracellular cyclophilins exhibit cytokine-like properties and mediate numerous intracellular events through their interactions with the CD147 receptor (18,25) (Fig. 1,mechanism 2). For example, CypA displays potent chemotactic activity that is dependent on its interaction with CD147(24) and inhibition of the CypA/CD147 interaction greatly decreases migration of immune cells to the sites of inflammation in mouse models of such diseases as acute lung injury, asthma, and RA (26-28). Many viruses utilize host cell CypA for infection, including vesicular stomatitis virus, vaccinia virus, human immunodeficiency virus type 1 (HIV-1), and severe acute respiratory syndrome (SARS). However, a subset of these viruses has been shown to rely on host cell virus-associated CypA that is "hijacked" from a previous host cell anchored within the viral membrane has been proposed to increase infection by targeting CD147 on a new host cell. (29-32) In support of such a proposal, specifically blocking CypA/CD147 interactions has been shown to inhibit infection in cell culture. (32, 33) Therefore, understanding the molecular mechanisms of cyclophilin/CD147 interactions may have therapeutic value with respect to therapies for chronic inflammatory diseases (including cancer) and viral infection.

As described above and further in the Examples below, cyclophilins are not merely ligands but are also enzymes that may play a catalytic role in cyclophilin/CD147 signaling. These active enzyme/receptor complexes can be probed during catalysis, to directly identify the cyclophilin-induced conformational changes imparted onto the CD147 receptor that are critical for signaling as opposed to inferring them from static structures. Thus, the structural regions of CD147 responsible for its ligand independent activity can be determined and the cyclophilin-induced conformational changes that lead to intracellular signaling can be probed.

Methods for inhibiting an adaptive immune response or inhibiting proinflammatory cytokines may be accomplished by administering a composition that may include one or more inhibitors for the following targets: CD147 and FKBP52. In an embodiment, the composition may include two or more inhibitors for simultaneous dual inhibition of CD147 and FKBP52. Inhibition may be carried out by administering any suitable inhibitor that results in a reduction in activity or expression of the target of inhibition. Possible target sites for a representative inhibitor of CD147 or FKBP52 are shown in Figure 4.

According to the present invention, inhibition of proinflammatory cytokine release may be accomplished through RNA interference (RNAi) by contacting a cell with a small nucleic acid molecule, such as a short interfering nucleic acid (siNA), a short interfering RNA (siRNA), a double-stranded RNA (dsRNA), a micro-RNA (miRNA), or a short hairpin RNA (shRNA) molecule, or modulation of expression of a small interfering RNA (siRNA) so as to provide for decreased levels of expression of CD147 and FKBP52. (e.g., through a decrease in mRNA levels and/or a decrease in polypeptide levels).

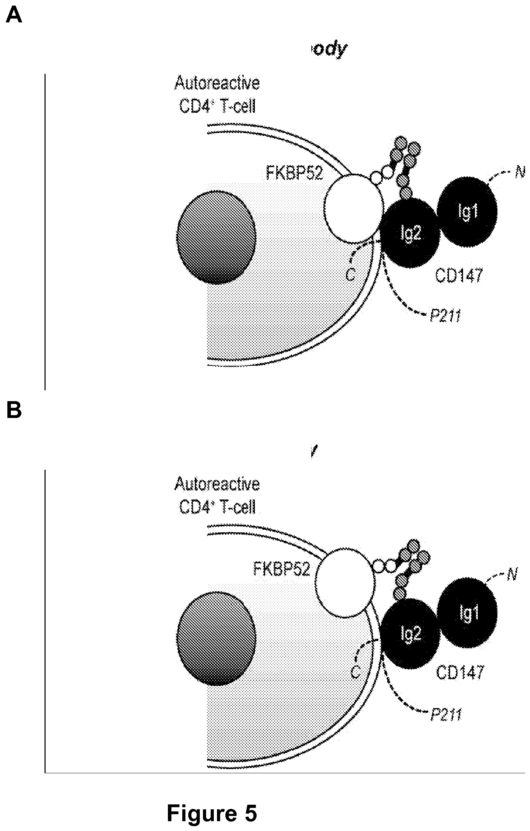

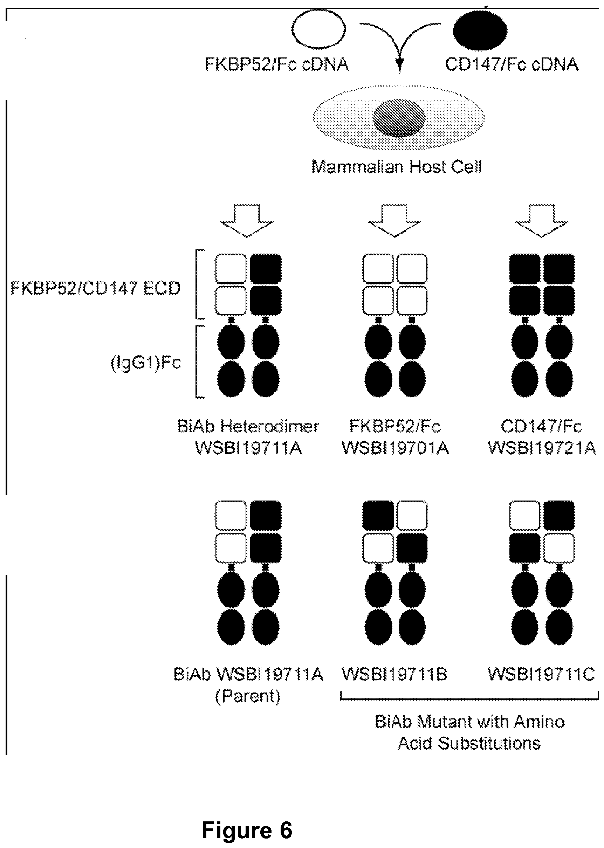

In another embodiment, inhibition of proinflammatory cytokine release may be accomplished by contacting a cell with an antibody or functional fragment thereof. An antibody or functional antibody fragment thereof refers to an immunoglobulin molecule that specifically binds to, or is immunologically reactive with a particular antigen, and includes both polyclonal and monoclonal antibodies. The term antibody includes genetically engineered or otherwise modified forms of immunoglobulins, such as intrabodies (128), chimeric antibodies, fully human antibodies, humanized antibodies, and heteroconjugate antibodies (e.g., bispecific antibodies, (127) diabodies, triabodies, and tetrabodies). The term functional antibody fragment includes antigen binding fragments of antibodies, including e.g., Fab', F(ab').sub.2, Fab, Fv, rIgG, and scFv fragments. The term scFv refers to a single chain Fv antibody in which the variable domains of the heavy chain and of the light chain of a traditional two chain antibody have been joined to form one chain. As shown in Figure 5, a bispecific antibody (bsAb) that may be used in the methods described herein may recognize (Figure 5A) or block (Figure 5b) FKBP52 and CD147 interactions on the surface of human CD4+ cells. One example of such mammalian-cell derived bsAbs and how they may be produced is shown in Figure 6.

Inhibition of proinflammatory cytokine release may also be accomplished by contacting a cell with a fusion protein. A fusion protein is a protein that is encoded by 2 or more related or unrelated fused genes or functional fragment thereof. Other suitable inhibitors may include, but are not limited to, proteins, peptides, peptibodies, chimeric proteins, and other biologics. In some embodiments, small molecules may be used as inhibitors. Examples of small molecules can be found in International Application No. PCT/US2008/065992, filed June 5, 2008.

The above mentioned compositions are for use in methods for treating a condition relating to chronic inflammation. Conditions that are related to chronic inflammation include, but are not limited to, autoimmune diseases, cancer, certain infectious diseases, and cardiovascular diseases. Specific, conditions and diseases that are related to or are associated with chronic inflammation including, but not limited to, rheumatoid arthritis (RA), multiple sclerosis, Crohn's disease, psoriasis, psoriatic arthritis, osteoarthritis, Graves disease, lupus, scleroderma, vasculitis, Sjogrens' syndrome, poly- and dermatomyositis, autoimmune polyendocrine syndromes, hereditary proteinuria syndrome, type I diabetes, systemic lupus erythematosus, primary bilary cirrhosis, autoimmune thyroiditis, hepatitis, Acquired Immunodeficiency Disease (HIV), graft versus host disease, allograftdisease, asthma, Chronic Obstructive Pulmonary Disease (COPD), chronic inflammatory bowel disease, celiac disease, chronic pelvic inflammatory disease, Chronic inflammatory polyneuropathy, neovascular glaucoma, diabetic retinopathy, retinoblastoma, retrolental fibroplasia, uveitis, retinopathy of premature macular degeneration, corneal graft neovascularization, cutaneous T-cell lymphoma, HTLV-1-associated cutaneous T-cell lymphoma, HTLV-II-associated lymphoma, hairy cell leukemia, melanoma, atherosclerosis, aortic valve stenosis, pancreatitis, allergies, and congestive heart failure. The methods of treatment are directed to autoimmune diseases that are mediated by chronic inflammation such as Rheumatoid Arthritis (RA), Chronic Plaque Psoriasis (CPPs), and Psoriatic Arthritis (PsA). These chronic inflammatory diseases are characterized by hyperproliferation of fibroblasts and synoviocytes (in RA) and keratinocytes (in CPPs/PsA) and the infiltration of activated T- cells, Mφ, and other immune cells into lesional skin and/or joints. Proinflammatory cytokines, including Tumor Necrosis Factor-a (TNFα), Interleukin-1β (IL-1β), and Interleukin-32γ (IL-32γ) are produced in these patients, and induce and maintain chronic inflammation: Up-regulation of these factors by activated immune system cells including M(p drives the pathogenesis of these disorders. It has been shown in relevant activation and the animal models that T cells play a key role in Mφ pathogenesis of autoimmune diseases. In addition to autoimmune diseases, chronic inflammation also plays a role in many cancers and cardiovascular diseases.

The method for treating a condition relating to chronic inflammation may include administering a pharmaceutical composition that simultaneously inhibits activity and/or expression of FKBP52 and CD147. The pharmaceutical composition may include, but is not limited to, an FKBP52 inhibitor, a CD147 inhibitor, and a pharmaceutically acceptable carrier.

The pharmaceutical compositions of the subject invention can be formulated according to known methods for preparing pharmaceutically useful compositions. Furthermore, as used herein, the phrase "pharmaceutically acceptable carrier" means any of the standard pharmaceutically acceptable carriers. The pharmaceutically acceptable carrier can include diluents, adjuvants, and vehicles, as well as implant carriers, and inert, non-toxic solid or liquid fillers, diluents, or encapsulating material that does not react with the active ingredients of the invention. Examples include, but are not limited to, phosphate buffered saline, physiological saline, water, and emulsions, such as oil/water emulsions. The carrier can be a solvent or dispersing medium containing, for example, ethanol, polyol (for example, glycerol, propylene glycol, liquid polyethylene glycol, and the like), suitable mixtures thereof, and vegetable oils. Formulations containing pharmaceutically acceptable carriers are described in a number of sources which are well known and readily available to those skilled in the art. For example, Remington's Pharmaceutical Sciences (Martin E W [1995] Easton Pa., Mack Publishing Company, 19th ed.) describes formulations that can be used in connection with the subject invention. Formulations suitable for parenteral administration include, for example, aqueous sterile injection solutions, which may contain antioxidants, buffers, bacteriostats, and solutes which render the formulation isotonic with the blood of the intended recipient; and aqueous and nonaqueous sterile suspensions which may include suspending agents and thickening agents. The formulations may be presented in unit-dose or multi-dose containers, for example sealed ampoules and vials, and may be stored in a freeze dried (lyophilized) condition requiring only the condition of the sterile liquid carrier, for example, water for injections, prior to use. Extemporaneous injection solutions and suspensions may be prepared from sterile powder, granules, tablets, etc. It should be understood that in addition to the ingredients particularly mentioned above, the formulations of the subject invention can include other agents conventional in the art having regard to the type of formulation in question.

The pharmaceutical composition described above is administered and dosed in accordance with good medical practice, taking into account the clinical condition of the individual patient, the site and method of administration, scheduling of administration, patient age, sex, body weight, and other factors known to medical practitioners. The therapeutically effective amount for purposes herein is thus determined by such considerations as are known in the art. For example, an effect amount of the pharmaceutical composition is that amount necessary to provide a therapeutically effective decrease in proinflammatory cytokines. The amount of the pharmaceutical composition must be effective to achieve improvement including but not limited to total prevention and to improved survival rate or more rapid recovery, or improvement or elimination of symptoms associated with the chronic inflammatory conditions being treated and other indicators as are selected as appropriate measures by those skilled in the art. In accordance with the present invention, a suitable single dose size is a dose that is capable of preventing or alleviating (reducing or eliminating) a symptom in a patient when administered one or more times over a suitable time period. One of skill in the art can readily determine appropriate single dose sizes for systemic administration based on the size of the patient and the route of administration.

**Dual inhibition of CD147 and FKBP52 act synergistically to inhibit proinflammatory cytokine release**

It is shown in the current disclosure that activated T-cells from cell lines and healthy human volunteers, as well as T-cells obtained from patients with *Psoriasis vulgaris,* induce human macrophages (MΦ) to up regulate proinflammatory cytokine production in a cell-cell contact dependent fashion, leading to a chronic inflammatory phenotype. FKBP52 is up regulated in activated T cells upon PMA/ionomycin stimulation. Additionally, CD147/EMMPRIN expression, measured by FACS, is also increased in the same T-cell populations. Experiments conducted *in vitro* using NMR demonstrate a physiological association between the two proteins. Using a validated cell-cell contact bioassay whereby human CD3+ T-cells (obtained from leukopacks) and monocyte-derived MΦ (obtained from leukopacks) are combined in a cell-cell contact manner, relatively large amounts of TNFα, IL-1β and IL-32 are produced by the activated MΦ during a 48-h period of culture.

As mentioned above, FKBP52 is an immunophilin protein with *cis/trans-prolyl isomerase* activity that functions as a protein folding chaperone for proteins containing proline residues. It also binds the immunosuppressant tacrolimus, used in treating patients suffering from autoimmune disorders. Silencing FKBP52 by using specific DHARMACON siRNA smart pool probes made to human FKBP52 did not result in any significant T-cell-driven MΦ cytokine suppression. Similarly, when specific DHARMACON siRNA smart pool probes made to human CD147 were transfected into human T cells, subsequent T-cell-mediated MΦ activation and cytokine release was only slightly decreased. However, silencing both FKBP52 and CD147 before T-cell activation *in vitro* led to a nearly 90% reduction in T-cell driven MΦ release of cytokines over a 48 h period These data indicate that the interaction of these two proteins is important for T-cell:MΦ signal transduction mechanisms during immune synapse formation, secretion of proinflammatory cytokines, and establishment of chronic inflammation and autoimmunity. Additionally, specific inhibition of these two proteins using dual inhibitors such as RNAi molecules (e.g., siRNA molecules), antibodies (e.g., monospecific or bispecific antibodies), functional antibody fragments, peptides, fusion proteins, chimeric proteins, peptibodies, intrabodies, small molecules, and/or other biologics can be used to control adaptive immunity while not significantly affecting innate immunity.

The studies above are described further in Example 1 below. FKBP52 is a T-cell cytoplasmic protein that likely interacts with the T-cell membrane protein CD147 to subsequently mediate MΦ activation through the CD147 receptor on MΦ. FKBP52 may be translocated from the cytoplasm to or near the T cell membrane. Approaches using proteomics, including multiple reaction monitoring, tandem mass spectrometry and database searching were used to elucidate the secreted protein profile following T-cell stimulation and subsequent cell-cell contact with MΦ. Further, soluble proteins secreted from activated T cells after contact with MΦ serve as highly specific drug discovery targets in psoriasis and other inflammatory skin diseases and are targets for the adaptive immune response while leaving the innate immune response substantially intact.

**Characterization of cyclophilin-mediated EMMPRIN signaling and the production of proinflammatory cytokines**

*EMMPRIN secondary structure and interactions with Cyclophilin A*. CD147 is a type I integral membrane protein containing two extracellular immunoglobulin-like (Ig) domains called Ig1 and Ig2, a single transmembrane domain (TM), and a short about 30 residue intracellular domain. Extracellular cyclophilin-A (CypA) and cyclophilin-B (CypB) target CD147 and stimulate the phosphorylation of several intracellular proteins that include ERK1/2 through an unknown mechanism. Like CD147, the cyclophilin ligands are highly expressed in cancer tissue.

The cyclophilin target site on CD147 has been identified and it has been shown that the receptor is catalyzed by its cyclophilin enzyme ligands (see Example 4). In addition, a catalytic role in cyclophilin-mediated CD147 signaling has been discovered. First, CypA is involved in several intracellular signal transduction pathways through at least two protein interactions identified to date, interleukin-2 tyrosine kinase and Crk. The downstream interactions of these proteins appear to be regulated by CypA-induced conformational changes and thus, a similar mechanism may apply to cyclophilin/CD147 interactions on the outside of the cell. Second, prolyl-isomerization is in general involved in many biological processes. For example, in filamentous phage a *cis*-to-*trans* prolyl-isomerization of a coat protein acts as a "timer" allowing for viral entry and prolyl-isomerization is also involved in the opening of an anion channel. Third, inactive cyclophilin mutants failed to activate ERK1/2 phosphorylation. Fourth, the two cyclophilin target residues on CD147, Pro180 and Pro211, can also serve as substrates since cyclophilins catalyze peptidylprolyl isomerization.

Both cyclophilin target sites on CD147, Pro180 and Pro211, can be accessible to extracellular cyclophilins. Pro180 of the extracellular CD147 region resides within the extracellular Ig2 domain and while its mutation disrupts cyclophilin interactions, this is believed to be due to a change in the structural integrity of the receptor. In contrast, Pro211 lies at the interface between the extracellular Ig2 domain and the TM domain. Both extracellular cyclophilins, CypA and CypB, readily traverse the membrane, suggesting that catalysis of this residue could induce a conformational change that leads to intracellular signaling. Interestingly, Pro211 is also utilized by Cyp60 to shuttle the receptor to the cell surface, and thus, CD147 interacts with cyclophilins both intracellularly and extracellularly. However, multiple mechanisms exist for CD147 cell-surface expression, since broad-spectrum inhibitors of cyclophilins lead to a reduction, and not complete abrogation, of cell-surface CD147. The conformational changes that occur upon cyclophilin/CD147 complex formation and catalysis can be identified using methods disclosed herein at atomic resolution.

It is known that targeting CD147 and/or cyclophilins has an effect on metastasis. For example, RNA interference (RNAi) of CD147 in both melanoma and ovarian cancer decreases cellular invasiveness *in vitro* as well as tumor size in mouse models. Anti-CD147 antibody blocks proliferation of pancreatic cancer cells and RNAi of CypA diminishes tumor growth in non-small cell lung cancer. Considering that CD147 is present in the vast majority of tumors where it induces the secretion of multiple MMPs and pro-inflammatory cytokines, blocking CD147 would likely inhibit tumor development. Thus, CD147 may be useful as a therapeutic target for multiple cancers.

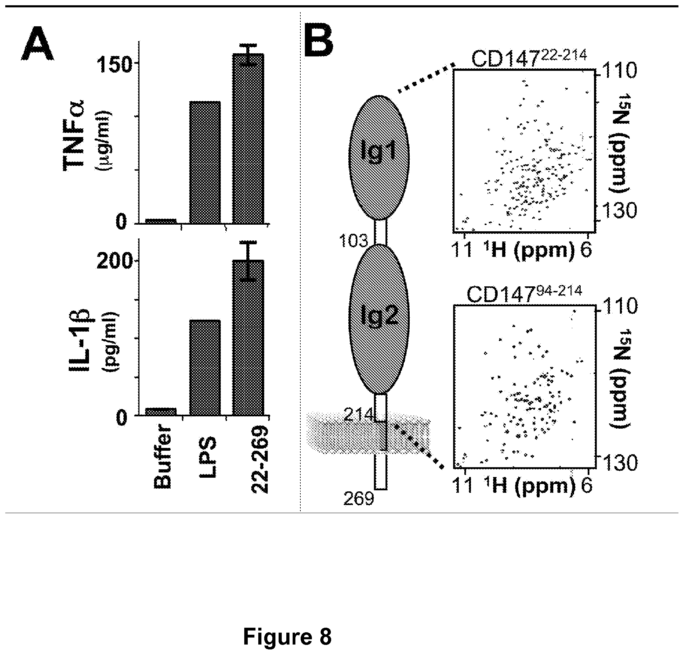

*EMMPRIN stimulates cytokine release and can be studied in solution.* Multiple CD147 constructs have been expressed and purified in milligram quantities and the full-length protein has been found and is both soluble and active. Briefly, activity of recombinant CD147$^{22-269}$, lacking only the signal peptide, was confirmed by its ability to augment secretion of pro-inflammatory cytokines in cell culture that include TNFα and IL-1β (Figure 8A). While full-length CD147 is soluble, it has been found that the transmembrane domain induces the formation of large aggregates as assessed by gel-filtration. This soluble form of CD147 is biologically relevant, since tumor cells secrete CD147 in its entirety both within the presence and absence of microvesicles. Like membrane-bound CD147, it has been determined that soluble CD147 contributes to tumor growth by inducing MMPs and pro-inflammatory cytokine secretion (Figure 8A). In addition, the CD147 residues that dictate both MMP and proinflammatory cytokine induction have been determined.

The entire extracellular region (CD147$^{22-214}$) and the Ig2 domain alone (CD147$^{94-214}$) are well folded as shown by the NMR solution spectra (Figure 8B). No concentration-dependent chemical shifts were observed that suggests a lack of intermolecular interactions and this is further probed by the dynamics studies detailed below. This lack in intermolecular interactions also suggests that CD147 homophilic interactions in cell culture and *in vivo* can be modulated by other molecules such as, for example, cadherin dimerization, that is modulated by calcium or fibroblast growth factor receptor interactions that are mediated by heparins. For CD147, these small molecules do not induce significant self-association (data not shown), but these findings are an important step in identifying the surfaces of CD147 that dictate MMP and cytokine induction. Thus, methods disclosed herein can be used as a tool to identify the specific residues responsible for its activities that play a critical role during tumor development.

*The extracellular region of CD147 alone does NOT self-associate.* An advantage of NMR solution studies is that both the dynamics and structure of macromolecules can be studied simultaneously and can be used to probe CD147 self-association. It has been suggested that CD147 acts as its own receptor analogous to other cellular adhesion receptors and this interaction is likely of weak affinity. NMR relaxation experiments can be used as sensitive markers of such protein/protein interactions on multiple timescales and are therefore well suited to probe interactions with various affinities. Specifically, R1 (longitudinal) and R2 (transverse) relaxation rates provide the high resolution details of the dynamics of each residue and, thus, if the extracellular region of CD147 self-associates with millimolar affinity (or tighter) then this will be observed by NMR solution studies.

NMR relaxation measurements indicate that the two CD147 extracellular domains, Ig1 and Ig2, neither interact with each other nor self-associate in any significant amount. Using the R2/R1 ratio that defines the correlation time of each amide, CD147 residues 22-101 and residues 105-205 were found to be independently rotating domains exhibiting correlation times that differed by nearly 3 ns, consistent with their difference in size (Figure 9A). This is immediately apparent in the average R2 rates that are about 22 Hz for residues 22-101 (Ig1) and 35 Hz for residues 104-205 (Ig2). While these dynamics studies are consistent with the two independently folded domains found within the recent X-ray crystal structure, the fact that these domains tumble with different correlation times also provides direct evidence that the crystal contacts are not maintained within solution. Consistent with these dynamics findings, no noticeable chemical shift changes were observed upon a titration of $^{15}$N-CD147$^{94-205}$ with CD147$^{22-103}$, thereby proving that these two domains do not interact as separate entities (data not shown). Moreover, no concentration dependent chemical shift changes are observed for CD147$^{22-205}$ that would be expected to occur if there were even weak interactions (i.e. $K_d$ >1 mM). Finally, the R2/R1 derived correlation times for each domain and the observed line widths are completely consistent with monomeric proteins. Thus, if these domains of CD147 self-associate *in vivo* then this likely occurs in the presence of either a co-receptor or small molecule.

Dynamics studies also provide important insight into residues 94-101 that comprise both of the predominant CD147 isoforms, isoform-2 that accounts for ~98% of CD147 (residues 1-269 that contain Ig1 and Ig2) and isoform-3 (residues 94-269 and contains only Ig2). Namely, residues 94-101 form a β-strand only in the context of Ig1, but these residues are disordered within the shorter extracellular region of the naturally occurring isoform-3 (Fig. 9B). Thus, biophysical studies of CD147 have allowed probing of the extracellular region of both dominant isoforms that have been found to play a role in cancer progression.

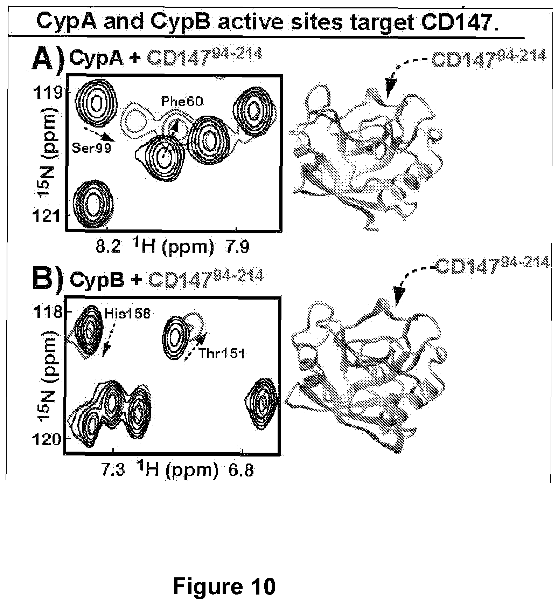

*Cyclophilins engage CD147 through their active sites.* To determine how cyclophilins interact with their receptor, the cyclophilin interaction surfaces have been identified using NMR solution studies (Fig. 10). To this end, all of the backbone resonance assignments for CypB have been determined using standard NMR methods. Titration experiments have revealed that both CypA (Fig. 10A) and CypB (Fig. 10B) target CD147 exclusively through their active sites, supporting a catalytic role in cyclophilin/CD147 cell signaling. Furthermore, the observation of a single averaged set of cyclophilin resonances during these titrations indicates that the cyclophilin/CD147 affinities are within the high micromolar range. Many ligand/receptor interactions are far weaker than the cyclophilin/CD147 binding that is characterized here (e.g., alcohols engage their receptors with millimolar affinities).

*CD147 Pro211 exists in a slowly interconverting equilibrium and is catalyzed by Cyclophilin A.* CD147 residues 206-214 exist in a *cis:trans* equilibrium dictated by Pro211 and this equilibrium is altered upon CypA catalysis. While several previous reports have postulated that both CD147 Pro180 and Pro211 may be targeted by cyclophilins, these binding sites have only been probed within the context of small peptides and not within the folded protein. The current disclosure provides the first direct evidence that shows that cyclophilins exclusively target CD147 Pro211, and not Pro180, and that CypA catalyzes peptidyl-prolyl isomerization of only this Pro211 target site.

CD147 residues 206-214 lie proximal to the transmembrane domain and exhibit two sets of resonances that indicate this region is interconverting between two conformations on the timescale of seconds. An example of one such observed set of two resonances is shown for W210 that immediately precedes the catalyzed Pro211 (Fig. 11A). Mutation of the central proline to an alanine (i.e., P211A) resulted in only one set of resonances, i.e., the *trans* resonances, confirming that a *cis:trans* equilibrium of Pro211 was the underlying cause for this observed conformational heterogeneity. Proline residues are the only amino acid that can adopt both a *cis* and *trans* conformation, yet this phenomenon is observed in only a subset of proteins that slowly interconvert between the two conformations. Thus, CD147 is a unique receptor in that it exhibits two dominant conformations adjacent to the transmembrane region. Moreover, the relative resonance intensities are indicative of the relative populations sampled and thus, based on such intensities CD147 residues 206-214 exhibit a *cis:trans* ratio of 33%:67% (~33% in *cis,* 67% in *trans,* $K_{eq,free}=33/67=0.5$) in the absence of its cyclophilin enzyme ligands.

CypA enhances the *cis:trans* interconversion of Pro211 as evidenced by the appearance of "exchange peaks" that are only observed in the presence of the enzyme (Fig. 11B, black arrows). These exchange peaks arise only if there is enough time for both *cis* and *trans* conformers to be sampled within the timeframe of the NMR experiment. In other words, this *cis/trans* interconversion is too slow in the absence of the enzyme to observe exchange peaks, but CypA-mediated catalysis enhances their interconversion proving directly for the first time that a cyclophilin catalyzes its CD147 receptor. Thus, once again CD147 exhibits a unique property in that its ligands are also enzymes that catalyze proline isomerization of their target site.

These results suggest that the CD147 *cis:trans* ratio is altered by its cyclophilin ligands, and that this may be the link between extracellular cyclophilins and CD147 intracellular signaling. Evidence for this is seen in the increased loss of intensities of the *cis* resonances relative the to *trans* resonances of CD147 upon addition of CypA (Fig. 11C and D). This is referred to as "line-broadening" and it is due to both binding and catalysis. In effect, CD147 is similar to a "volume control" that regulates intracellular signal transduction. In the present model, CD147 is always on (i.e., low level signaling) yet cyclophilins "turn up the volume" (increase signaling) by way of increasing the *cis* conformation, i.e., "a conformational switch".

*Characterizing CypA/CD147 binding and catalysis.* The present disclosure goes beyond structural studies to characterize both binding and catalysis within the CypA/CD147 complex. Cyclophilins have been shown to differ in their catalytic activities for model substrates and if biophysical characterizations reveal catalytic differences for their CD147 receptor substrate then comparative studies in cell culture is expected to mirror such differences. In other words, if different cyclophilins exhibit different equilibrium constants of their CD147 P211 target site, then there will be differences in their activation of intracellular signaling. Of course this is also dependent on their relative affinities.

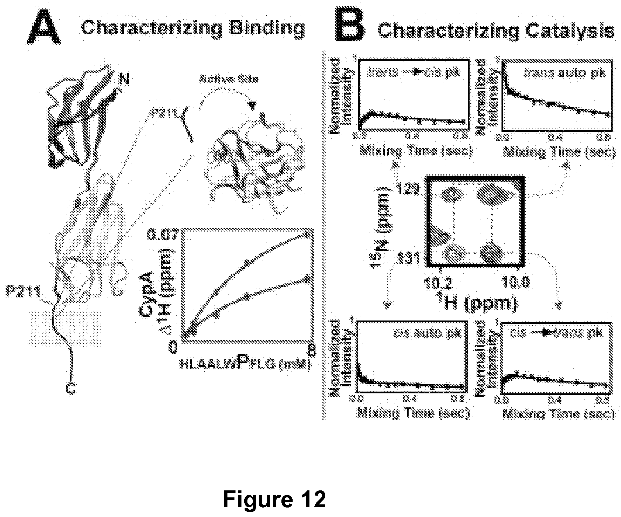

To quantify the binding of CypA to CD147 P211, a peptide has been produced that mimics this target site. This CD147 peptide was produced because it was not possible to determine the affinity of CypA for this site in the context of CD147 constructs due to the millimolar affinity requirement for such high concentrations of the receptor. This has proven a general problem for characterizing cyclophilin interactions since the transient nature and low affinity to their substrates often makes it difficult to even identify many cyclophilin targets. This is illustrated in the viral world where many viruses are dependent on host cell cyclophilins for infection, yet identifying the specific viral proteins that target host cell cyclophilins has proven illusive (i.e., "pull-down" experiments are difficult with millimolar affinities). This peptide binds within the active site of CypA just as it does in the context of larger CD147 constructs (Fig. 12A) with an associated $K_D$~5 mM. Thus, CypA engages CD147 weakly but specifically.

Cyclophilin-mediated catalysis of CD147 has been quantified by using a technique called "ZZ-exchange" (Figure 12B). Briefly, the exchange peaks of CD147 observed with catalytic amounts of CypA can be used to quantify catalysis since these provide the explicit rate constants of the *cis→trans* and *trans→cis* interconversion, $k_{ct}$ and $k_{tc}$, respectively. A varied mixing time (i.e., 32 relaxation period) is arrayed and the modulation in all peak intensities that include the *trans, cis, cis→trans,* and *trans→cis* peaks are simultaneously fit to extract the apparent catalytic rates. Although the CD147 peptide described above can be used, a CD147 construct that more accurately represents the receptor, CD147[94-214] was used for the experiments described herein. ZZ-exchange does not require the higher concentrations needed above to obtain the full binding isotherm and there are only a few resonances that overlap with the observed exchange peaks that result from CypA-mediated catalysis. With a CypA:CD147 ratio of 1:10, both catalytic rates were determined as $k_{ct}$ =40 s$^{-1}$ and $k_{tc}$ =20 s$^{-1}$, noting that NMR provides both of these rates with high pre*cision* while classic UV-assays cannot. An uncertainty of less than 5 s$^{-1}$ for these measurements was obtained by independently fitting each of the four peaks and illustrates the high quality of this data. While these are apparent catalytic rates that are dependent on the relative enzyme concentrations, ZZ-exchange measurements were collected at multiple CypA:CD147 ratios to extrapolate the true catalytic rate constants at saturating concentrations. This data already provides valuable insight into the catalytic efficiency of CypA for CD147 described below.

From the ZZ-exchange data, it can be seen that CypA-mediated catalysis of CD147 is remarkably efficient relative to model peptide substrates. When describing a reversible process such as cyclophilin-mediated isomerization, the total rate of exchange is a useful parameter to compare catalytic efficiencies and is defined as $k_{ex}$ (=$k_{ct}$+$k_{tc}$). Using the calculated $K_D$~5 mM determined from the peptide binding (Fig. 12A) and a $k_{ex}$ ~60 s$^{-1}$ under the substoichiometric CypA concentrations used above for ZZ-exchange (Fig. 12B), a $k_{ex}$ can be roughly extrapolated under stoichiometric conditions. Only 1% of CD147$^{94\text{-}214}$ is bound to CypA under these conditions and, thus, $k_{ex}$ ~6000 s$^{-1}$ (=100*60 s$^{-1}$) for a 1:1 complex. This rate of isomerization is slightly higher than that of model peptide substrates previously measured and corresponds to a CypA-mediated rate enhancement of the uncatalyzed exchange rate of approximately four orders of magnitude. The explicit rate-enhancement is more accurately extrapolated using multiple CypA: CD147$^{94\text{-}214}$ ratios along with a comparative analysis of CypB-mediated catalysis. As can be seen, CD147 is efficiently catalyzed by its major cyclophilin enzyme ligand, CypA.

It is possible that a cyclophilin-mediated peptidyl-prolyl "conformation switch" is a general phenomenon. For example, the only other human cyclophilin substrate studied to date is the intracellular interleukin-2 tyrosine kinase (Itk), and it too exhibits a similar inherent *cis:trans* equilibrium similar to CD147. Thus, cyclophilin-mediated catalysis may regulate signal transduction events both inside and outside the cell via a common mechanism. Moreover, deregulation of cyclophilins, their intracellular Itk target, or their extracellular CD147 target, all lead to cancer progression. Accordingly, some aspects of the disclosure show the atomic resolution details of such pathways to discern how these proteins interact and provide a foundation for rational-based therapeutic intervention.

*Dual inhibition of CD147 and FKBP52 inhibits proinflammatory cytokine release from keratinocytes*

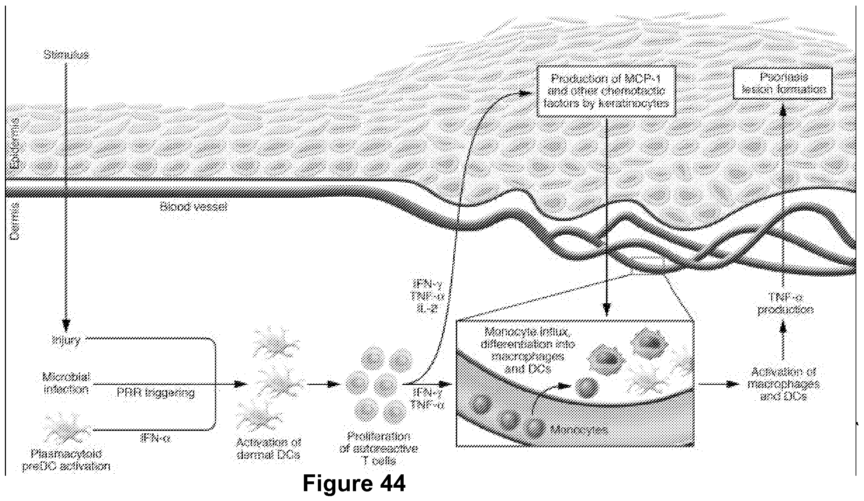

Psoriasis is a result of a complex interaction between hyperplastic keratinocytes, dendritic cells, neutrophils, memory T-cells, and M$\varphi$ (Figure 44). IL-32 (originally termed natural killer cell transcript 4 (NK4)) is a proinflammatory cytokine recognized to be associated with inflammation in humans and has been demonstrated to be associated with disease severity in diseases like Crohn's disease, ulcerative colitis, RA, COPD and psoriasis. IL-32 activates typical cytokine signal pathways of nuclear factor-kappa B (NF-$\kappa$B) and p38 mitogen-activated protein kinase for inducing IL-1$\beta$, IL- 6, IL-8, IL-12 and TNF$\alpha$. IL-32 with NOD1/NOD2 (nucleotide oligomerization domain) ligands activates the Caspase 1 pathway for inducing IL-1$\beta$ and IL.6. Further, IL-32 with Proteinase 3 induces MIP2 and IL-8.

Stimulated and unstimulated subpopulations of T cells from human whole blood were measured for their ability to stimulate cytokine production after indirect and direct cell-cell contact with primary human keratinocytes (Hka). Induction of IL-32$\gamma$ and its isoform IL-32$\gamma$ was detected in Hka lysates, but not supernatants, after stimulation with rhuTNF$\alpha$ or rhuIL-1$\beta$, ECL, and qRT-PCR results demonstrated that IL-32$\gamma$, as well as TNF$\alpha$ and IL-1$\beta$, were significantly *(P<0.05)* increased in psoriatic skin compared to healthy human skin. Further, both human H9 and CLA+ antigen positive CD4+CD45RO+CCR4+ Th22 T cells stimulate Hka through direct cell-cell contact mechanisms to up-regulate IL-32y, TNF$\alpha$, and IL-1$\beta$ at significantly (P<0.05) increased levels compared to non-stimulated H9 T cells or CD4+CD45RO+CCR4-, CD4+CD45RA+CCR4+, and CD4+CD45RA+CCR4- memory T-cells. These findings (further discussed in Example 2 below) demonstrate for the first time that the proinflammatory cytokine IL-32$\gamma$ is up-regulated in HKa after T cell:Hka cell-cell contact and is likely involved in the early stages of the pathogenesis of psoriasis.

Further, when monoclonal antibodies are used to block FKBP52 and/or CD147 in psoriatic Th22 Tcells, cytokine pr6duction by primary human keratinocytes is significantly inhibited, as shown in Figure 52. This shows that both single and dual inhibition may have important therapeutic implications for psoriasis, Rheumatic diseases and other inflammatory diseases.

The examples are set forth to aid in understanding the invention but are not intended to, and should not be construed to limit its scope in any way. For example, many of the examples described herein relate to the pathophysiology and treatment of chronic plaque, but one skilled in the art would recognize that the examples may also apply to other conditions that are related to chronic inflammation, such as those conditions discussed above. The examples do not include detailed descriptions of conventional methods. Such methods are well known to those of ordinary skill in the art and are described in numerous publications.

**EXAMPLES**

**Example 1: Simultaneous silencing of FKBP52 and EMMPRIN significantly reduces cytokine production**

Human T-cell lymphoma H9 cells were stimulated with PMA/Ionomycin. Cells were lysed and proteins fractionated using a commercially available membrane protein extraction kit, then separated by 2D gel electrophoresis. Protein spots showing greater than 2-fold change between stimulated and unstimulated samples were *excised,* digested with trypsin, and analyzed by nano LC/MS/MS. Western blots, siRNA knockdown, and a cell-cell contact bioassay were employed to validate protein identification, quantitation, and function.

Materials and methods

*Patients, materials, and methods.* A statement that the research was approved by the relevant institutional review boards or ethics committees and that all human participants gave written informed consent.

*Culture of cell lines.* The human cell lines were cultured in a standard medium consisting of RPMI 1640 (Biochrom, Berlin, Germany) supplemented with 10% (v/v) FCS serum, 2 mM L-glutamine, 100 units/ml penicillin and 100 units/ml streptomycin. Hut78, H9, Molt4, Jurkat, Raji and THP-1 cell lines were obtained from the ATCC.

*Cell isolation of peripheral blood mononuclear cells (PBMCs).* PBMCs were isolated by Ficoll density gradient centrifugation. The viability of obtained PBMCs was always >95%, as determined by trypan blue staining. The viable cells were quantified in a Neubauer chamber (Zeiss, Oberkochen, Germany) and stored in liquid nitrogen.

*CD3+ T cells.* Centrifuge the thawed PBMC again at 1,500 rpm for 5 minutes, then the supernatant was discarded. The pellet was then resuspended in MACS buffer and the cells were disturbed into single cell suspension. The cells were suspended in 0.8ml buffer per $10^8$ cells. 0.2ml Hapten-Antibody Cocktail was added per $10^8$ cells. The cells and Antibody Cocktail were mixed well and incubated for 20 minutes on ice. The cells were then washed by adding 20x the labeling volume, centrifuged and the supernatant removed. The cell pellet was resuspended in 0.8ml of buffer per $10^8$ cells. 0.2ml MACS Anti-Hapten MicroBeads per $10^8$ cells was added to label the cell magnetically, then the mixture was mixed well and incubated for 15 minutes on ice. The mixture was washed with 20x of the volume (If using frozen cells from Leukophoresis packs, pass cells through a 45 $\mu$m mesh filter to remove clustered dead cells), centrifuged and then the supernatant was removed completely. The cells were then resuspended in 1 ml of MACS buffer per $10^8$ cells. The LS+ column was placed in the magnetic field of an appropriate MACS separator and the column was prepared by washing with 3ml of buffer. The cell suspension was applied on the column; letting the unlabeled cells pass through. The effluent was collected as negative fraction, representing the enriched T cell fraction. The column was rinsed with 4 x 3ml of buffer, collecting the effluent. Columns were then centrifuged and the supernatant removed, and the cell pellet was resuspended in 50 ml tissue culture medium. Cells were counted and a suspension of $10^6$ cells/ml was made. These are the purified T cells with above 95% purity. Cell purity can be determined by CD3-FITC labeling and FACS analysis. For monocytes, the method is the same as discussed above for the CD3+ T cell separation.

*Immunophenotyping of the cells.* Harvested cells were washed in FACS medium [ phosphate buffered saline (PBS) containing 1% bovine serum albumin (BSA)] and stained at 4°C for 20 min by antibodies directly conjugated with Fluorescein isothiocyanate (FITC) or phycoerythrin (PE). Thereafter cells were washed three times with PBS and analyzed by FACScan (Becton Dickinson, Heidelberg, Germany) using the CellQuest software (Becton Dickinson). Antibodies were the following: PE-labeled anti-mouse IgG, anti-human CD40L and CD147.

*Cell-cell contact assay* Primary T cells or H9 cells are washed with cold PBS and cell pellets are resuspended in freshly made 1% paraformaldehyde at $5\times10^6$ cells/ml. Cell fixation is kept on ice for 2 hours, and then washed with $20\times$volume of cold PBS three times. After the third wash, keep the cells in PBS at 4°C overnight to let any trace amount of paraformaldehyde to leak out the cells. Centrifuge cells again the next morning and wash one more time. Resuspend the fixed T cells in medium at $1\times10^7$ cells/ml. Dispense $1\times10^6$/ml THP-1 cells 100$\mu$l per well into a 96 well U bottom plate. Add 8x, 4x, and $2\times10^6$ cells/ml Primary T cells or H9 cells 100$\mu$l per well. Place the plates in humidified 37°C, 5% $CO_2$ incubator for 48 hours. Centrifuge plate at 1,500 rpm for 5 minutes and carefully transfer 120$\mu$l of the supernatant into a fresh plate. Keep the supernatant in -20°C freezer till use.

Fixed T cells were plated at a density of approximately $5\times10^5$ cells/100$\mu$l, then THP M$\varphi$ were added at 5 x $10^4$ cells /100$\mu$l. The cells were then incubated at 37°C for 24 hours and 48 hours (Figure 53). The supernatant was then assayed for TNF$\alpha$, IL-1$\beta$ and IL-32$\gamma$.

*siRNA.* RNA interference was performed by transfecting siRNA (Nucleofection, amaxa) to specifically knock down the candidate gene expression in T cells. The candidate gene expression was measured by FACS analysis.

*RNA sample preparation and hybridization.* Extract total RNA and purified using the RNeasy MinElute kit (Qiagen)Qiagen Mini RNeasy kit according to the manufacturer's protocol. cDNA synthesis was carried out as described in the Expression Analysis Technical Manual (Affymetrix, two-cycle protocol) using 100 ng of total RNA for each sample. The cRNA reactions were carried out using the BioArray High-Yield Transcript Labeling kit (Enzo). Fifteen micrograms of labeled cRNA was fragmented and sequentially hybridized to the GAPS Slides (Corning) following the manufacturer's instructions.

*Microarray data analysis.* All arrays were normalized and analyzed using the PerfectMatch program (Zhang et al. 2003). A two-way ANOVA test was performed on wild-type and ectopic eyeless antennal, leg, and wing discs. To adjust for multiple testing, the false discovery rate (FDR) was calculated and a total of 956 genes with the cutoff set at 0.001 and at least twofold induction in one sample pair were identified. To further reduce false positives, genes that are not expressed in the top 40 percentile were considered absent and were excluded from further analysis. Furthermore, only genes that are expressed at a higher level in wild-type eye discs than in wild-type leg, wing, or antennal discs were included for further analysis.

*Two-dimensional gel electrophoresis and analysis.* Membrane proteins were extracted from control and stimulated H9 cells using a 2D sample preparation kit for membrane proteins from Mammalian cells available from Pierce. Following extraction and precipitation, proteins were solubilized in chaotropic lysis buffer (7M urea, 2M thiourea, 4% CHAPS, 50 mM DTT, 0.2% carrier ampholytes (pH 4-7), 1% protease inhibitor mix) and quantitated using a modified Bradford assay (BioRad). Isoelectric focusing for the first dimension will be performed by rehydrating 24 cm long pH 4-7 Immobiline DryStrips (GE Healthcare) with 450 mL of chaotropic lysis buffer containing 1000 mg of protein. Following 12 hours of active rehydration (50V, 20°C) proteins were separated using a Protean® IEF cell (Bio-Rad) with a maximum current of 50 mA per strip and a voltage gradient ending at 10 kV with 70,000 volt-hours (Vh). After the first dimension separation, strips were placed at -80°C for at least 2 hours.

Prior to the second dimension separation (SDS-PAGE), thawed strips were first incubated for 15 min with 10 mg/mL DTT in equilibration buffer (20% glycerol, 20% SDS, 1.5M Tris pH 8.8 and 6M urea), then with 25 mg/mL iodoacetamide in the same buffer. The second dimension of separation was carried out using an Ettan DALT-12 system with 12.5% precast polyacrylamide gels (26 x 20 cm) and the DALT buffer Kit (GE Healthcare). Proteins were separated by molecular weight using 60W for 30 minutes and 180W for approximately 5 hours and maintained at 15°C. Proteins were stained overnight with Coomassie stain (Bio-SafeTM, Bio-Rad Laboratories, CA, USA) following manufacturer instructions. Imaging will be performed on a LabScan (GE Healthcare) at 300 dots per inch (dpi) following destaining with water. Statistically significant changes in protein abundances were determined by IMAGE-Master platinum II software version 5.0 (GE Healthcare). The intensity (3-dimensional volume) of each protein spot was normalized to the total intensity of all spots detected on a gel. A detection threshold which results in an average of 1500 protein spots per gel were individually adjusted before comparing the 24 gels (6 groups, n = 4 each group). Spots appearing in at least 23 out of 24 gels were clustered. Differences in protein expression were identified using the relative volume (%Vol) option of the software. All statistically significant spots (Student T test p<0.05 compared to the control) were manually investigated to ensure that two spots are not fused or that the spots are not misgrouped. Change in average density larger than ±40% of the average spot volume as compared to the control was the criterion used for ex*cis*ion of spots with subsequent identification by mass spectrometry.

Proteins were digested in the gel spots using a modification of the methods of Havlis and Jimenez. Briefly, spots from at least 2 replicates were combined and destained two to three times with 1/1 acetonitrile and 100 mM ammonium bicarbonate, then contracted with 100% acetonitrile and vacuum dried. Spots were rehydrated with 25 ng/ml trypsin and incubated overnight at 37°C. The supernatants were collected and pooled with 2 additional extracts using 1 % formic acid (aqueous) with 30% acetonitrile. Pooled extracts were vacuum-concentrated to approximately 10 μL and stored at -80°C until used. Approximately 30% of the in gel-digested sample was analyzed by reverse phase nanospray LC-MS/MS (Agilent 1100 HPLC, 75 mm ID x 15 cm column, Zorbax C18). Buffer A 0.1% formic acid. Peptides were eluted from the separating column into the mass spectrometer using a gradient of increasing buffer B (90% ACN, 0.1 % formic acid) at a flow rate of 300 nl/min. Spectra was collected over a m/z range of 350-1800 Da (Agilent LC/MSD Trap XCT Ultra). Three MS/MS spectra will collected for the six most abundant m/z values, then those masses were excluded from analysis for 1 min and the next six most abundant m/z values were selected for fragmentation.

Proteins were identified by searching the NCBInr, IPI human and SwissProt databases using both Mascot (Matrix Science) and Spectrum Mill (Agilent) programs. For Mascot, compound lists of the resulting spectra were generated using an intensity threshold of 10,000 and a minimum of 0.2% relative abundance with grouping within 5 scans. The compound lists were exported as mgf files and searched against databases (updated weekly) using a taxonomy filter for human. Parameters used in the database search are as follows: monoisotopic mass, peptide mass tolerance of 2.4 Da, fragment ion mass tolerance of 0.7 Da, tryptic peptides only allowing for 2 missed cleavages, carbamidomethylation of Cys as a fixed modification and deamidation (N,Q) and acetylation (K) as variable modifications. Similar parameters were used for the SpectrumMill search. SpectrumMill protein scores above 13, with peptide scores above 10 and scored percent intensity (SPI) above 70% were the cutoff for initial hit validation.

FKBP52 is upregulated in stimulated CD3+ T cells

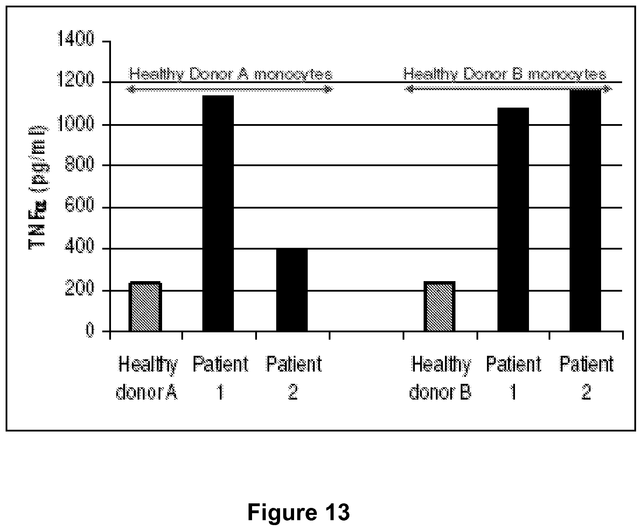

The pro-inflammatory cytokine TNFα is upregulated in T cells taken from the blood of psoriasis patients as compared with those obtained from the blood of healthy donors (Figure 13). Upregulation of proinflammatory cytokines by activated macrophages (MΦ) has been shown to drive the pathogenesis of chronic plaque psoriasis, most likely via direct cell-cell contact through an immune synapse (IS) mechanism in the skin. In some embodiments, T cell and M(p proteins that mediate adaptive immunity in this system are provided.

Differential two-dimensional gel electrophoresis of stimulated and unstimulated human T cells was used to select upregulated protein candidates for subsequent identification by tandem mass spectrometry.

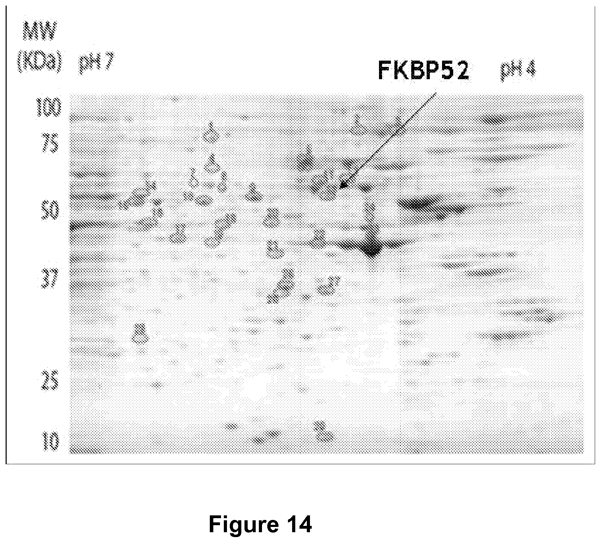

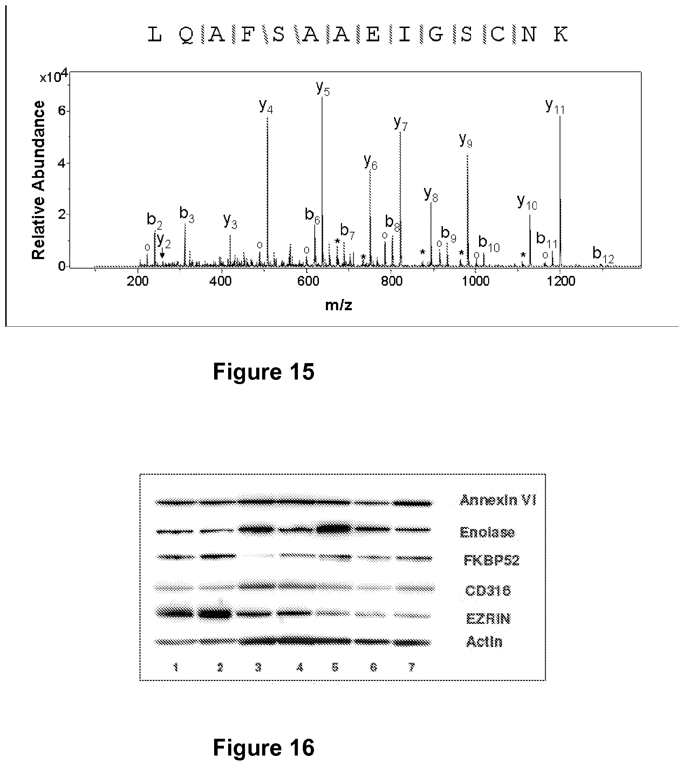

Two-dimensional gel-based proteomics is a well-accepted method for identifying proteins that change their expression or modification status in response to some trigger. This approach was investigated for differential expression analysis of stimulated human T-cell line H9 cells. The pH range for protein separation was optimized by charge and the acrylamide percentage for separating proteins by size using a whole cell lysate extraction. These conditions (11 cm IPG strip, pH 10 4-6, 10.5 - 14% gradient SDS-PAGE gel) were applied to a preparation of stimulated and unstimulated H9 cells. The protein separation of the stimulated sample is shown in Figure 14. The labeled spots, representing a change in expression of at least 1.5 fold, were ex*cis*ed, digested using trypsin, and then analyzed by nanoLC/MS/MS on an Agilent Ultra high capacity ion trap. Database searching with Spectrum Mill was used to identify the proteins. High scoring, validated protein identifications were obtained for all spots. Spot number 13 was identified as FKBP52 using Tandem mass spectroscopy (Figure 15).

Western blot analysis confirmed that expression of FKBP52 was slightly increased in stimulated H9 cells (data not shown) and significantly increased in stimulated CD3+ T cells (Figure 16).

Microarray analysis summarized in Table 1 shows elevated up regulation of FKBP52 and FKBP12 in H9 cells (as compared with unstimulated controls) and significant up-regulation in psoriatic lesional skin, bolstering the proteomics results.

| **Table 1:** Set of candidate genes upregulated from both anti-CD3/anti-CD28 stimulated T cell lines and psoriasis lesional skin biopsies.* | | | |
|---|---|---|---|
| **Gene** | **H9** | **Jurkat** | **Psoriasis** |
| MCL-1 | 108.00% | 136.50% | 351.00% |
| PBEF-1 | 150.50% | 95.50% | 256.00% |
| FKBP52 | 65.50% | 44.30% | 297.00% |
| FKBP12 | 69.80% | 41.70% | 297.00% |
| ICAM-1 | 357.00% | 0.14% | 197.00% |
| AREG | 109.00% | 155.00% | 128.00% |
| CD69 | 200.00% | 1330.00% | 79.50% |
| PRSS7 | 100.00% | -27.10% | 72.00% |
| uPAR | 89.30% | 0.29% | 68.90% |
| PLAG4A | 134.00% | N/A | 60.30% |
| p21 | 318.00% | 7.34% | 54.00% |
| GADD45A | 258.00% | 41.00% | 46.00% |
| *Selection criteria: Statistically significant changes (>40% gene upregulation and p s 0.05) in anti-CD3/anti-CD28 stimulated over unstimulated T cell lines and in psoriasis lesional skin over normal healthy skin biopsies. | | | |

Protein expression was validated by Western blot, qRT-PCR and FACs analysis. Expression of proteins of interest was inhibited using siRNA, and a cell-cell contact assay measuring cytokine levels was used to establish protein functionality (discussed below). Results from the proteomics study suggested that expression of FKBP52 was significantly increased following T cell activation by PMA/Ionomycin treatment.

Knockdown of FKBP52 does not appreciably affect cytokine levels using H9 or CD3+ T cells

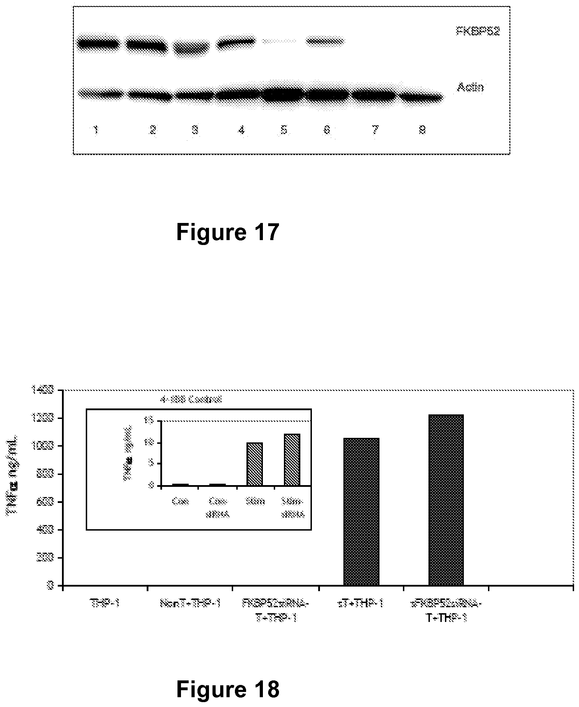

To determine if FKBP52 is an important molecule in T cell:MΦ signal transduction leading to cytokine upregulation, siRNA was used to inhibit expression of FKBP52. Results of the siRNA experiments are discussed below. Western blot demonstrates effective knockdown in CD3+ T cells (Figure 17). siRNA did not effectively inhibit FKBP52 expression in H9 cells.

A cell-cell contact assay demonstrated that knockdown of FKBP52 did not have a significant inhibitory effect on TNFα levels (4-1 BB, a marker for activated lymphocytes, is shown in the inset as a positive control), suggesting that either FKBP52 is not important in this signaling pathway or it operates in conjunction with another protein (Figure 18).

CD147 is upregulated in human CD3+ T cells after contact with macrophages and interacts with FKBP52

FKBP52 is an immunophilin with *cis-trans* prolyl isomerase activity. Other members of this superfamily include cyclophilins, for example, cyclophilin A and B. These proteins bind to or otherwise associate with CD147 (EMPRINN) (Figure 7) and are mediators of inflammatory diseases.

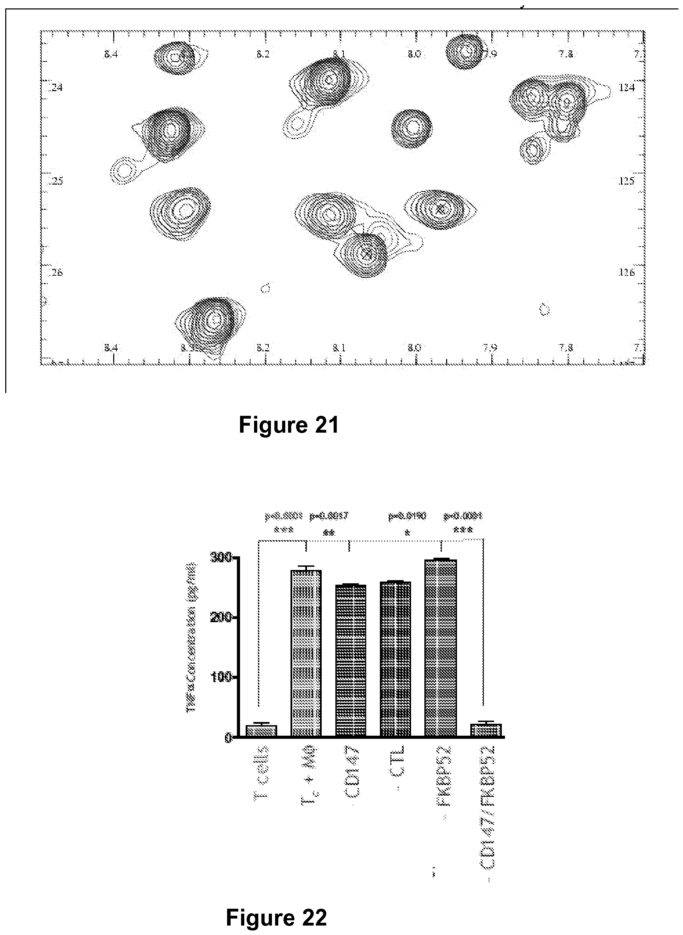

As shown in Figure 21, NMR was used to demonstrate the interaction between CD147 and FKBP52. $^{15}$N-labeled CD147 at 0.5 mM was titrated with 0.5 mM unlabeled FKBP52. Resonances of CD147 corresponding to the receptor's membrane proximal region (residues 207-234) exhibit NMR chemical shift changes, indicating a weak, but physiological association. This weak interaction is consistent with known cyclophilin A/CD147 binding.

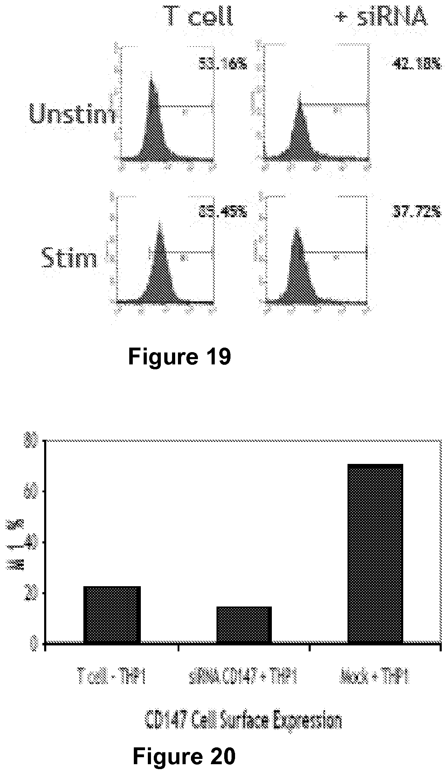

FACS analysis revealed that CD147 cell surface expression is upregulated in stimulated T cells and is induced by T cell:MΦ contact (Figures 19-20).

Measurement of TNFα levels following T cell:MΦ contact revealed that TNFα was significantly upregulated when activated T cells came into contact with Mφ. However, TNFα levels were not significantly inhibited by siRNA knockdown of CD147, a mock siRNA used as a control (CTL), or siRNA knockdown of FKBP52. TNFα concentrations were significantly reduced when both CD147 and FKBP52 were knocked down, suggesting that these two proteins work together to mediate T cell:Mφ signaling during T cell activation (Figure 22).

Dual inhibition of FKBP52 and CD147 by siRNA or monoclonal antibodies synergistically Inhibits T-cell-mediated MΦ activation to secrete proinflammatory cytokines

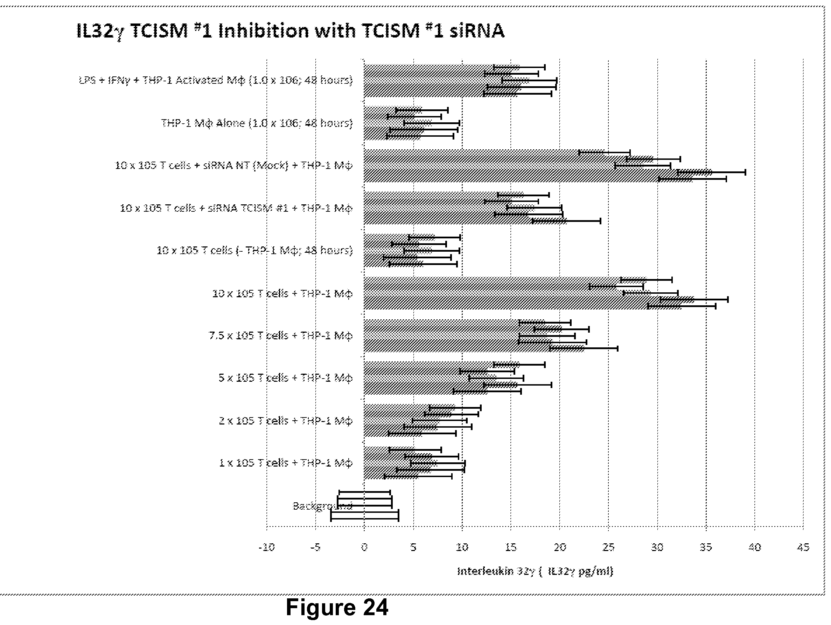

To determine whether inhibition of CD147 or FKBP52 alone or together results in a decrease in proinflammatory cytokine release from THP-1 Mφ, CD3+ cells from Healthy Human Donors and Th22 cells (a type of memory T cell) from *Psoriasis vulgaris* patients were targeted by siRNA or monoclonal antibodies able to knockdown FKBP52 or CD147 and stimulated before placing them in various concentrations relative to THP-1 Mφ in a cell-cell contact assay. After 48 hours of incubation, the supernatants were aspirated and assayed for levels of IL32γ, TNFα and IL-1β. The T cells were isolated, and may be used to measure FKBP52 and CH147 by FACs, qRT-PCR, western blot or immunohistochemistry (IHC). The Mφ may by lysed and tested for intracellular IL-32γ and other Mφ cytokines by qRT-PCR or any other suitable method. In Figures 22-32, TCISM #1 is CD147 and TCISM #2 is FKBP52.

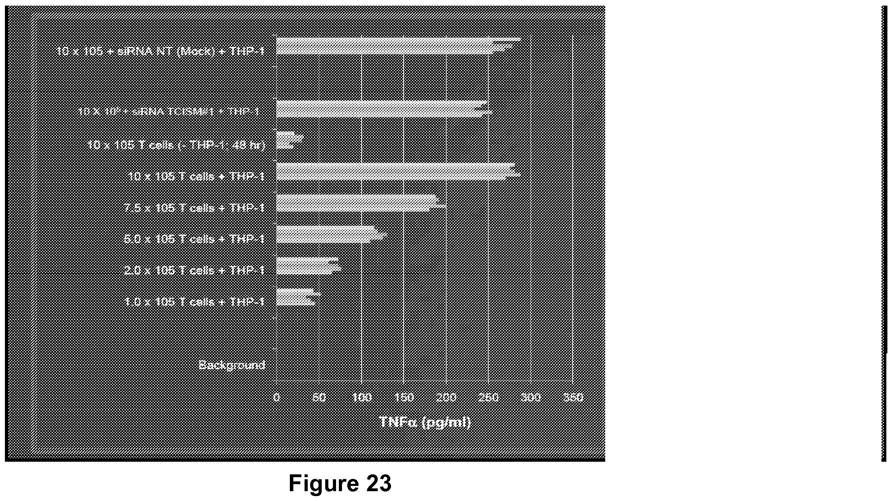

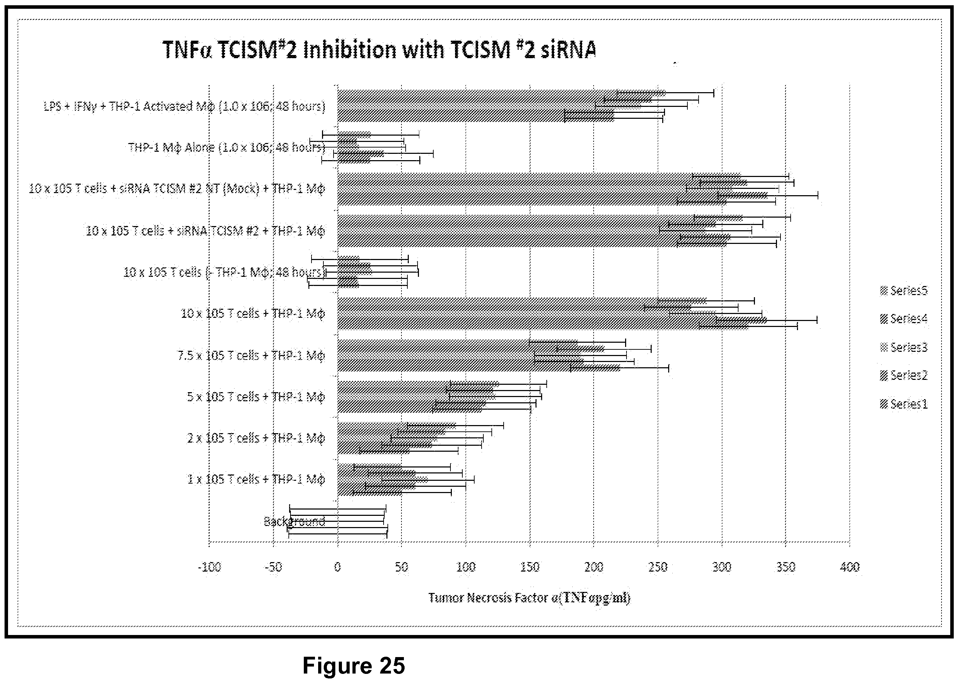

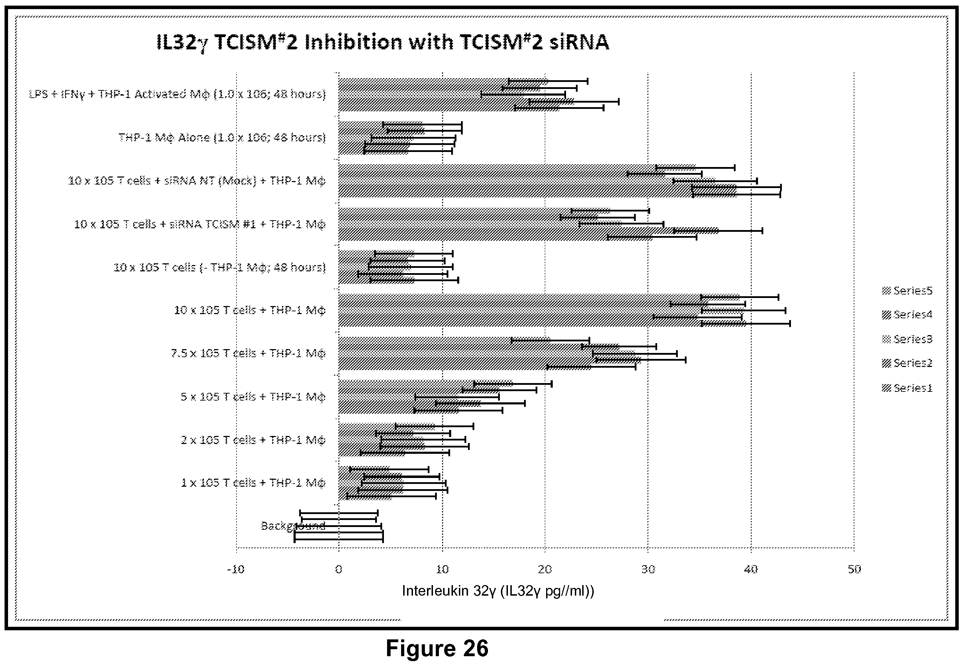

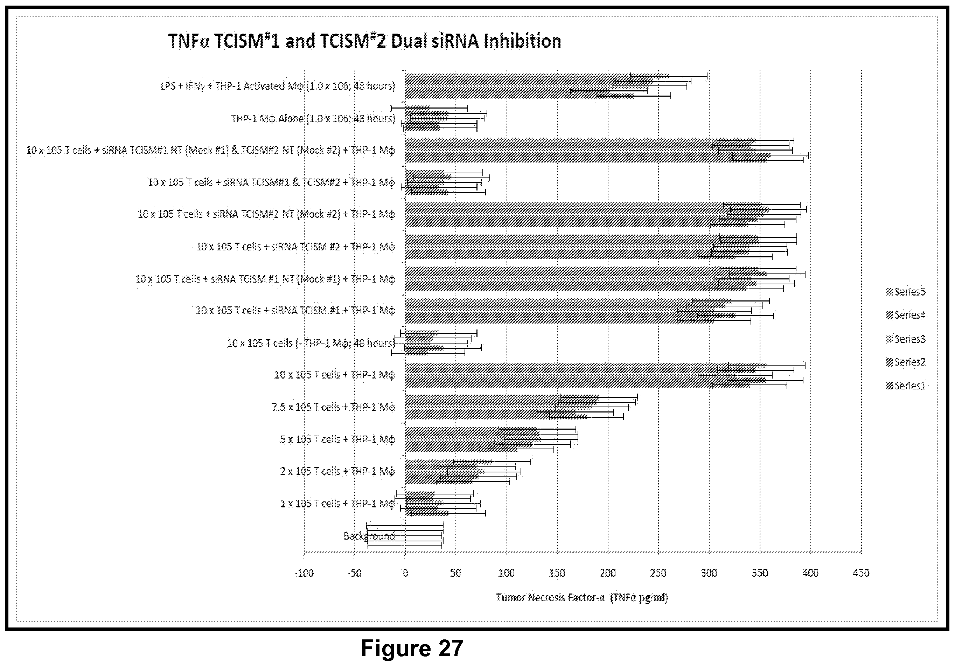

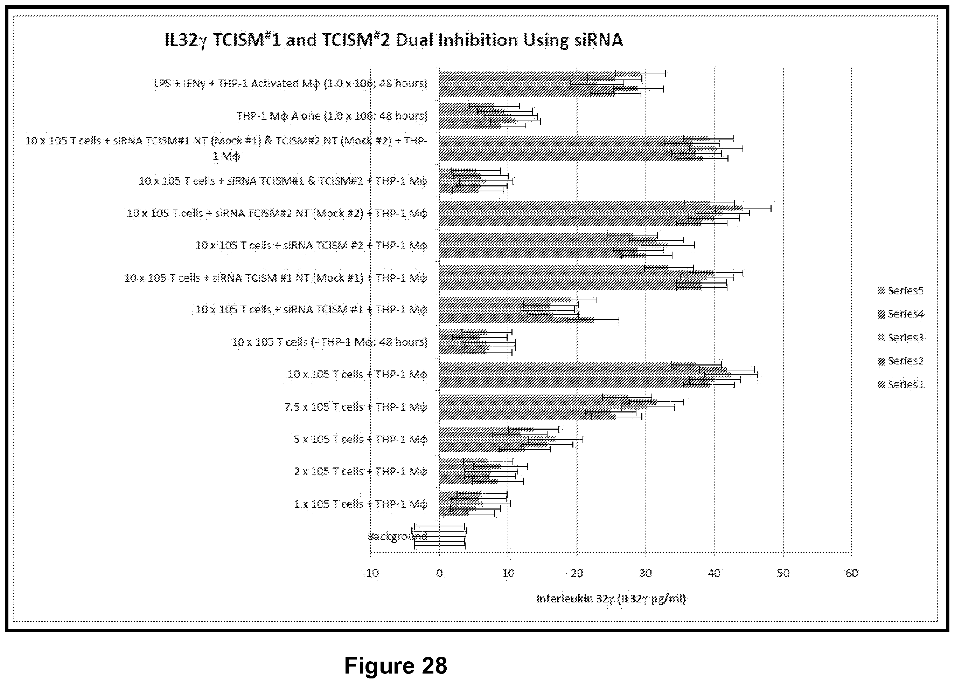

*Inhibition studies in CD3+cells.* Human CD3+ T cell-mediated Mφ production of TNFα appears to be proportional to the number of T cells. siRNA knockdown of CD147 or FKBP52 alone does not inhibit T cell mediated Mφ TNFα production (Figures 22, 25). siRNA knockdown of CD147 or FKBP52 alone moderately inhibits T cell mediated Mφ IL-32γ production by 50% (CD147, Figure 23-24) and 20% (FKBP52, Figure 26). However, simultaneous siRNA knockdown of CD147 and FKBP52 inhibits T cell mediated Mφ TNFα (Figure 27) and IL32γ (Figure 28) production by approximately 90%.

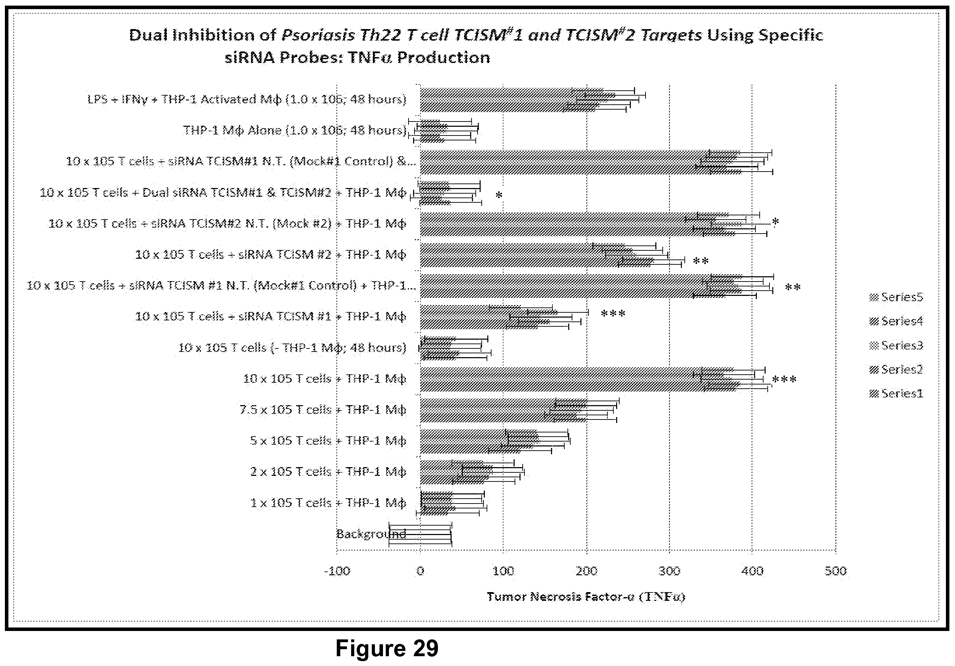

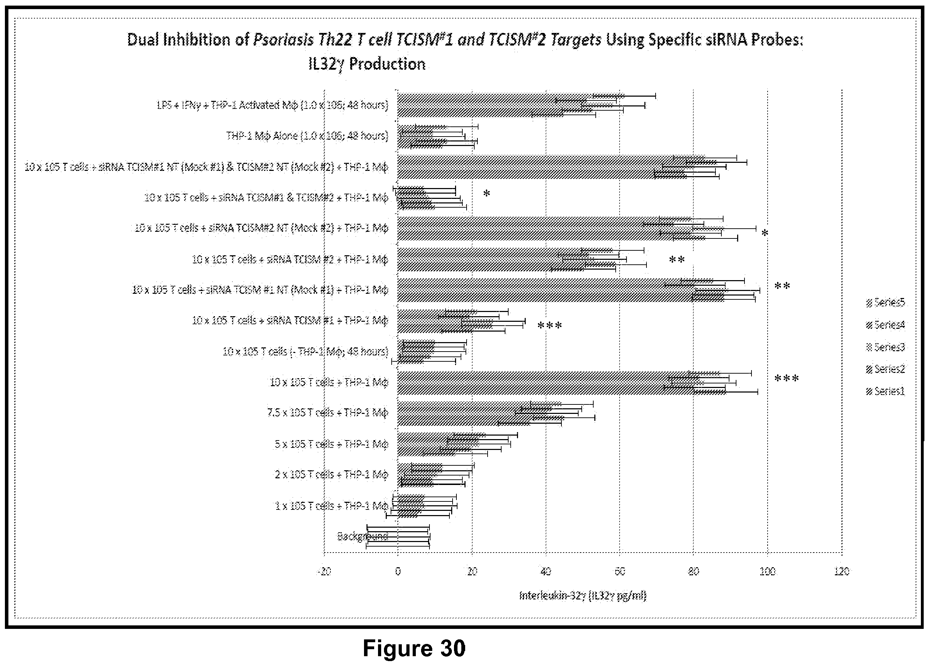

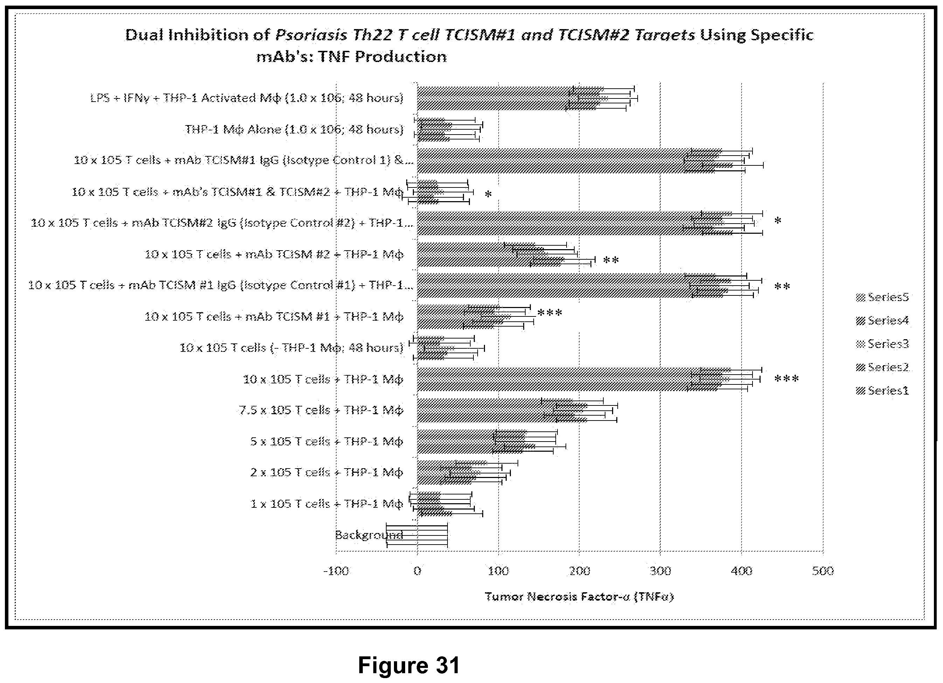

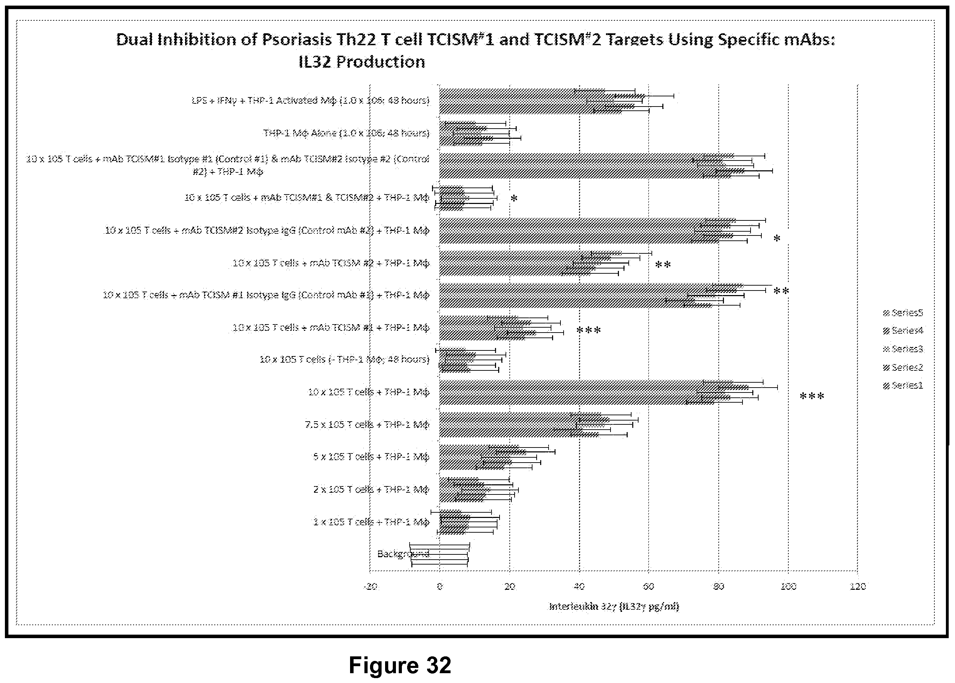

*Dual Inhibition studies in Th22 cells.* In Th22 cells, simultaneous siRNA knockdown of CD147 and FKBP52 inhibits T cell mediated Mφ TNFα (Figure 29) and IL32γ (Figure 30) production by approximately 95%. Likewise, simultaneous blocking of CD147 and FKBP52 by specific monoclonal antibodies inhibits T cell mediated Mφ TNFα (Figure 31) and IL32γ (Figure 32) production by approximately 95%.

This data shows that while CD147 and FKBP52 may have acceptable independent effects on Mφ cytokine release, the two proteins act synergistically for a more potent inhibition.

## Example 2: IL-32 is produced by keratinocytes upon stimulation by T cells

To further explore its role and pathogenesis in psoriasis, the expression and characteristics of IL-32 in human psoriatic skin tissue through immunohistochemistry (IHC) and electrochemilucent (ECL) measurements of cytokines, qRT-PCR measurement of cytokine mRNA, and primary cell culture experiments using Hka and isolated subpopulations of human memory effector T cells were investigated. The results show that IL-32 is a novel proinflammatory cytokine that may be involved in the early stages of cutaneous inflammatory diseases including psoriasis vulgaris, and that a unique CD4+CD45RO+CCR4+ effector Th22 T cell population may be involved in the initiation of this disease.

Materials and methods

*Psoriasis subjects.* In two different University of Colorado Denver, Anshutz Medical Campus Institutional Review board approved protocols (*COMIRB #05-0275* [Dr. Carl Edwards, Principle Investigator], and *COMIRB #06-0303* [Dr. Carl Edwards, Principle Investigator]), 15 adults (between the ages of 18-65) with psoriasis (affecting 5-10% of body surface area), provided written consent for LS and NL 3-6 mm punch skin biopsies. The study was conducted according to the Declaration of Helsinki Principles. Psoriasis patients were required to have at least one active psoriatic lesion visible by their physician. Before this, patients had no systemic and topical treatment for 4 and 2 weeks, respectively.

Additionally, biopsies from N=21 healthy human volunteers were obtained for IHC and qRT-PCR studies. Half of each biopsy was frozen in OCT (Sakura Fine technical, Tokyo, Japan) and stored at -80oC for IHC, and the remaining section stored in liquid nitrogen until analysis.

*Purification of recombinant IL-32α and IL-32γ proteins.* Recombinant IL-32α and IL-32γ proteins were expressed in *E. coli* as his6 tag N-terminal fusion proteins, followed by affinity purification on a TALON affinity column (Invitrogen) as previously described (90, 123). The TALON affinity-purified recombinant IL-32α and IL-32γ proteins were digested with tobacco etch virus protease (Invitrogen) for 2h at 30°C to remove the N-terminal his6 tags. The preparations were dialyzed and subjected to ion exchange by using a 1-ml HiTrapTM QFF column on ÁKTA FPLC (Amersham Pharmacia Biosciences) using a 0- to 500-mM NaCl gradient. The eluted protein peak fractions were dialyzed against 50 mM Tris-base buffer (pH 8) and then subjected to size-exclusion chromatography (Superdex75, AKTA FPLC). The resulting fractions were identified by SDS/PAGE with silver staining.

The final three-step-purified recombinant IL-32α and IL-32γ proteins were used for monocyte differentiation and cytokine assays. Recombinant IL-32α and IL-32γ were added to cultures of human PBMCs in the presence of 10 μg/ml polymyxin B (Sigma-Aldrich), which did not affect the production of TNFα or IL-1β.

*Cell isolation.* Peripheral blood mononuclear cells (PBMCs). PBMCs were isolated directly from the whole blood of healthy human volunteers, or from leukopacs containing white blood cells only from healthy human donors (Denver Bonfils Blood Center, University of Colorado Denver University Hospital, Aurora, CO). After informed consent, venous blood was drawn from the antecubital vein into heparin tubes and processed for PBMCs as described in the approved COMIRB protocol. The study was approved by the Colorado Multiple Institutional Review Board. Ficoll density gradient centrifugation of PBMC was performed as described previously (124). The viability of obtained PBMCs was always >95%, as determined by trypan blue staining. The viable cells were quantified in a Neubauer chamber (Zeiss, Oberkochen, Germany) and stored in liquid nitrogen. The donors were free of prescribed and over-the-counter medications.

*In vitro cell studies.* Normal human primary adult keratinocytes (Hka) were cultured in serum-free EpiLife medium with human KC growth supplement (0.2% bovine pituitary extract (v/v), 5 μg/ml bovine insulin, 5 μg/ml bovine transferrin, 0.2 ng/ml human EGF, and 0.18 μg/ml hydrocortisone), all purchased from Cascade Biologics (OR, USA). The H9 T-cell lymphoma cell line (ATCC, Manassas, VA, USA), normal primary human CD3+ T cells (Belle Bonfils Blood Donor Center, University of Colorado University Hospital, Aurora, CO, USA), and Th22 T cells obtained from Psoriasis patient blood, were cultured in RPMI 1640 with 10% fetal calf serum (FCS), 100mM Hepes, 100 U/ml penicillin, and 100 μg/ml streptomycin, all purchased from Invitrogen Life Technologies. PMA and Ionomycin were purchased from Calbiochem (San Diego, CA).

*Cell stimulation.* Primary T-cells, H9 cells and Th22 cells (1x106 cells/ml) were stimulated with PMA (1ng/ml) and Ionomycin (500ng/ml) for 18 hrs at 37oC. HKa were stimulated with TNFα (1 ng/ml), TNFα (10 ng/ml) + PEG sTNF-RI (10μg/ml), IL-1β (10ng/ml), IL-1β ng/ml+IL-1ra 10g/ml, IL-1β ng/ml +TNFα 10ng/ml and IL-32α or IL-32β for 24 hrs. Hka were stimulated with activated H9 and primary T-cells for 24 hrs at 37°C. PEGsTNF-RI and IL-1ra was obtained from Amgen (Thousand Oaks, CA).

*Cytokine stimulation.* Freshly isolated human PBMCs were incubated with either control RPMI medium or 30 ng/ml IL-32 in 96-flat well plates. After 24h incubation at 37°C, supernatants were collected, and proinflammatory cytokines (TNF, IL-1β, IL-32 and IL-6) concentrations were measured by electrochemiluminescence using a BioVeris apparatus. Antibodies for biotinylation and ruthenylation as well as standards were obtained from R&D Systems. In some experiments, the MAPK inhibitor (2 μM SB203580; Calbiochem) and a pan-caspase inhibitor (10 M z-VAD-fmk; Calbiochem) were examined.

*Cytokine detection ELISA.* Quantitation of cytokine production in cell culture supernatants was determined by enzyme linked immunosorbent assay (R&D Systems) according to the manufacturer's instructions. Cytokine-producing cells were determined by intracellular staining using phycoerythrinconjugated anti-IFNγ (BD PharMingen), Alexa Fluor 647-conjugated anti-human IL-17 (eBioscience, San Diego, CA), and Alexa Fluor 488-conjugated anti-human forkhead box P3 (FoxP3; BioLegend, San Diego, CA). Briefly, cells were stimulated with phorbol myristate acetate and ionomycin for 4 hours, and Golgiplug (BD Biosciences, San Jose, CA) was added after 2 hours. Cells were fixed in 4% paraformaldehyde, permeabilized with 0.1 % saponin, stained with fluorescent antibodies, and analyzed on a FACSCalibur flow cytometer (BD Biosciences). Cell Quest software (BD Biosciences) was used for data acquisition, and Flow Jo software (Tree Star, Ashland, OR) was used for analysis.

*Primary human T cell and effector memory T cell purification after in vitro activation.* Human CD3+ T cells were isolated by negative selection using the Pan T cell Isolation Kit II along with the AutoMACCs instrumentation (Miltenyi, CA, USA) following the manufacturer's provided protocols. The resulting purified T cells had >98% purity as determined by CD3-FITC labeling and FACS analysis. CD3+ T cells were activated for 6h using 25 ng ml-1 phorbol myristate acetate and 2 mg ml-1 naive CD4+ T cells and effector memory CD4+CD45RA+ or CD4+CD45RO+ T cells were further purified, after PMA+ionomycin stimulation using a human naive or memory CD4+ T cell isolation kit (Miltenyi Biotec, Bergisch Gladbach, Germany). The purity of CD45RA+ and CD45RO+ T cells was >90%.

*CD3+ T cell populations purification, flow cytometry, cell-cell contact assay.* PMA+ionomycin-activated CD3+

T cells, obtained from leukopacks (Belle-Bonfils Blood Center, UCD Hospital, Denver, CO), were further purified using Memory CD4+, CD45RA+ and CD45RO+ T cell isolation kits (Miltenyi Biotec). Cells were stained with CD45RO-FITC, CD45RA-FITC, CD4-APC, and CCR4-PE (all obtained from Beckman Coulter) for 20 minutes at room temperature. Cells were then washed in PBS followed by fixation in 2% parafomaldehyde. Flow cytometry was performed on an FC500 (Beckman Coulter, Hialeah, FL). 5000 events were collected for each sample. Data shown are representative from a single experiment where a total of four separate experiments were performed. 1% paraformaldehyde-fixed T cells added to the Hka cells induced Hka cell IL-32γ mRNA or protein (data not shown). PMA+ionomycin-activated human memory effector T cell populations (CD4+CD45RO+CCR4+, CD4+CD45RO+CCR4-, CD4+CD45RA+CCR4+, CD4+CD45RA+CCR4- [10 x 105 total T cells, purified as outlined above]), were contacted with primary Hka after direct cell-cell contact for 48 hours. Hka cell supernatants and lysates were measured for IL-32γ, TNFα, or IL-1β by ECL as described above. A total of 6 individual measurements were assessed for each cell population. Each bar represents mean±SEM where (*) $P < 0.05$ (ANOVA), and where *# $P < 0.001$ (ANOVA). A total of two separate experiments using separate healthy human donors were completed with similar results.

*ECL.* A polyclonal antibody produced in rabbits against recombinant IL-32y was used for ECL. IgG was prepared with Econo-Pac and then affinity purified through IL-32-immobilized agarose bead column of Affi-gel 15. An aliquot of the affinity purified anti-IL-32γ antibody was labeled with biotin and another aliquot with ruthenium according to the manufacturer's instructions (BioVeris, Gaithersburg, MD). The biotin, and ruthenium, labeled antibodies were used to construct a standard curve using recombinant IL-32γ for ECL assay. The liquid-phase ECL method was used to measure TNFα, IL-1β, IL-6, IL-8 and IL-32γ in human primary keratinocytes cell culture media and cell lysates in 0.5% Triton-X. The amount of ECL was determined by using an Origen Analyzer (BioVeris).

*MTT assay.* The proliferation of Hka was assessed using a MTT cell proliferation kit I (Roche Applied Science). Hka were seeded at 2000 cells/well in 96-well plates in EpiLife medium with complete human KC growth supplement. The medium was changed to EpiLife supplemented with Human recombinant IL-32γ and IL-32y in serial concentration (0, 1, 5, 10, 20, 40, 60, 80, 100 pg/ml) after letting the cells grow overnight. The cell cultures were incubated for 24 hrs at 37°C and 5% CO2. After the incubation period, the proliferation is detected according to the manufacturer's protocol (Roche Applied Science).

*Cell-cell contact bioassay.* Hka were seeded at 2.5x105 cells/well in Control Inserts 6-well plate Micron) (Cat. No. 354567, BD) in EpiLife medium with complete human KC growth supplement. 16hrs later the medium was removed None stimulated and stimulated H9 cell (5x105, 7.5x105 and 1x106) and primary T cell (1x106) in RPMI 1640 with 10% fetal calf serum (FCS), 100mM Hepes, 100 U/ml penicillin, and 100 μg/ml streptomycin were added on the top of Hka with and without control Inserts for 24 hrs. The H9 cells and primary T cells were removed, and Hka were washed with 1x PBS three time. For IL-32 protein concentration, the Hka were lysed with 0.5% Triton-X for, and for IL-32 gene expression Hka were lysated with lyse buffer according to the protocol of RNeasy Mini Kit (Qiagen, Valencia, CA, USA).

*Immunohistochemistry and histology.* The skin tissue samples were fixed in 10% formalin and embedded in paraffin. Three sections from the paraffin blocks were used for IHC of IL-32 with the ABC detection system (DakoCytomation, Carpinteria, CA); monoclonal mouse antibody against human IL-32γ (Mab) was used (90). IHC steps were carried out at room temperature as previously described (125). After deparaffinization, the sections were placed into a pressure cooker in 10 mM sodium citrate buffer (pH 6.0) at full power for 4min, followed by treatment with 3% hydrogen peroxide for 10 min. The primary antibody (5 mg/ml) was diluted (1:16,000), incubated for 30min with the tissue sections, and exposed to EnVision reagent (DAKO) for 30min. The slides were then sequentially incubated with the chromogen reagent for 10 min, counterstained with Meyer's hematoxylin, and mounted. Negative control staining was performed by using mouse IgG1 Isotype antibody (90). Negative controls were performed on all sections using an equivalent concentration of a subclass-matched IgG1 (BD Pharmingen, San Diego, CA). IL-32 Peptide (0.4 mg/ml; Dr. Charles A. Dinarello) were used for the antibody blocking study: 1 μl of IL-32 peptide was added to 400 I of diluted IL-32γ Mab, mixed and incubated at 40 C for overnight. After centrifuge at 15k/rpm for 15 minutes, the supernatant were used for incubation of skin tissue. Hematoxylin and eosin (HE) staining were used for Hka histology study.

*Evaluation of Immunohistochemical Staining.* Slides were observed by light microscopy. A review of the entire tissue section was performed to evaluate for heterogeneity in antigen distribution and the proportion of IL-32γ positive cells throughout each tissue section. The cellular distribution pattern of IL-32γ expression was classified in the same manner as previously described (126).

*Quantitative real-time quantitative polymerase chain reaction (TaqMan) analysis (qRT PCR).* Skin samples from psoriatic patients and normal healthy donors were fixed in 10% formalin and paraffin-embedded. Total RNA was extracted from 4 freshly cut 10 M sections with RNeasy FFPE Kit (Qiagen, Valencia, CA, USA). The IL-1β, IL-6, IL-8. IL-32 and TNFα gene expression kits (ABI) were used for qRT-PCR. Total RNA of the Hka was extracted using the RNeasy Mini Kit (Qiagen, Valencia, CA, USA). Complementary DNA was synthesized with the use of a TaqMan Reverse Transcription kit (Applied Biosystems, Foster City, CA) using random hexamers as primers according to the manufacturer's instructions. Reactions were run in four replicates (24 gene transcripts in a 96-well plate) on an ABI Prism 7700 Sequence Detection System. Each well also contained specific primers and probes to measure 18S rRNA as an internal control. The amount of cDNA added to each reaction was held to a relatively narrow range, determined by the measurement of 18S RNA. Hypoxanthine guanine phosphoribosyltransferase (HPRT) was used as an endogenous control. The results of real-time PCR are shown in terms of relative expression compared with IL-32 basal line.

*Statistical analysis.* Statistical analyses were performed using SigmaStat 3.1 software (StataCorp, College Station, TX). Experiments were performed at least three times and data are presented as means$\pm$SEM. Comparisons between the groups were performed by using a paired nonlogarithmic Wilcoxon test and/or 2-way ANOVA where $P$ <0.05 is considered significant. Two-tailed paired Student's t-test was used to compare normal versus psoriasis nonlesional and lesional cell counts for IL-32$\alpha$ and IL-32$\gamma$ T-cell populations. The two-tailed $P$-values are designated as $P<0.05$ (*), $P<0.01$ (**), and $P<0.001$ (***).

Results

*Expression of IL-32$\gamma$ in psoriatic skin lesions.* The presence and pattern of expression of IL-32$\gamma$ in psoriatic skin was compared to normal human skin by IHC. Sections of fixed formalin paraffin embedded (FFPE) biopsy tissue of whole skin from 15 psoriasis subjects with active disease revealed a unique pattern of IL-32 expression including cytoplasmic granule staining in basal layer keratinocytes and increased dendritic cell (DC) staining in the epidermis (Figure 33A-C). Increased nuclear staining in various DC as well as epithelial cells staining in small vascular vessels in the dermis were observed (Figure 33B). As an additional control, skin obtained from healthy adult volunteers (N=6) was stained, and found that positive IL-32$\gamma$ staining was present, although not as consistent in all fields or brightly apparent as skin obtained from psoriasis patients with active disease (Figure 33F). Additionally, the specificity of IL-32$\gamma$ in psoriatic skin was confirmed by antibody-blocking assays using either a specific anti-IL-32$\gamma$ Mab which does not cross-react with IL-32$\gamma$ (90), or a competitive antagonist peptide (a gift by Dr. Charles Dinarello) (Figure 33E). Given that IL-32$\gamma$ is now known to be a proinflammatory cytokine which works in conjunction with NOD ligands resulting in additional cytokine production (91), it's strong presence in psoriatic skin but not in healthy adult skin suggests an important role in psoriasis.

To further investigate IL-32 expression in psoriatic skin, total RNA from the FFPE human tissues noted above was extracted and the presence of mRNA for IL-32 was assessed by qRT-PCR using a highly validated assay system (92). The qRT-PCR analysis revealed that psoriatic skin tissues synthesize elevated mRNA levels for IL-32$\gamma$ (Figure 34). Both normal and psoriatic lesional skin expressed mRNA for IL-32$\gamma$, although the IL-32$\gamma$ mRNA levels obtained from the psoriatic skin tissues were nearly 10 times higher than those expressed in healthy human skin (Figure 34). IL-32$\gamma$ expression in these psoriatic lesional skin biopsy samples was significantly ($P<0.01$) higher than healthy human controls. In addition, the presence of IL-1$\beta$, IL-8, and TNF in these psoriatic skin biopsy samples was discovered. Like IL-32$\gamma$ expression, these cytokine expression levels in psoriatic lesional skin were significantly ($P<0.01$) increased compared to healthy volunteer biopsy samples (Figure 34). Although the IL-32$\gamma$ transcript has not previously been described in adult human skin, it has been demonstrated in several other human tissues (86, 93). IL-32$\gamma$ expression has been previously demonstrated to be consistently more prominent in immune cells than in non-immune tissue (94). The results described above show that IL-32 is expressed in human skin and particularly in Hka obtained from healthy human volunteers.

*Production of IL-32$\gamma$ and its biological activity in Hka.* Next, it was determined that Hka produce IL-32$\gamma$ at both the protein and RNA level. It has recently been shown that rhuIL-32 up-regulates IL-32 with no apparent cross-reactivity of MAb's used for ECL quantitation (90). After being subjected to TNF, IL-1$\beta$ or recombinant human IL-32 (rhuIL-32) treatment as inflammatory stimuli, the cell lysates and supernatants from Hka were analyzed for newly produced proinflammatory cytokines using ECL analysis after +48 hours culture. Basal levels of IL-32$\gamma$ were detected primarily in the cell lysates of Hka indicating that IL-32is an intracellular cytokine in this skin cell type (Figure 35). Additionally, stimulation of Hka with either rhuTNF$\alpha$ or rhuIL-1$\beta$ induced highly significant ($P<0.001$) levels of cytoplasmic IL-32$\gamma$ (Figure 35). Furthermore, qRT-PCR results indicated thatTNF$\alpha$ and IL-1$\beta$ reciprocally induced IL-32 mRNA expression (Figure 36). These responses were reversed by a recombinant human p55 TNF soluble receptor with high-affinity binding to TNF$\alpha$ (i.e., PEGs TNF-RI, a kind gift from Amgen)(95), and recombinant human IL-1 ra (i.e., Anakinra, Amgen) (96). As shown in Figure 37, rhuIL-32$\gamma$ induced significant amounts of IL-1$\beta$, IL-6, IL-8 and TNF$\alpha$, while the IL-32$\gamma$ protein levels were not increased, as has been previously demonstrated (90).

Finally, Hka were treated with optimal activating levels of rhuIL-32 and other cytokines *The effect of IL-32 on Hka proliferation.* We next evaluated whether IL-32$\alpha$ or IL-32$\gamma$ could induce Hka proliferation in monolayer cultures. Unexpectedly, we found that primary Hka proliferation was significantly inhibited by stimulation with either IL-32 isoform (Figure 39). MTT assay results at +12, +24, and +48 hrs-post rhuIL-32 stimulation show a dose-dependent inhibitory response on Hka proliferation despite their activated phenotypes shown in Figure 38. These data also demonstrate that there were negligible differences between the two IL-32 isoforms to inhibit Hka proliferation.

*Cell-cell contact bioassay of Hka and human T-cells.* Next, it was determined whether the IL-32$\gamma$ produced by Hka could be induced and/or up-regulated by naïve or activated human T-cells. For these experiments, we employed a validated cell-cell contact bioassay system (97, 98) (Figure 40). As shown in Figure 41, it was found that PMA+ionomycin-activated human H9 T cells, but not naïve H9 T cells, significantly (*P<0.05*) up-regulated Hka lysate-derived IL-32 levels indirectly using a transwell insert. There was a H9-cell dependent IL-32 increase through +48 hrs; Hka IL-32 levels were highly significant (*P<0.01*) in comparison to non-activated H9 cells (Figure 41). We also found that the IL-32 mRNA levels correlated to the increased IL-32 ECL protein levels (Figure 42).

It was then determined whether H9 cells contacted to Hka in a direct cell-cell contact manner could induce IL-32. It was found that there was a significant *(P<0.05)* cell-dependent increase in IL-32at +48hr of Hka culture after cell-cell contact with activated H9 T cells. Finally, it was measured whether Hka lysate-derived IL-32 could be produced after cell-cell contact with primary human CD3+T cells. A significant (*P<0.001*) increase in activated T-cell-dependent increase in Hka-derived IL-32 protein was observed. These results demonstrate that there are both soluble and T-cell membrane-bound mediators present, both with primary human and human T-cell lines, which induce Hka to produce IL-32.

*Cell-cell contact interactions between human memory effector T cell subpopulations and Hka to produce IL-32 y production.* Pathogenic effector memory T cells have been shown to play a critical role in psoriasis by triggering the chain reaction of the cellular and molecular networks in the formation of psoriatic lesions (99, 100). To better determine which effector memory CD4+ T cell population is important in up-regulating Hka-derived IL-32, we obtained CD3+ T cells from healthy human volunteers and, after *in vitro* activation with PMA+ionomycin, these T cells were further purified using magnetic beads and sorted using flow cytometry. Separation protocols were used to purify CD4+CD45RO+ and CD4+CD45RA+ T cell populations since both of these populations have been found in psoriatic tissue (101, 102). Although there did not appear to be distinct differences in the forward scatter of the control, non-activated CD4+RO+ and CD4+RA+ populations, there did appear to be more CD4+CD45RA+ T cells present. After T-cell activation, there appeared to be at least two distinct populations of CD4+CD45RO+ T cells in comparison to the activated CD4+CD45RA+ T cells (Figure 43A). Since the chemokine CCR4 has previously been shown to be critically important in intraepidermal T cell migration to psoriatic dermis (103, 104), magnetic beads were used to further sort these populations into CD4+CD45RO+CCR4+, CD4+CD45RO+CCR4-, CD4+CD45RA+CCR4+, and CD4+CD45RA+CCR4- T cell populations. There appeared to be more abundant and brighter CD4+CD45RO+CCR4+ cells present than CD4+CD45RA+CCR4+ T cells. Using the isolated T cell subpopulations CD4+CD45RO+CCR4+, CD4+CD45RO+CCR4-, CD4+CD45RA+CCR4+, and CD4+CD45RA+CCR4-, the ability of each individual population was compared to stimulate proinflammatory cytokine production by Hka after direct cell-cell contact through +48 hours. Supernatants and lysates obtained from the CD4+CD45RO+CCR4+ T cell/Hka co-cultures measured for IL-32, TNFα, and IL-1β demonstrated statistically significantly *(P<0.05)* increased production of each Hka-derived cytokine when compared to the other T cell subpopulations tested (See Figure 43A). It appears that CD4+CD45RO+CCR4+ and CD4+CD45RA+CCR4+ T cells are important for this process since CCR4- T cells were not as effective at up-regulating Hka-derived cytokines (Figure 43B). These are the first data to demonstrate that distinct human effector memory T cell populations can induce Hka-derived IL-32γ in a cell-cell contact fashion.

**Example 3: Human keratinocyte production of TNFα is significantly reduced by inhibition of FKBP52 and CD147 (EMMPRIN)**

Using methods described in Example 2, a cell-cell contact bioassay of Hka and human Th22 T cells was carried out using monoclonal antibodies specific to FKBP52 and CD147. When anti-FKBP52 was added alone, the reduction in TNFα production was significantly reduced by approximately 60% (Figure 52; group 4). When anti-EMMPRIN was added alone, the reduction in TNFα production was significantly reduced by approximately 70% (Figure 52; group 6). Dual inhibition using both anti-EMMPRIN and anti-FKBP52 resulted in nearly complete inhibition of TNFα production, the reduction reaching almost 95%. These data show that inhibition of FKBP52 and CD147, either alone or in combination, could markedly reduce inflammation in populations of keratinocyes in vivo and could show potential for an effective treatment in psoriasis (or other inflammatory condition) patients.

**Example 4: Characterization of the extracellular region of EMMPRIN in solution and its interaction with its enzyme ligand, cyclophilin A (CypA)**

Materials and Methods.

*Protein expression, refolding, and purification.* In general, unlabeled proteins were produced in LB broth while labeled proteins were produced in M9minimal media supplemented with required combinations of 15N ammonium chloride, 13C-glucose, and 99% D2O for NMR studies. All proteins were expressed in BL21 (DE3) cells, grown at 37 °C, and protein expression was induced using 0.1 mM β, D-thiogalactopyranoside at an optical density of approximately 0.6 (600 nm). CypA was purified as previously described (49, 63) while both CD147 and the extracellular region of syndecan-1 were purified as described below. Unless otherwise noted, all columns and resins were used on an AKTA FPLC purchased from GE Healthcare.

For CD147, all constructs were PCR-amplified from the previously described vectors 24 and ligated into the pET15b vector (EMD Biosciences, Inc., San Diego, CA) using Ndel and BamHI. All primers contained the appropriate restriction sites with six base-pair overhangs for cleavage prior to ligation. All CD147 proteins were refolded at 4 °C as follows. For a typical 2-L growth, cells were lysed via sonication in 35 ml of 100mMTris, pH 7.5, 0.5MNaCl, and 1 mM ethylenediaminetetraacetic acid, centrifuged at 12,000g in a Sorvall SS-34 rotor (Sorvall, Asheville, NC), and the insoluble fraction was collected. The insoluble fraction was sonicated to homogeneity in 35 ml of denaturation buffer (5 M guanidine, 100 mM Tris, pH 7.5, 100mMNaCl, and 2-mercaptoethanol), centrifuged again, and dialyzed into refolding buffer (1 M arginine, 100 mM Tris, pH 7.5, and 100 mM NaCl) for 48 h. Samples were subsequently dialyzed into 100 mM Tris, pH 7.5, and 300 mM NaCl for at least 12 h and centrifuged to remove any precipitation. The soluble refolded proteins were then further dialyzed into 50 mM NaPO4, pH 7.5, 0.5 M NaCl, and 10 mM imidazole for purification via Ni Sepharose equilibrated in the same buffer and eluted in the same buffer supplemented with 0.4 M imidazole. Eluted proteins were cleaved overnight with thrombin; purified via a pre-packed S-100 size-exclusion column equilibrated with 50 mM NaPO4, pH 6.5, 150 mM NaCl, and 50 mM imidazole; and then applied to a 1-ml benzamidine column in the same buffer to remove residual thrombin.

For syndecan-1, the extracellular region that corresponds to residues 23-251 was PCR-amplified from the commercially available plasmid (Open Biosystems, Huntsville, AL) and ligated into the pET15b vector (EMD Biosciences, Inc.) using Ndel and BamHI sites. Primers contained the appropriate restriction sites for cleavage prior to ligation. While there was no measurable expression of this pET15b-syndecan-1 from this constructed vector, subsequent insertion of streptococcal protein G (GB1) led to high protein expression. This was accomplished by PCR amplification of the 56-residue GB1 encoding region (vector described below) with Ndel restriction sites both 5' and 3' and subsequent insertion into the single Ndel-digested pET15b-syndecan-1 plasmid. Upon confirming that the GB1 sequence was inserted correctly, all further syndecan-1 purifications utilized this pET15b-GB1-syndecan-1 fusion that was entirely found in the soluble expressed fraction both before and after thrombin cleavage that released syndecan-1 residues 23-251 (with an N-terminal Gly-Ser). For a typical 2-L growth, cell pellets were lysed with 35 ml of 50mMNaPO4, pH7.5, 0.5 M NaCl, and 10 mM imidazole and applied directly to a Ni Sepharose column in the same buffer. All subsequent purification steps were identical with that of the CD147 proteins described above.

*Peptide expression and purification.* A novel protocol for recombinant peptide production was employed to produce a peptide corresponding to CD147 residues 205-214, CD14710mer. Unless otherwise noted, all columns and resins were used on an AKTA FPLC purchased from GE Healthcare. Using standard PCR amplification, we constructed a PCR product with a 6×His, a thrombin site, and then the sequence of CD147 205-214 (HLAALWPFLG). A 5' BamHI site and a 3' EcoRI site were also included, and the PCR product was digested with the appropriate enzymes. Ligation of this PCR product into the pET30b vector that encoded GB1 (a kind gift from Gerhard Wagner, Harvard University) produced a vector encoding GB1 followed by our peptide of interest. This GB1-peptide fusion protein was expressed in BL21 (DE3) cells in LB broth supplemented with kanamycin. Cells were lysed in 50mMNaPO4, pH7.5, 0.5MNaCl, and 10 mM imidazole and then purified via a Ni Sepharose column equilibrated in the same buffer. The peptide was cleaved from this purified fusion with thrombin at room temperature for 12 h, leaving an N-terminal Gly-Ser followed by the CD147 residues 205-214 and then reapplied to Ni Sepharose resin in a gravity flow column. The pH of the eluted sample was decreased to 2 with trifluoroacetic acid (TFA) and then applied to a reversedphase column (Agilent, Zorbax SB300, 9.6 mm×25 cm) using an HPLC (Agilent Technologies, Inc., Santa Clara, CA) equilibrated in 0.2% TFA/water and 0.18% TFA/ acetonitrile solvent system. Finally, fractions containing the peptide were verified by analytical HPLC (Agilent, Zorbax SB300, 4.6 mm×15 cm) and mass spectrometry, pooled, and lyophilized for further use.

*Analytical size-exclusion chromatography, gel electrophoresis, and Western blot analysis.* Approximately 1 mg of the indicated CD147 purified proteins was applied to an analytical Superose-12 column (column volume, ~24 ml) that was equilibrated in the same buffer utilized for most NMR experiments (50 mM NaPO4, pH 6.5, 150 mM NaCl, and 50 mM imidazole). Protein samples for SDS-PAGE contained 10 µl of 0.02mM protein either with or without DTT, and the same concentrations of protein were used for native PAGE analysis. For Western blot analysis, SDS-PAGE gel electrophoresis of 10 µl of 0.02 mM recombinant CD14722-205 was performed in the absence and presence of 1mMDTTand then boiled for 5 min. The gel was transferred to an Immobilon PVDF membrane and blotted with 1:1000 Human Monoclonal EMMPRIN (R&D Systems, Minneapolis, MN) followed by 1:3000 anti-mouse horseradish peroxidase (Cell Signaling Technology, Danvers, MA). Protein band detection was performed with the ECL Chemiluminescence Detection Kit (Perkin Elmer, Waltham, MA) at three different exposure times (10, 30, and 60 s).

*NMR sample preparation, spectroscopy, and data analysis.* All NMR spectra were collected at 25 °C on a Varian 600 -, 720- , 800-, or 900-MHz spectrometer in 50 mM NaPO4, pH 6.5, 150 mM NaCl, and 50 mM imidazole supplemented with 7% D2O. There were only modest changes to the resonance positions with imidazole present (data not shown), yet imidazole did increase the lifetime of each sample slightly and, thus, was maintained in all samples. Samples utilized for assignment purposes contained 0.5-1 mM protein, while all samples utilized for titration purposes contained 0.5 mM protein with the indicated final concentration of the titrant. All spectra were processed using NMRPipe software65 and analyzed using CCPNmr software.66 Unless otherwise noted, all pulse sequences were obtained from standard Varian Biopack libraries. Transverse relaxation optimized spectroscopy (i.e., TROSY-based) HSQC experiments were used only when noted.

The backbone assignments of several CD147 constructs that included CD14722-205 and CD14794-205 as well as constructs containing the CypA target of CD147 Pro211, CD14722-214, and CD14794-214 were determined. Standard multidimensional NMR experiments that included an HNCACB and CBCA(CO)NH for each construct were used, while both a 3D-15N-NOESY and a 3D-15N-TOCSY (total correlated spectroscopy) were useful in confirming each spin system.67 Our resonance assignments that were stored within the CCPNmr database were used to calculate secondary-structure propensities using the program TALOS.43

For relaxation experiments, standard R1 and R2 relaxation experiments were applied with recycle delays of 2.5 s at 900 MHz. Relaxation delays for R1 experiments were 0.01, 0.1, 0.3, 0.5, 0.7, 0.9, and 1 s, and relaxation delays for R2 experiments were 10, 30, 50, 70, and 90 ms. While compensating pulses prior to the recycle delay were utilized for R2 measurements to account for potential sample heating, all relaxation experiments were arrayed within a single experiment to also account for any potential field in homogeneities. Correlation times for each amide were calculated using R2/R1 ratios as described in Larsson et al.44 and correlation times for each domain using all R2/ R1 ratios within one standard deviation of the mean. Correlation times for each domain were calculated using FAST-Modelfree employed Modelfree version 4. 45 Since calculations using Modelfree have recently been shown to become trapped in local minima,68 a range in initial correlation times from 11 to 18 ns was used with 1-ns increments. While most calculations converged to the reported values and local minima were readily excluded due to large chi-squared values. In-house scripts that combined both NMRPipe and Gnuplot software were used to fit all relaxation rates using peak heights.

For ZZ-exchange experiments, the pulse scheme of Farrow et al. was employed with a selective adiabatic CHIRP pulse applied in the center of the mixing period for water suppression. (69) Two data sets were collected at 720 MHz on two samples with either 0.01 or 0.05 mM CypA and both containing 0.5 mM $^{15}$N-CD147$^{94-214}$ at 25 °C and analyzed as previously described to calculate the apparent *cis/trans* exchange rate ($k_{ex}$). (36,70,71) Briefly, 14 mixing times ($\tau_m$) were acquired for each of these two data sets ranging from 0 to 840 ms, and R1 relaxation rates used in fitting these data, that is, R1 for *cis* (R1$_c$) and R1 for *trans* (R1$_t$), were collected directly on these samples. Equations (1a), (1b), (1c), and (1d) below were used to simultaneously fit the auto *trans* peak ($I_{tt}$), the auto *cis* peak ($I_{cc}$), the *trans→cis* exchange peak ($I_{tc}$) and the *cis→trans* exchange peak ($I_{ct}$), respectively, as a function of $\tau_m$● $I_{tt}(0)$ and $I_{cc}(0)$ are the initial intensities of the *trans* and *cis* auto peaks, respectively, $a_{11}$=R1$_t$+$k_{tc}$, $a_{22}$=R1$_c$+$k_{ct}$, $a_{21}$=-$k_{tc}$, and $a_{12}$=-$k_{ct}$. Finally, under these conditions, $k_{tc}$ and $k_{ct}$ can be recast as a function of their free $K_{eq}$ (P$_{cis}$/P$_{trans}$=0.33/0.67), and thus, $k_{tc}$=0.33$k_{ex}$ and $k_{ct}$=0.67$k_{ex}$.

$$I_{tt}(\tau_m) = I_{tt}(0)(-(\lambda_2 - a_{11})exp(-\lambda_1\tau_m) + (\lambda_1 - a_{11})exp(-\lambda_2\tau_m))/ (\lambda_1 - \lambda_2) \qquad (1a)$$

$$I_{cc}(\tau_m) = I_{cc}(0)(-(\lambda_2 - a_{22})exp(-\lambda_1\tau_m) + (\lambda_1 - a_{22})exp(-\lambda_2\tau_m))/ (\lambda_1 - \lambda_2) \qquad (1b)$$

$$I_{tc}(\tau_m) = I_{tt}(0)(a_{21}exp(-\lambda_1\tau_m) + a_{21}exp(-\lambda_2\tau_m))/(\lambda_1 - \lambda_2) \qquad (1c)$$

$$I_{ct}(\tau_m) = I_{cc}(0)(a_{12}exp(-\lambda_1\tau_m) + a_{12}exp(-\lambda_2\tau_m))/(\lambda_1 - \lambda_2) \qquad (1c)$$

GraphPad Prism version 4.0 (GraphPad Software Inc., San Diego, CA) was used to simultaneously fit all resolvable peaks within CD147 residues 207-214. Both Ala207 and Gly214 could not be used since their respective *trans* and *cis* resonances exhibited little to no dispersion in the nitrogen dimension.

*Recombinant CD147 activity.* Recombinant CD14722-205 was added to differentiated THP-1 cells at 50, 100, and 200 μg/ml final concentrations for 8 h, and the amount of cytokine released from the cells was measured by sandwich ELISA (ElisaTech, Aurora, CO). THP-1 cells were cultured in RPMI (supplemented with 10% fetal bovine serum) and stored at 37 °C and 5% CO2 at all times. Differentiation of THP-1 cells was achieved by adding 100 nM phorbol-12-myristate-13-acetate to 1 ml suspended cells in a 24-well cell culture dish at 1 million cells/ml for 24 h, removing undifferentiated cells by aspiration, washing with phosphate buffered saline (PBS), and then replacing the media. The cells were then incubated with CD147, and cytokines secreted into the media were measured by ELISA. ELISAs were performed under the manufacturer's protocol (ElisaTech).

Assessing proper refolding of recombinant CD147 and probing CD147 oligomerization

Intracellularly expressed CD147 constructs in bacteria are initially unfolded due to improper disulfide formation but can be refolded *in vitro* to yield milligram quantities of highly soluble and biologically active proteins. The extracellular region of the dominant CD147 isoform (isoform 2, which is CD147[22-269]) contains two Ig-like domains, herein called Ig1 (residues 22-103) and Ig2 (residues 105-205), and each contains two cysteine residues that forma single disulfide bond within each domain. The extracellular region of a less widely found CD147 isoform comprises only the Ig2 domain (isoform 3, which is CD14794-269). The presence of these disulfide bonds was recently confirmed by the X-ray crystal structure of the CD147 extracellular region purified from a bacterial periplasmic expression system that relies on spontaneous folding within this oxidizing environment. (34) However, here we have discovered that both of these isoforms can be readily refolded in their entirety after intracellular bacterial expression to produce soluble proteins (both with and without the signal peptide sequence of residues 1-21, although these residues lead to soluble oligomers). While we can produce many soluble constructs of CD147 that represent different regions of the receptor via our *in vitro* refolding method, CD147 constructs containing residues 215-229, which correspond to the hydrophobic transmembrane (TM) region, form large aggregates as assessed by size-exclusion chromatography (Figure 45A, CD147[22-269]). Removal of residues 205-229 that comprise both the membrane-proximal and TM region completely abrogates aggregation (Figure 45A, CD147[22-269]Δ™). Refolded CD147 constructs comprising the extracellular Ig-like domains were all found to elute as a single monomeric species on size exclusion and were therefore further analyzed by both SDS-PAGE denaturing gels and native gel analysis below.

Three CD147 constructs representing either the individual Ig-like domains alone, CD147[12-103] (Ig1) and CD147[94-205] (Ig2), as well as both domains, CD147[22-205] (Ig1 and Ig2), were used to probe both proper disulfide formation and the oligomeric properties of CD147. The single disulfide bond within each extracellular CD147 domain was correctly formed by our refolding procedure as confirmed by SDS-PAGE denaturing gels where the presence of dithiothreitol (DTT) reduced each disulfide bond and led to a slower migrating species for all constructs (Figure 45B). Furthermore, all extracellular CD147 constructs migrate as a single species on a native gel (Figure 45C) and, together with the aforementioned size-exclusion data, suggest that with proper disulfide formation, each Ig-like domain neither self-associates nor interacts with the other Ig-like domain.

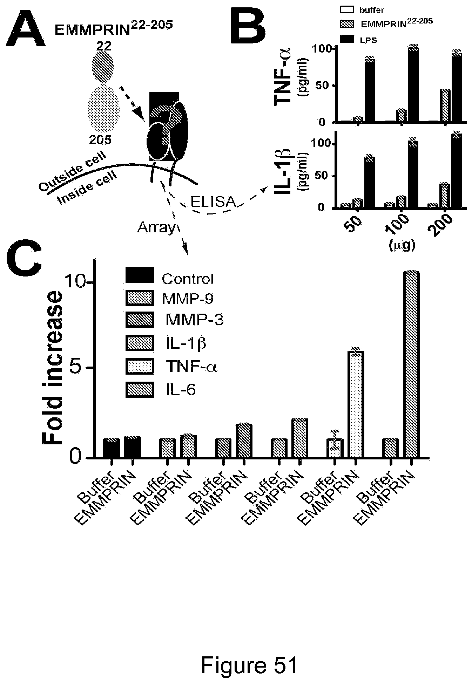

A final confirmation of proper refolding is shown in the biological activity of our recombinant CD147. Specifically, our refolded recombinant CD147[22-205] stimulates the release of two well-characterized pro-inflammatory cytokines in cell culture, TNFα and IL 1β (Figure 51), providing the first evidence that glycosylation of CD147 is not required for cytokine stimulation as has recently been shown for MMP stimulation.

Considering that several recent reports have identified trace amounts of CD147 dimerization using one of the exact same constructs that we have begun characterizing here, namely, Cud147[22-205], it is important to rationalize why we do not observe such a phenomena.14,34 The most likely explanation for these observed differences is intermolecular disulfide bond formation. Specifically, the previous purification schemes of recombinant CD147[22-205] relied on spontaneous refolding in the oxidizing environment of the Escherichia coli periplasm and this likely leads to a small population of incorrectly folded protein, which would be susceptible to intermolecular disulfide formation. In contrast, our *in vitro* refolding procedure may simply be more efficient since we do not observe such dimers on SDS-PAGE or native gel analysis. However, such cross-linked dimers can still be observed within our protein preparations using Western blot analysis, indicating that even our purification procedure produces trace amounts of this cross-linked dimer, albeit at far lower relative concentrations to the monomeric species. While we suspect that these dimers are simply a by-product of purification rather than a biologically relevant protein, it would be naïve to completely rule out a potential role of disulfide shuffling for CD147 homophilic interactions. However, receptor interactions that rely on disulfide shuffling require other protein interactions such as protein disulfide isomerase (41) and such interactions have not been identified for CD147. Regardless of such hypothetical interactions, our data indicate that the extracellular region of the intact fully folded monomeric extracellular domains of CD147 do not self-associate in the absence of another mediator.

NMR solution studies of the CD147 Ig-like domains

While the extracellular domains of CD147 neither self-associate nor interact with each other under the concentrations used for native gels (i.e., low micromolar concentrations), receptors implicated in cellular adhesion, such as CD147, sometimes exhibit weak affinities. Since several reports have indicated that CD147 acts as its own receptor analogous to other cellular adhesion receptors, (42) such affinity could potentially be under the detection limit of our native gel analysis performed above. In contrast, NMR solution experiments can be used as particularly sensitive markers of such protein/protein interactions on multiple timescales and are therefore well suited to probe interactions with various affinities. For example, NMR titration experiments offer an obvious means of monitoring interactions that exhibit affinities on the order of the concentrations utilized (micromolar to millimolar concentrations). Additionally, NMR relaxation experiments provide the dynamic details of each residue and, thus, if the individual Ig-like domains of CD147 self-associate, then this may be detected at atomic resolution. To this end, we have completed the backbone resonance assignments of the extracellular regions of the two most prevalent CD147 isoforms CD147[22-205] (isoform 2 comprising both Ig1 and Ig2) and CD14794-205 (isoform 3 comprising Ig2) in order to characterize CD147 solution properties and its potential interactions at atomic resolution (Supplementary Material, Tables S1 and S2 and BMRB accession numbers 16322 and 7433).

A comparison of the aforementioned X-ray crystal structure of CD147[22-205] with our NMR solution studies indicates that there are both similarities and differences between the crystal structure and the solution structure. In regard to the monomeric structure in solution, the secondary-structure propensities of CD147[22-205] were calculated from our resonance assignments using the program TALOS (43) and are largely consistent with that found in the crystal. In other words, the monomeric structure in solution is highly similar to that in the crystal. The only major difference is that the resonances corresponding to residues 151-159, which comprise a short $\alpha$-helix and part of $\beta$-strand "D" in the crystal, are not observed in solution. (34) This indicates that at least part of residues 151-159 undergo chemical exchange (i.e., conformational exchange between two different chemical environments) on the intermediate NMR timescale and, therefore, sample multiple conformations with exchange rates that are similar to the chemical shift differences between sampled states (i.e., intermediate exchange).

NMR spectra, NMR relaxation experiments, and NMR titration experiments all indicate that the intermolecular contacts observed in the crystal are not maintained in solution. The extracellular regions of both CD147 isoforms give rise to high-quality spectra and are shown here in the context of 2D $^{15}$N edited heteronuclear single quantum coherence (HSQC) spectra (Fig. 9). While the high dispersion is indicative of characteristically well-folded proteins, the relatively narrow line widths observed in solution already provide evidence that the four proposed subnanomolar interactions in the crystal do not persist in solution (Fig. 9A and B, top right). R1 (longitudinal) and R2 (transverse) relaxation rates measured here define the correlation times of each amide and indicate that CD147 residues 22-101 (i.e., Ig1) and residues 105-205 (i.e., Ig2) are independently rotating domains within CD147[22-205] (Fig. 9A and B, bottom). The correlation time for each domain was calculated from all residues with R2/R1 ratios within one standard deviation using the equation described by Larsson et al., (44) and the differences are immediately apparent in the raw data. For example, the average R2 rates are about 22 Hz for residues 22-101 and 35 Hz for residues 105-205, indicating that these domains exhibit markedly different tumbling times in solution. While these differing R2/R1 ratios are consistent with the two independently folded Ig-like domains found within the X-ray crystal structure, (34) the fact that these domains tumble with different correlation times provides evidence that the inter-domain interactions observed in the crystal are not maintained in solution. In other words, the CD147 Ig1 and Ig2 domains would be expected to exhibit the same correlation time if they self-associated in solution. To rule out the possibility of anisotropic rotation, correlation times for each of the individual domains were also calculated using the FAST Modelfree (Facile Analysis and Statistical Testing for Modelfree) analysis (45) for each domain with anisotropic model and an anisotropic model. For the anisotropic model, each of the respective domains within the X-ray crystal structure was used. Correlation times calculated using an isotropic model were 11.8 and 14.1 ns for Ig1 and Ig2, respectively, and for an anisotropic model, the correlation time for Ig1 was found to increase slightly to 12.6 ns while the correlation time for Ig2 was the same as that of the isotropic model. Thus, regardless of rotational anisotropy, the major finding from these relaxation experiments is that the tumbling times of the two CD147 Ig-like domains in solution differ by at least 1.5 ns. Consistent with these findings, no chemical shift changes were observed upon a titration of 15N CD147[14-205] with stoichiometric amounts of CD147[22-103], thereby proving that these two domains do not interact as separate entities even weakly (i.e., Kd~1.0 mM). Moreover, no concentration-dependent chemical shift changes are observed for CD147[22-205] that would be expected to occur if there was even a weaker self-association (i.e., KdN1 mM). Finally, the R2/R1-derived correlation times for each domain and the observed line widths are completely consistent with monomeric proteins. Thus, the NMR solution data are consistent with the aforementioned native gels that also indicate that CD147 homophilic interactions do not occur through the extracellular region in the absence of another mediator.

NMR studies also offer important insight into residues 94-101 that comprise the N-terminal portion of the extracellular region of CD147 isoform 3 (i.e., CD147[94-269]). Namely, both resonance-based secondary-structure propensities and relaxation rates indicate that residues 94-101 form a $\beta$-stand only in the context of Ig1 but are disordered within the context of this shorter CD147 isoform 3 that only comprises Ig2 (Figure 9B). With the use of our resonance assignments for this CD147 construct, no secondary-structure propensities were predicted by TALOS and the corresponding low R2 rates and high R1 rates are consistent with disordered residues (Figure 9B, bottom).

Identification and initial characterization of the CypA target site of CD147

A region within CD147 exists in an inherent *cis/trans* equilibrium dictated by a central proline residue and that CypA both enhances the rate of this interconversion and likely alters the *cis:trans* ratio upon complex formation. Moreover, this preexisting equilibrium may be a general phenomenon for biological substrates of cyclophilins since an unusually high *cis* content has also been observed for other CypA protein substrates. (19,36,37) Several previous studies have suggested that CypA could target both CD147 Pro180 (24) and Pro211, (46) yet cyclophilin binding to CD147 has only been investigated within the context of peptides.(46,47) Thus, we now provide the first direct evidence that shows that CypA exclusively targets CD147 Pro211, and not Pro180, in the context of the fully folded protein. Furthermore, CypA catalyzes peptidyl-prolyl isomerization of only this single Pro211 site. Our determination of the CD147 resonance assignments allows for the characterization in solution of this receptor with its enzyme ligand CypA that has itself previously been assigned in solution both free (48) and in the context of several substrates. (36,49)

NMR titration experiments were first conducted using 15N-CypA with unlabeled CD147, and chemical shift changes were only observed for extracellular recombinant CD147 constructs that included the membrane-proximal region of CD147 residues 206-214. No interactions between CypA and the Ig-like domains of CD147 were detected using either CD14794-205 or CD14722-205 (data not shown), while identical chemical shift changes were observed for two constructs containing residues 206-214, CD14794-214 and CD147[22-214] (Figure 46A). These CD147 residues are predicted to lie at the surface of the extracellular TM region, and some of these residues may even lie within the membrane itself. However, both extracellular cyclophilin ligands of CD147, CypA and CypB, are known to readily traverse the membrane. (20) Upon titration of these latter CD147 comprising residues 206-214 to 15NCypA, both CypA amide residues (Figure 46A) and the single CypA indole of Trp121 (Figure 46B) were found to exhibit chemical shift changes that were all localized to the CypA active site (Figure 46C, left). Thus, CypA specifically engages CD147 within residues 206-214 through its active site and there is only a single potential targeted residue, Pro211, within this region (Figure 46C, right). Furthermore, only a single set of CypA resonances was observed during such titrations, indicating that exchange between the free and bound states is fast on the NMR chemical shift timescale and the affinity of these two proteins is relatively weak. Also in support of such a weak affinity of the CypA/CD147 complex, the measured chemical shift changes were linear over the titration range employed, indicating our inability to fully saturate all CypA binding sites (CD147 constructs were not stable beyond 1-2 mM). Our data therefore imply that the binding affinity of CypA/CD147 is weaker than the highest CD147 concentration used (i.e., KdN1 mM since 1 mM was our final titration point) and is consistent with the relatively weak affinity cyclophilins have for most of their substrates. 50 Such weak affinity also explains the failure of previous pull-down experiments.24 Thus, CypA targets CD147 within residues 207-214 specifically but with relatively weak affinity.

While the relative weak affinity of CypA for CD147 residues 206-214 does not allow for saturating concentrations necessary to accurately determine their binding affinity, we have used a peptide representing this region of interest as an alternative method. Specifically, we have estimated the affinity of CypA to CD147 residues 206-214 using a peptide, herein called the CD147[10mer] (sequence of 205-HLAALWPFLG-214) (SEQ ID NO:1), which corresponds to the CypA target site on CD147. The use of a representative peptide for this CD147 target site was warranted based on both R1 and R2 measurements, since this membrane-proximal region is highly flexible on the fast timescale. In accord with previous studies that have suggested that the CypA/CD147 interaction is of relative weak affinity, a Kd of 4.2±0.2 mM was calculated from the binding isotherms between 15N-CypA and the CD147[10mer] (Figure 47A). Many more residues exhibit chemical shift changes using this peptide due to our ability to titrate to saturating conditions (Figure 47B) and, as expected, these perturbations are still confined to the active site, confirming CypA specificity for this peptide (Figure 47C).

Upon reversing the labeling scheme to monitor CypA-induced changes to 15N-CD14722-214, two sets of resonances were found for residues 207-214 corresponding to an inherent *cis/trans* conformational exchange of Pro211 (Figure 48A-B, black spectra). A similar preexisting conformational equilibrium has been observed for the two CypA substrates studied to date, the HIV-1 capsid (HIV-1 CA)36,37 and Itk.(19) In CD147, the mutation of this proline to an alanine, that is, CD147P211A, probed in the context of either CD14722-214 or CD14794-214, resulted in only a single set of resonances, confirming that proline isomerization is the underlying cause of this inherent conformational heterogeneity. Many of the resonances within CD147P211A also overlapped with those resonances corresponding to the major conformation observed in the wild-type receptor, simultaneously confirming that the *trans* conformer dominates in the wild-type receptor and allowing us to assign the *cis* resonances. From the relative peak intensities in wild-type CD147, a *cis:trans* ratio of 33%:67% was calculated (i.e., 33% of CD147 Pro211 molecules are in the *cis* conformation), which is about twice the *cis* content as that of the HIV-1 CA36 and very similar to that of Itk. (51) Thus, all three CypA protein substrates that have now been studied at the molecular level, that is, the HIV-1 CA, Itk, and CD147, exhibit this intrinsic conformational equilibrium of the targeted proline with an unusually high *cis* content. Moreover, it is likely that enzyme-mediated catalysis alters these equilibriums (upon complex formation) as described below.

Evidence for CypA-mediated catalysis of CD147 was initially provided by a spectral comparison of free 15N-CD14794-214 to that titrated with stoichiometric concentrations of unlabeled CypA (Figure 48A-B). The fact that two distinct sets of resonances are observed for free 15N-CD147 residues 207-214 means that the intrinsic rate of peptidyl-prolyl isomerization is slow on the NMR timescale (b0.1 s[-1]). In contrast, upon addition of stoichiometric concentrations of CypA, amide resonances within $^{15}$N-CD147 residues 207-214 either converge or are so severely linebroadened that they disappear (Figure 47A and B, respectively). This implies that in the presence of CypA, CD147 undergoes a conformational exchange on both the fast and the intermediate NMR timescale. In other words, the intrinsic interconversion of CD147 Pro211 is higher in the presence of CypA, and therefore, the conformational exchange of peptidyl-prolyl interconversion of Pro211 is enhanced by CypA-mediated catalysis. Those residues that converge undergo "fast exchange" and those residues that disappear undergo "intermediate exchange". However, this exchange is not simply relative to the bound chemical shift differences, since it also includes exchange to the free states due to the weak affinity of the complex. Finally, linebroadening of the active-site $^{15}N$-CypA amide resonances is also observed above with the addition of unlabeled CD147 constructs, yet these do not completely disappear because it is the CypA side chains that likely make direct contact with the CD147 substrate.

Beyond the observed line broadening of CD147 resonances in the presence of CypA, two additional NMR experiments provided direct experimental proof for CypA-mediated rate enhancement of CD147 peptidyl-prolyl isomerization. The appearance of exchange peaks between the corresponding 15N-CD147 *cis* and *trans* resonances was observed in the presence of catalytic amounts of the enzyme for both "ZZ-exchange" experiments (Figure 48C) and within standard 3D-nuclear Overhauser enhancement spectroscopy (NOESY) experiments (Figure 48D). Such exchange peaks arise if the apparent rate of catalysis allows for transfer of magnetization between the two sampled states (i.e., *cis* and *trans*), occurring within the time frame of the experiment (i.e., the mixing times), and are only observed in the presence of CypA. Thus, the appearance of exchange peaks within both of these experiments indicates that CypA increases the intrinsically slow exchange of Pro211 and once again provides evidence thatCD147 is a substrate of its CypA enzyme ligand.

Characterizing CypA-mediated catalysis of CD147\

ZZ-exchange was used to determine the apparent catalytic exchange rate of CypA-mediated peptidyl-prolyl isomerization of CD147 Pro211, providing a quantitative analysis of the active CypA/CD147 complex (Figure 49). Specifically, two ZZ-exchange experiments were collected using identical concentrations of 0.5 mM 15N-CD147$^{94-214}$ but with two substoichiometric concentrations of the enzyme (i.e., catalytic concentrations) of 0.01 mM (Figure 49A) and 0.05 mM (Figure 49B) CypA. Under each condition, the apparent exchange rate ($k_{ex}$), which is the sum of the apparent *cis→trans* (i.e., $k_{ct}$) and *trans→cis* (i.e., $k_{tc}$) catalytic rates, was determined to quantify the rate enhancement of CypA-mediated catalysis (Table 1).

**Table 1.** Apparent catalytic exchange rates of CypA-mediated interconversion of CD147 residues 207-214 determined from ZZ-exchange.

| [CypA] | $k_{ex}$ (s-1) | Fits used |
|---|---|---|
| 0.01 mM | 9.6 ± 3.5 | 24 |
| 0.05 mM | 44.3 ± 8.1 | 20 |

Both the raw data and fits are shown for CD147 Trp210$^{N\varepsilon1}$ for all four peaks (i.e., both auto peaks and both exchange peaks), which is adjacent to the catalyzed CD147 Pro211. A verification of our sample preparations is shown by the fact that the maximum cross-peak intensities occur approximately 5-fold apart and corresponds to the 5-fold difference in enzyme concentrations (Figure 49A and B spectra shown in center). For example, we observe maximum cross-peak intensities for CD147 exchange peaks at ~240 ms and ~48 ms for the 0.01 mM CypA sample and the 0.05 mM CypA sample, respectively. These data along with all nonoverlapping amides within the CypA target site of CD147 residues 207-214 were used to calculate the apparent catalytic rate constants at each CypA concentration as shown in Table 1. Using our quantitative estimation of the Kd for the CypA/CD147 complex derived from our model peptide substrate above, 0.2% and 1.1 % complex relative to the free $^{15}$NCD147$^{94-214}$ would exist for 0.01 and 0.05 mM CypA, respectively (see Supplementary Material, Section 2). Thus, extrapolating these apparent exchange rates (Table 1) to the fully bound complex gives approximately 4000-4800 s$^{-1}$ for CypA-mediated interconversion of CD147 at 25 °C. While this is only a rough approximation of the CypA-mediated exchange rate, it is clear that CypA provides a significant rate enhancement relative to the uncatalyzed *cis/trans* exchange rate of peptidyl-prolyl isomerization that is less than 0.1 s$^{-1}$. (52) However, this rate enhancement is less than that in our previous study of a model peptide substrate measured at the same temperature.49 A possible explanation for this lower catalytic rate is that CD147 has a bulky Trp residue preceding the catalyzed CD147 Pro211 (i.e., CD147 Trp210-Pro211) that likely slows the interconversion rate relative to the model peptide substrates. In fact, in a study conducted nearly two decades ago with various amino acid substitutions preceding the catalyzed Pro, the authors found that the Trp-Pro sequence exhibited the slowest kct rate for human CypA.53 In conclusion, CypA catalysis serves to increase the uncatalyzed rate of interconversion by at least several orders of magnitude.

The extracellular region of CD147 does not self-associate

We have directly characterized the extracellular regions of the two most dominant CD147 isoforms, isoform 2 (CD147[22-269]) and isoform 3 (CD147[94-269]), using multiple solution techniques and have shown that neither self-associate in the absence of a mediator. Only single monomeric species were detected for all CD147 constructs that correspond to these extracellular regions as confirmed by gel filtration analysis, native gel analysis, and multiple NMR solution studies. In regard to the monomeric species, secondary-structure predictions based on our NMR resonance assignments are largely consistent with the X-ray crystal structure of the extracellular region of isoform 2, CD147[22-205], thereby confirming that the solution structure of the monomer is similar to that within the crystal. The only noted difference is that resonances corresponding to CD147 residues 151-159 are not observed in solution, and thus, this region likely undergoes a conformational exchange within the intermediate timescale (relative to the chemical shift differences between sampled conformations). Interestingly, this same region contains one of the three potential N-linked glycosylation sites (Asn152) and implies that inherent flexibility may be important for function. However, there are likely no contacts between the Ig-like domains and no preferred orientation relative to each other as observed within the recently solved X-ray crystal structure of CD147[22-205]. (34) This conclusion is drawn from several experiments conducted here that indicate that the Ig-like domains themselves do not interact. Specifically, native gel analysis shows that both CD147 Ig1 and Ig2 domains exhibit the same migration patterns when combined as they do separately. Furthermore, in the context of the larger CD147 isoform 2, NMR relaxation experiments indicate that these domains exhibit very different correlation times in solution and, therefore, most likely tumble independently. No chemical shifts are observed within $^{15}$N-HSQC experiments upon labeling one domain and titration of the other domain, confirming that the Ig-like domains do not interact as separate entities. Thus, our data provide direct evidence of free rotation around the short linker region between these two Ig-like domains (i.e., CD147 residues 103-105 that comprise the sequence Gly-Pro-Pro, respectively). Finally, we have, for the first time, characterized the solution properties of the extracellular region of the second most abundant CD147 isoform, isoform 3 (i.e., CD147[94-269]). Here, we have found that the N-terminal residues 94-104 of isoform 3 are disordered as compared to the formation of a β-strand in the context of the Ig1 domain within the larger CD147 isoform 2.

Although the conclusions of several recent reports have suggested that a fraction of recombinant CD147 self-associates in solution, (14,34) there has been no direct proof that such an interaction occurs without mediation by another molecule *in vivo* (i.e., co-receptors or small molecules). CD147 oligomerization is likely a complicated process that involves mediation by other molecules such as a co-receptor to induce signaling (Figure 50A).

Intracellular signaling by CypA-mediated catalysis

While extracellular cyclophilins stimulate several intracellular signaling events through their CD147 receptor, the underlying mechanism has remained unclear. A direct interaction between cyclophilins and CD147 has been shown to occur both through yeast two-hybrid assays (24) and through the use of specific antibodies that block these cyclophilin/CD147 interactions. (23) Here, we have initiated studies that have allowed us to directly monitor the CypA/CD147 interaction in aqueous buffer during catalysis. Such studies are made possible due to the reversible nature of the cyclophilin-mediated reaction and serve as an important first step in understanding the link between peptidyl-prolyl isomerization on the outside of the cell and intracellular signaling. While both CypA and CypB have been shown to serve as the extracellular ligands of CD147, CypA is a more abundant protein and, thus, has been the focus of our studies here. Moreover, although NMR is a powerful technique that can reveal the atomic resolution details of molecular interactions, it is particularly useful in probing cyclophilin interactions that are of moderate to weak affinity. (50) Many weak but biologically important interactions occur in nature, which include adhesion proteins that exhibit micromolar affinities (59) and alcohols that exhibit millimolar affinities. (60) Thus, the high sensitivity of NMR to detect weak interactions was the initial impetus behind employing NMR methods to study the CypA/CD147 interactions at atomic resolution in solution.

Using NMR solution studies, CypA is shown to both bind and catalyze CD147 Pro211, a site predicted to lie on the outside of the cell and adjacent to the TM region of the receptor. This membrane-proximal region of CD147 residues 207-214 already exists in its free form in a *cis:trans* equilibrium characterized by an unusually high *cis* content of 33% (Figure 50B, left). However, this uncatalyzed *cis/trans* isomerization is very slow, as evidenced by our observation of both sampled *cis* and *trans* resonances for all residues within this region of CD147. Upon addition of stoichiometric concentrations of CypA, 15N-HSQC spectra collected on the active CypA/CD147 complex showed both the collapse of several *cis* and *trans* resonances and severe linebroadening for others, suggesting that a conformational exchange event occurs within the active CypA/CD147 complex (i.e., catalysis). Further proof of catalysis came from several NMR experiments collected under substoichiometric conditions, which unambiguously confirmed that CypA catalyzes CD147 Pro211. Namely, the appearance of exchange peaks in the both ZZ-exchange and 3D-NOESY experiments verifies a CypA-induced rate enhancement of the inherently slow *cis/trans* equilibrium of CD147 residues 207-214 and, therefore, provides direct evidence of CypA-mediated catalysis of its CD147 receptor. A quantitative analysis of our ZZ-exchange data reveals that the catalysis may be only slightly slower than that of nonnatural substrates previously studied, a finding reflective of the bulky CD147 Trp210 residue preceding the targeted Pro211.

Confirmation shown here that CD147 is a substrate of its CypA enzyme ligand along with several previous reports suggests an underlying mechanism for CypA-mediated signaling through the CD147 receptor (Figure 50B, right). Specifically, this interaction likely encompasses a "conformational switch" responsible for regulating downstream signaling, whereby the relative *cis:trans* ratio of free CD147 Pro211 is increased by way of its CypA interaction. Both crystallography and NMR studies support such a model.

## REFERENCES

1. Guo, H. M., Majmudar, G., Jensen, T. C., Biswas, C., Toole, B. P. & Gordon, M. K. (1998). Characterization of the gene for human EMMPRIN, a tumor cell surface inducer of matrix metalloproteinases. Gene, 220, 99-108.

2. Schmidt, R., Bültmann, A., Fischel, S., Gillitzer, A., Cullen, P., Walch, A. et al. (2008). Extracellular matrix metalloproteinase inducer (CD147) is a novel receptor on platelets, activates platelets, and augments nuclear factor {kappa}B dependent inflammation inmonocytes. Circ. Res. 107, doi:10.1161/CIRCRESAHA.107.157990.

3. Ruiz, S., Castro-Castro, A. & Bustelo, X. R. (2008). CD147 inhibits the nuclear factor of activated T-cells by impairing vav1 and rac1 downstream signaling. J. Biol. Chem. 283,5554-5566.

4. Gabison, E. E., Hoang-Xuan, T., Mauviel, A. & Menashi, S. (2005). EMMPRIN/CD147, an MMP modulator in cancer, development and tissue repair. Biochimie, 87, 361-368.

5. Zheng, H. C., Takahashi, H., Murai, Y., Cui, Z. G., Nomoto, K., Miwa, S. et al. (2006). Upregulated EMMPRIN/CD147 might contribute to growth and angiogenesis of gastric carcinoma: a good marker for local invasion and prognosis.Br. J. Cancer, 95, 1371-1378.

6. Tang, Y., Nakada, M. T., Kesavan, P., McCabe, F., Millar, H., Rafferty, P. et al. (2005). Extracellular matrix metalloproteinase inducer stimulates tumor angiogenesis by elevating vascular endothelial cell growth factor and matrix metal loproteinases. Cancer Res. 65, 3193-3199.

7. Velez, W. & Kyprianou, N. (2007). EMMPRIN is a biomarker of prostate cancer progression in TRAMP mice. FASEB J. 21, A621.

8. Xu, H. Y., Qian, A. R., Shang, P., Xu, J., Kong, L. M., Bian, H. J. & Chen, Z. N. (2007). siRNA targeted against HAb18G/CD147 inhibits MMP-2 secretion, actin and FAK expression in hepatocellular carcinoma cell line via ERK1/2 pathway. Cancer Lett. 247, 336-344.

9. Millimaggi, D., Mari, M., D'Ascenzo, S., Carosa, E., Jannini, E. A., Zucker, S. et al. (2007). Tumor vesicleassociated CD147 modulates the angiogenic capability of endothelial cells. Neoplasia, 9, 349-357.

10. Sidhu, S. S., Mengistab, A. T., Tauscher, A. N., LaVail, J. & Basbaum, C. (2004). The microvesicle as a vehicle for EMMPRIN in tumor-stromal interactions. Oncogene, 23, 956-963.

11. Taylor, P. M.,Woodfield, R. J., Hodgkin, M. N., Pettitt, T. R., Martin, A., Kerr, D. J. &Wakelam, M. J. O. (2002). Breast cancer cell-derived EMMPRIN stimulates fibroblast MMP2 release through a phospholipase A (2) and 5-lipoxygenase catalyzed pathway. Oncogene, 21, 5765-5772.

12. Zhu, P., Ding, J., Zhou, J., Dong, W. J., Fan, C. M. & Chen, Z. N. (2005). Expression of CD147 on monocytes/macrophages in rheumatoid arthritis: its potential role in monocyte accumulation and matrix metalloproteinase production. Arthritis Res. Ther. 7, R1023-R1033.

13. Zhu, P., Lu, N., Shi, Z. G., Zhou, J., Wu, Z. B., Yang, Y. et al. (2006). CD147 overexpression on synoviocytes in rheumatoid arthritis enhances matrix metalloproteinase production and invasiveness of synoviocytes. Arthritis Res. Ther. 8, 1-12.

14. Belton, R. J., Chen, L., Mesquita, F. S. & Nowak, R. A. (2008). Basigin-2 is a cell surface receptor for soluble basigin ligand. J. Biol. Chem. 283, 17805-17814.

15. Yoshida, S., Shibata, M., Yamamoto, S., Hagihara, M., Asai, N., Takahashi, M. et al. (2000). Homo-oligomer formation by basigin, an immunoglobulin superfamily member, via its N-terminal immunoglobulin domain. Eur. J. Biochem. 267, 4372-4380.

16. Melchior, A., Denys, A., Deligny, A., Mazurier, J. & Allain, F. (2008). Cyclophilin B induces integrinmediated cell adhesion by a mechanism involving CD98-dependent activation of protein kinase C-delta and p44/42 mitogen-activated protein kinases. Exp. Cell Res. 314, 616-628.

17. Pakula, R., Melchior, A., Denys, A., Vanpouille, C., Mazurier, J. & Allain, F. (2007). Syndecan-1/CD147 association is essential for cyclophilin B-induced activation of p44/42 mitogen-activated protein kinases and promotion of cell adhesion and chemotaxis. Glycobiology, 17, 492-503.

18. Yurchenko, V., Constant, S. & Bukrinsky, M. (2006). Dealing with the family: CD147 interactions with cyclophilins. Immunology, 117, 301-309.

19. Andreotti, A. H. (2003). Native state proline isomerization: an intrinsic molecular switch. Biochemistry, 42, 9515-9524.

20. Yao, Q. Z., Li, M., Yang, H., Chai, H., Fisher, W. & Chen, C. Y. (2005). Roles of cyclophilins in cancers and other organ systems. World J. Surg. 29, 276-280.

21. Pap, T. (2005). Cyclophilins in rheumatoid arthritis-stepping into an undiscovered country? Clin. Immunol. 116, 199-201.

22. Billich, A., Winkler, G., Aschauer, H., Rot, A. & Peichl, P. (1997). Presence of cyclophilin A in synovial fluids of patients with rheumatoid arthritis. J. Exp. Med. 185, 975-980.

23. Yurchenko, V., O'Connor, M., Dai, W. W., Guo, H. M., Toole, B., Sherry, B. & Bukrinsky,M. (2001). CD147 is a signaling receptor for cyclophilin B. Biochem. Biophys. Res. Commun. 288, 786-788.

24. Yurchenko, V., Zybarth, G., O'Connor, M., Dai, W. W., Franchin, G., Hao, T. et al. (2002). Active site residues of cyclophilin A are crucial for its signaling activity via CD147. J. Biol. Chem. 277, 22959-22965.

25. Jin, Z. G., Lungu, A. O., Xie, L.,Wang, M.,Wong, C. & Berk, B. C. (2004). Cyclophilin A is a proinflammatory cytokine that activates endothelial cells. Arterioscler. Thromb. Vasc. Biol. 24, 1186-1191.

26. Arora, K., Gwinn, W. M., Bower, M. A., Watson, A., Okwumabua, I., MacDonald, H. R. et al. (2005). Extracellular cyclophilins contribute to the regulation of inflammatory responses. J. Immunol. 175, 517-522.

27. Gwinn, W. M., Damsker, J. M., Falahati, R., Okwumabua, I., Kelly-Welch, A., Keegan, A. D. et al. (2006). Novel approach to inhibit asthma-mediated lung inflammation using anti-CD147 intervention. J. Immunol. 177, 4870-4879.

28. Damsker, J. M., Okwumabua, I., Bukrinsky, M. I. & Constant, S. L. (2006). Contribution of cyclophilin-CD147 interactions in rheumatoid arthritis. J. Immunol. 176, S47.

29. Bose, S., Mathur, M., Bates, P., Joshi, N. & Banerjee, A. K. (2003). Requirement for cyclophilin A for the replication of vesicular stomatitis virus New Jersey serotype. J. Gen. Virol. 84, 1687-1699.

30. Castro, A. P. V., Carvalho, T. M. U., Moussatche, N. & Damaso, C. R. A. (2003). Redistribution of cyclophilin A to viral factories during vaccinia virus infection and its incorporation into mature particles. J. Virol. 77, 9052-9068.

31. Sorin, M. & Kalpana, G. V. (2006). Dynamics of virus-host interplay in HIV-1 replication. Curr. HIV Res. 4, 117-130.

32. Chen, Z. N., Mi, L., Xu, J., Yu, J. Y.,Wang, X. H., Jiang, J. L. et al. (2005). Function of HAb18G/CD147 in invasion of host cells by severe acute respiratory syndrome coronavirus. J. Infect. Dis. 191, 755-760.

33. Pushkarsky, T., Zybarth, G., Dubrovsky, L., Yurchenko, V., Tang, H., Guo, H. M. et al. (2001). CD147 facilitates HIV-1 infection by interacting with virus-associated cyclophilin A. Proc. Natl Acad. Sci. USA, 98, 6360-6365.

34. Yu, X. L., Hu, T., Du, J. M., Ding, J. P., Yang, X. M., Zhang, J. et al. (2008). Crystal structure of HAb18g/CD147-implications for immunoglobulin superfamily homophilic adhesion. J. Biol. Chem. 283, 18056-18065.

35. Yang, H., Chen, J., Yang, J., Qiao, S., Zhao, S. & Yu, L. (2007). Cyclophilin A is upregulated in small cell lung cancer and activates ERK1/2 signal. Biochem. Biophys. Res. Commun. 361, 763-767.

36. Bosco, D. A., Eisenmesser, E. Z., Pochapsky, S., Sundquist, W. I. & Kern, D. (2002). Catalysis of cis/trans isomerization in native HIV-1 capsid by human cyclophilin A. Proc. Natl Acad. Sci. USA, 99, 5247-5252.

37. Campos-Olivas, R. & Summers, M. F. (1999). Backbone dynamics of the N-terminal domain of the HIV-1 capsid protein and comparison with the G94D mutant conferring cyclosporin resistance/dependence. Biochemistry, 38, 10262-10271.

38. Sun, J. X.&Hemler,M. E. (2001). Regulation of MMP-1 and MMP-2 production through CD147/extracellular matrix metalloproteinase inducer interactions. Cancer Res. 61,2276-2281.

39. Jia, L., Wang, H. J., Zhou, H. M., Cao, J., Hu, Y. C. & Zhang, J. N. (2006). Caveolin-1 up-regulates CD 147 glycosylation and the invasive capability of murine hepatocarcinoma cell lines. Int. J. Biochem. Cell Biol. 38, 1584-1593.

40. Jia, L., Zhou, H. M., Wang, S. J., Cao, J., Wei, W. & Zhang, J. N. (2006). Deglycosylation of CD147 downregulates matrix metalloproteinase-11 expression and the adhesive capability of murine hepatocarcinoma cell HcaF in vitro. IUBMB Life, 58, 209-216.

41. Markovic, I., Stantchev, T. S., Fields, K. H., Tiffany, L. J., Tomic, M., Weiss, C. D. et al. (2004). Thiol/disulfide exchange is a prerequisite for CXCR4-tropic HIV-1 envelope-mediated T-cell fusion during viral entry. Blood, 103, 1586-1594.

42. Iacono, K. T., Brown, A. L., Greene, M. I. & Saouaf, S. J. (2007). CD147 immunoglobulin superfamily receptor function and role in pathology. Exp. Mol. Pathol. 83, 283-295.

43. Cornilescu, G., Delaglio, F. & Bax, A. (1999). Protein backbone angle restraints from searching a database for chemical shift and sequence homology. J. Biomol. NMR, 13, 289-302.

44. Larsson, G., Martinez, G., Schleucher, J. & Wijmenga, S. S. (2003). Detection of nano-second internal motion and determination of the overall tumbling times independent of the time scale of internal motion in proteins from NMR relaxation data. J. Biomol. NMR, 27, 291-312.

45. Cole, R. & Loria, J. P. (2003). FAST-Modelfree: a program for rapid automated analysis of solution NMR spin-relaxation data. J. Biomol.NMR, 26, 203-213.

46. Yurchenko, V., Pushkarsky, T., Li, J. H., Dai, W. W., Sherry, B. & Bukrinsky, M. (2005). Regulation of CD147 cell surface expression-involvement of the proline residue in the CD147 transmembrane domain. J. Biol. Chem. 280, 17013-17019.

47. Hanoulle, X., Melchior, A., Sibille, N., Parent, B., Denys, A., Wieruszeski, J. M. et al. (2007). Structural and functional characterization of the interaction between cyclophilin B and a heparin-derived oligosaccharide. J. Biol. Chem. 282, 34148-34158.

48. Ottiger, M., Zerbe, O., Guntert, P. & Wuthrich, K. (1997). The NMR solution conformation of unligated human cyclophilin A. J. Mol. Biol. 272, 64-81.

49. Eisenmesser, E. Z., Bosco, D. A., Akke, M. & Kern, D. (2002). Enzyme dynamics during catalysis. Science, 295, 1520-1523.

50. Piotukh, K., Gu,W., Kofler, M., Labudde, D., Helms, V. & Freund, C. (2005). Cyclophilin a binds to linear peptide motifs containing a consensus that is present in many human proteins. J. Biol. Chem. 280, 23668-23674.

51. Brazin, K. N., Mallis, R. J., Fulton, D. B. & Andreotti, A. H. (2002). Regulation of the tyrosine kinase Itk by the peptidyl-prolyl isomerase cyclophilin A. Proc. Natl Acad. Sci. USA, 99, 1899-1904.

52. Grathwohl, C. & Wuthrich, K. (1981). NMR studies of the rates of proline cis-trans isomerization in oligopeptides. Biopolymers, 20, 2623-2633.

53. Liu, J., Chen, C. M. & Walsh, C. T. (1991). Human and Escherichia coli cyclophilins-sensitivity to inhibition by the immunosuppressant cyclosporin A correlates with a specific tryptophan residue. Biochemistry, 30, 2306-2310.

54. Hanna, S. M., Kirk, P., Holt, O. J., Puklavec, M. J., Brown, M. H. & Barclay, A. N. (2003). A novel form of the membrane protein CD147 that contains an extra Ig-like domain and interacts homophilically. BMC Biochem. 4, 17.

55. Kern, D., Kern, G., Scherer, G., Fischer, G. & Drakenberg, T. (1995). Kinetic analysis of cyclophilincatalyzed prolyl cis/trans isomerization by dynamic NMR spectroscopy. Biochemistry, 34, 13594-13602.

56. Yap, A. S., Crampton, M. S. & Hardin, J. (2007). Making and breaking contacts: the cellular biology of cadherin regulation. Curr. Opin. Cell Biol. 19, 508-514.

57. Guerrini, M., Hricovini, M. & Torri, G. (2007). Interaction of heparins with fibroblast growth factors: conformational aspects. Curr. Pharm. Des. 13, 2045-2056.

58. Wilson, M. C., Meredith, D., Fox, J. E. M., Manoharan, C., Davies, A. J. & Halestrap, A. P. (2005). Basigin (CD147) is the target for organomercurial inhibition of monocarboxylate transporter isoforms 1 and 4-the ancillary protein for the insensitive MCT2 is embigin (gp70). J. Biol. Chem. 280, 27213-27221.

59. Barclay, A. N. & Brown, M. H. (2006). The SIRP family of receptors and immune regulation. Nat. Rev. Immunol. 6, 457-464.

60. Lands, W. E. M. (1998). A review of alcohol clearance in humans. Alcohol, 15, 147-160.

61. Ke, H., Mayrose, D. & Cao, W. (1993). Crystal structure of cyclophilin A complexed with substrate Ala-Pro suggests a solvent-assisted mechanism of cis-trans isomerization. Proc. Natl Acad. Sci. USA, 90, 3324-3328.

62. Zhao, Y. & Ke, H. (1996). Crystal structure implies that cyclophilin predominantly catalyzes the trans to cis isomerization. Biochemistry, 35, 7356-7361.

63. Eisenmesser, E. Z., Millet, O., Labeikovsky, W., Korzhnev, D. M., Wolf-Watz, M., Bosco, D. A. et al. (2005). Intrinsic dynamics of an enzyme underlies catalysis. Nature, 438, 117-121.

64. Pushkarsky,T.,Yurchenko, V., Laborico, A. & Bukrinsky, M. (2007). CD147 stimulates HIV-1 infection in a signalindependent fashion. Biochem. Biophys. Res. Commun. 363, 495-499.

65. Delaglio, F., Grzesiek, S., Vuister, G. W., Zhu, G., Pfeifer, J. & Bax, A. (1995). NMRPipe-a multidimensional spectral processing system based on UNIX pipes. J. Biomol. NMR, 6, 277-293.

66. Vranken,W. F., Boucher,W., Stevens, T. J., Fogh, R. H., Pajon, A., Llinas, P. et al. (2005). The CCPN data model for NMR spectroscopy: development of a software pipeline. Proteins: Struct., Funct., Bioinform. 59, 687-696.

67. Sattler, M., Schleucher, J. & Griesinger, C. (1999). Heteronuclear multidimensional NMR experiments for the structure determination of proteins in solution employing pulsed field gradients. Prog. Nucl. Magn. Reson. Spectrosc. 34, 93-158.

68. d'Auvergne, E. J. & Gooley, P. R. (2008). Optimisation of NMR dynamic models. I. Minimisation algorithms and their performance within the model-free and Brownian rotational diffusion spaces. J. Biomol. NMR, 40, 107-119.

69. Farrow, N. A., Zhang, O. W., Formankay, J. D. & Kay, L. E. (1994). A heteronuclear correlation experiment for simultaneous determination of N-15 longitudinal decay and chemical-exchange rates of systems in slow equilibrium. J. Biomol. NMR, 4, 727-734.

70. Farrow, N. A., Zhang, O. W., Formankay, J. D. & Kay, L. E. (1995). Comparison of the backbone dynamics of a folded and an unfolded Sh3 domain existing in equilibrium in aqueous buffer. Biochemistry, 34, 868-878.

71. Sarkar, P., Reichman, C., Saleh, T., Birge, R. B. & Kalodimos, C. G. (2007). Proline cis-trans isomerization controls autoinhibition of a signaling protein. Mol. Cell, 25, 413-426.

72. Schon,M.P., and Boehncke,W.H. 2005. Psoriasis. N. Engl J Med. 352:1899-1912.

73. Stern,R.S., Nijsten,T., Feldman,S.R., Margolis,D.J., and Rolstad,T. 2004. Psoriasis is common, carries a substantial burden even when not extensive, and is associated with widespread treatment dissatisfaction. J Investig. Dermatol. Symp. Proc.136-139.

74. Alamanos,Y., Voulgari,P.V., and Drosos,A.A. 2008. Incidence and prevalence of psoriatic arthritis: A systematic review.

75. Steinhoff,M., Brzoska,T., and Luger,T.A. 2001. Keratinocytes in epidermal immune responses. 469-476.

76. Lowes,M.A., Bowcock,A.M., and Krueger,J.G. 2007. Pathogenesis and therapy of psoriasis. 866-873.

76. Barker,J.N. 1991. The pathophysiology of psoriasis. 227-230.

77. Buske-Kirschbaum,A., Kern,S., Ebrecht,M., and Hellhammer,D.H. 2007. Altered distribution of leukocyte subsets and cytokine production in response to acute psychosocial stress in patients with psoriasis vulgaris. 92-99.

78. van Beelen,A.J., Teunissen,M.B.M., Kapsenberg,M.L., and de Jong,E.C. 2007. Interleukin-17 in inflammatory skin disorders. 374-381.

79. Lowes,M.A., Kikuchi,T., Fuentes-Duculan,J., Cardinale,I., Zaba,L.C., Haider,A.S., Bowman,E.P., and Krueger,J.G. 2008. Psoriasis vulgaris lesions contain discrete populations of Th1 and Th17 T cells. 1207-1211.

80. Tesmer LA, Lundy,S., Sarkar S, and Fox DA 2008. Th17 cells in human disease. 87-113.

81. Ma,H.-L., Liang,S., Li,J., Napierata,L., Brown,T., Benoit,S., Senices,M., Gill,D., Dunussi-Joannopoulos,K., Collins,M. et al 2008. IL-22 is required for Th17 cell-mediated pathology in a mouse model of psoriasis-like skin inflammation. 597-607.

82. Nickoloff,B.J., Qin,J.Z., and Nestle,F.O. 2007. Immunopathogenesis of psoriasis. 45-56.

83. Toichi,E., Torres,G., McCormick,T.S., Chang,T., Mascelli,M.A., Kauffman,C.L., Aria,N., Gottlieb,A.B., Everitt,D.E., Frederick,B. et al 2006. An anti-IL-12p40 antibody down-regulates type I cytokines, chemokines, and IL-12/IL-23 in psoriasis. 4917-4926.

84. Leonardi CL, Kimball AB, Papp KA, Yeilding N, Guzzo C, Wang Y, Li S, Dooley LT, Gordon KB, and PHOENIX 1 study investigators 2008. Efficacy and safety of ustekinumab, a human interleukin-12/23 monoclonal antibody, in patients with psoriasis: 76-week results from a randomized, double-blind, placebo-controlled trial (PHOENIX 1). 1665-1674.

85. Papp KA, Langley RG, Lebwohl M, Krueger GG, Szapary P, Yeilding N, Guzzo C, Hsu MC, Wang Y, Li S et al 2008. Efficacy and safety of usetekinumab, a human interleukin-12/23 monoclonal antibody, in patients with psoriasis: 52-week results from a randomized, double-blind, placebo-controlled trial (PHOENIX 2). 1639-1684.

86. Dinarello,C.A. 2007. IL-32, a novel cytokine with a possible role in disease. iii61-iii64.

87. Kim,S.H., Han,S.Y., Azam,T., Yoon,D.Y., and Dinarello,C.A. 2005. Interleukin-32: a cytokine and inducer pf TNF alpha. 131-142.

88. Shioya,M., Nishida,A., Yagi,Y., Ogawa,A., Tsujikawa,T., Kim-Mitsuyama,S., Takayanagi,A., Shimizu,N., Fujiyama,Y., and Andoh,A. 2007. Epithelial overexpression of interleukin-32 alpha in inflammatory bowel disease. 480-486.

89. Joosten,L.A., Netea,M.G., Kim,S.H., Yoon,D.Y., Oppers-Walgreen,B., Radstake,T.R., Barrera,P., van de Loo,A.A., Dinarello,C.A., and van den Berg,W.B. 2006. IL-32, a proinflammatory cytokine in rheumatoid arthritis. 3298-3303.

90. Kim,K.H., Shim,J.H., Seo,E.H., Cho,M.C., Kang,J.W., Kim,S.H., Yu,D.Y., Song,E.Y., Lee,H.G., Sohn,J.H. et al 2008. Interleukin-32 monoclonal antibodies for immunohistochemistry, western blotting, and ELISA. 38-50.

91. Netea M.G., Azam T., Ferwerda G, Girardin SE, Walsh M, Park J-S, Abraham E, Kim J-M, Yoon D-Y, Dinarello CA et al 2005. IL-32 synergizes with nucleotide oligomerization domain (NOD) 1 and NOD2 ligands for IL-1b and IL-6 production through a caspase 1-dependent mechanism. 16309-16314.

92. Edwards III,C.K., Volk,H., Schiff,M., Schiff,M.H., Kotzin,B., Mitsuya,H., Kawaguchi,T., Sakata,K.M., Cheronis,J., Trollinger,D. et al 2008. Anti-tumor necrosis factor-a therapy in rheumatoid arthritis patients reduces whole blood gene expression compared to DMARD therapy alone.

93. Rasool ST, Tang H, Wu J, Li Wei, Mukhtar MM, Zhang J, Mu Y, Xing HX, Wu J, and Zhu Y 2008. Increased level of IL-32 during human immunodeficiency virus infection suppresses HIV replication. 161-167.

94. Ko,NY., Chang,SH., Lee,J., Kim,N., Kim,Y., Choi,W., Choi,J., Bae,S., Hong,J., Jaekal,J. et al 2008. Unique expression of a small IL-32 protein in the Jurkat leukemic T cell line. 121-127.

95. Edwards III,C.K., Bendele,A.M., Reznikov,L., Fantuzzi,G., Chlipala,E.S., Li L, Moldawer,L.L., Mountz,J.D., Li,Y., and Dinarello CA 2006. Soluble human p55 and p75 tumor necrosis factor receptors reverse spontaneous arthritis in transgenic mice expressing transmembrane tumor necrosis factor a. 2872-2885.

96. Bendele,A.M., Chlipala,E.S., Scherrer,J., Frazier,J., Sennello,G., Rich,W.J., and Edwards,I.C. 2000. Combination benefit of treatment with the cytokine inhibitors interleukin-1 receptor antagonist and PEGylated soluble tumor necrosis factor receptor type I in animal models of rheumatoid arthritis. Arthritis-Rheum. 43:2648-2659.

97. Hyka,N., Dayer,J.M., Modoux,C., Kohno,T., Edwards-III,C.K., Roux,L.P., and Burger, D. 2001. Apolipoprotein A-I inhibits the production of interleukin-1 beta and tumor necrosis factor-alpha by blocking contact-mediated activation of monocytes by T lymphocytes. Blood 97:2381-2389.

98. Li L, Callaway,E., Liu,B., Fitzpatrick,D., Hu,L.-J., Dayer,J.-M., Norris,D.A., and Edwards III,C.K. 2008. Dissociation of human T-cell mediated macrophage activation to produce proinflammatory cytokines by CD40 ligand (CD154), IFN-g, or novel T-cell cytokine inducing surface molecules (TCISMs), in a cell-cell contact manner.

99. Kupper,T.S., and Fuhlbrigge,R.C. 2004. Immune surveillance in the skin: mechanisms and clinical consequences. Nat. Rev. Immunol. 4:211-222.

100. Conrad,C., Boyman,O., Tonel,G., Tun-Kyi,A., Laggner,U., de Fougerrolles,A., Kotelianski,V., Gardner,H., and Nestle,F.O. 2007. a1b1 integrin is crucial for accumulation of epidermal T cells and the development of psoriasis. 836-842.

101. Ellis,C.N., and Krueger,G.G. 2001. Treatment of chronic plaque psoriasis by selective targeting of memory effector T lymphocytes. N. Engl J Med. 345:248-255.

102. Takada,K., Danning,C.L., Kuroiwa,T., Schlimgen,R., Tassiulas,I.O., Davis,J.C., Jr., Yarboro,C.H., Fleisher,T.A., Boumpas,D.T., and Illei,G.G. 2003. Lymphocyte depletion with fludarabine in patients with psoriatic arthritis: clinical and immunological effects. 1112-1115.

103. Rottman,J.B., Smith,T.L., Ganley,K.G., Kikuchi,T., and Krueger,J.G. 2001. Potential role of the chemokine receptors CXCR3, CCR4, and the integrin alpha-e b-7in the pathogenesis of psoriasis vulgaris. 335-337.

104. Teraki,Y., Miyake,A., Taklebayashi,R., and Shiohara,T. 2004. Homing receptor and chemokine receptor on intraepidermal T cells in psoriasis vulgaris. 658-663.

105. Kupper,T.S. 2003. Immunologic targets in psoriasis. N. Engl J Med. 349:1987-1990.

106. Berth-Jones,J. 2005. The use of cyclosporin in psoriasis. 257-260.

107. Krueger,J.G., Wolfe,J.T., Nabeya,R.T., Vallat,V.P., Gilleaudeau,P., Heftler,N.S., Austin,L.M., and Gottlieb,A.B. 1995. Successful ultraviolet B treatment of psoriasis is accompanied by a reversal of keratinocyte pathology and by selective depletion of intraepidermal T cells. 2057-2068.

108. Gottlieb,S.L., Gilleaudeau,P., Johnson,R., Estes,L., Woodworth,T.G., Gottlieb,A.B., and Krueger,J.G. 1995. Response of psoriasis to a lymphocyte-selective toxin (DAB389IL-2) suggests a primary immune, but not keratinocyte, pathogenic basis. Nat. Med. 1:442-447.

109. Abrams,J.R., Kelley,S.L., Hayes,E., Kikuchi,T., Brown,M.J., Kang,S., Lebwohl,M.G., Guzzo,C.A., Jegasothy,B.V., Linsley,P.S. et al 2000. Blockade of T lymphocyte costimulation with cytotoxic T lymphocyte-associated antigen 4-immunoglobulin (CTLA4Ig) reverses the cellular pathology of psoriatic plaques, including the activation of keratinocytes, dendritic cells, and endothelial cells. J Exp. Med. 192:681-694.

110. Gordon,K.B., Papp,K.A., Hamilton,T.K., Walicke,P.A., Dummer,W., Li,N., Bresnahna,B.W., and Menter,A. 2003. Efalizumab for patients with moderate to severe plaque psoriasis: a randomized controlled trial. 3073-3080.

111. Nickoloff,B.J., Wrone-Smith,T., Bonish,B.K., and Porcelli,S.A. 1999. Response of murine and normal human skin to injection of allogeneic blood-derived psoriatic immunocytes. 546-552.

112. Nickoloff,B.J., and Wrone-Smith,T. 1999. Injection of pre-psoriatic skin with CD4+ T cells induces psoriasis. 145-158.

113. Prinz,J.C., Grob,B., and et al. 1994. T cell clones from psoriasis skin lesions can promote keratinocyte proliferation in vitro via secreted products. 593-598.

114. Vollmer,S., Menssen,A., Trommler,P., Schendel,D., and Prinz,J.C. 1994. T-lymphocytes derived from skin lesiona of patients with psoriasis vulgaris express a novel cytokine pattern that is distinct from that of T helper type I and T helper type 2 cells. 2377-2382.

115. Bata-Csorgo,Z., Hammerberg,C., Voorhees,J.J., and Cooper,K.D. 1995. Kinetics and regulation of human keratinocyte stem cell growth factors in short-term primary ex vivo culture. Cooperative growth factors from psoriatic lesional T lymphocytes stimulate proliferation among psoriatic uninvolved, but not normal stem keratinocytes. 317-327. 116. Lebwohl,M. 2003. Psoriasis. The Lancet 361:1197-1204.

117. Baier,G., Asadullah,K.A., and Zugel,U. 2006. The immunological synapse: Kinases in T cell signalling as potential drug targets. 3-5.

118. Ouyang,W., Kolls,J.K., and Zheng,Y. 2008. The biological functions of T helper 17 cell effector cytokines in inflammation. 454-467.

119. Dong,C. 2008. Th17 cells in development: an updated view of their molecular identity and genetic programming. 337-348.

120. McGeachy,M.J., Bak-Jensen,K.S., Chen,Y., Tato,C.M., Blumenschein,W., McClanahan,T., and Cua,D.J. 2007. TGF-b and IL-6 drive the production of IL-17 and IL-10 by T cells and restrain Th17 cell-mediated pathology. 1390-1397.

121. Chen,Z., and O'Shea,J.J. 2008. Regulation of IL-17 production in human lymphocytes. 71-78.

122. Chen,Z., Tato,C.M., Muul,L., Laurence,A., and O'Shea,J.J. 2007. Distinct regualtion of interleukin-17 in human T helper lymphocytes. 2936-2946.

123. Netea,M.G., Lewis,M., Azam,T., Joosten,L.A., Jackal,J., Bae,S.Y., Dinarello CA, and Kim,S.-H. 2008. Interleukin-32 induces the differentiation of monocytes into macrophage-like cells. 3515-3520.

124. Frishman,J.I., Edwards III,C.K., Sonnenberg,M.G., Kohno,T., Cohen,A.M., and Dinarello,C.A. 2000. Tumor necrosis factor (TNF)-alpha-induced interleukin-8 in human blood cultures discriminates neutralization by the p55 and p75 TNF soluble receptors. J-Infect-Dis. 182:1722-1730.

125. Mugler,K.C., Singh,M., Tringler,B., Torkko,K.C., Liu,W., Papkoff,J., and Shroyer,K.R. 2007. B7-h4 expression in a range of breast pathology: correlation with tumor T-cell infiltration. 363-370.

126. Swick;B.L., Ravdel,L., Fitzpatrick,J.E., and Robinson,W.A. 2008. Platelet-derived growth factor receptor alpha mutational status and immunohistochemical expression in Merkel cell carcinoma: implications for treatment with imatinib mesylate. 197-202.

127. Herrmann et al., 2008, Construction of optimized bispecific antibodies for selective activation of the death receptor CD95. Cancer Research. 68(4):1221-128. Manikandan J, Pushparaj PN, Melendez AJ. 2007, Protein i: interference at protein level by intrabodies. Front Biosci. 1;12:1344-52.

**Claims**

1. A composition that simultaneously inhibits activity and/or expression of FKBP52 and CD147 for use in a method for inhibiting pro-inflammatory cytokine production in a human having a chronic inflammatory disease, said method comprising administering an effective amount of a said composition, wherein the composition comprises

   (i) one or more siRNA molecules that silence expression of FKBP52 and CD147, or
   (ii) one or more antibodies or functional fragments thereof that inhibit the activity of FKBP52 and CD147.

2. The composition for the use according to claim 1, wherein the composition comprises a bispecific antibody that inhibits the activity of FKBP52 and CD147.

3. The composition for the use according to claim 1 or 2, wherein the composition inhibits production of TNF$\alpha$, IL-1, IL-32y or a combination thereof.

4. A composition that simultaneously inhibits activity and/or expression of FKBP52 and CD147 for use in a method for selectively inhibiting an adaptive immune response without significantly affecting an innate immunity response in a subject having a chronic inflammatory disease, wherein the composition comprises

   (i) one or more siRNA molecules that silence expression of FKBP52 and CD147, or
   (ii) one or more antibodies or functional fragments thereof that inhibit the activity of FKBP52 and CD147.

5. A pharmaceutical composition that simultaneously inhibits activity and/or expression of FKBP52 and CD147 for use in a method of treating a chronic inflammatory disease, wherein the pharmaceutical composition comprises:

   both a CD147 inhibitor and an FKBP52 inhibitor, wherein the FKBP52 and CD147 inhibitors comprise (i) one or more siRNA molecules to silence FKBP52 and CD147 expression, or (ii) one or more antibodies or functional fragments thereof that block FKBP52 and CD147 activity,
   and
   a pharmaceutical acceptable carrier.

6. The pharmaceutical composition for the use according to claim 5, wherein the chronic inflammatory disease is a psoriatic or autoimmune disease.

7. The pharmaceutical composition for the use according to claim 6, wherein the disease is psoriasis.

**8.** The pharmaceutical composition for the use according to claims 4 or 5, wherein the FKBP52 and CD147 inhibitors comprise a bispecific antibody that blocks FKBP52 and CD147 activity.

**Patentansprüche**

**1.** Zusammensetzung, die gleichzeitig Aktivität und/oder Expression von FKBP52 und CD 147 inhibiert, für die Verwendung in einem Verfahren für die Inhibierung der Produktion von proinflammatorischen Zytokinen bei einem Menschen, der eine chronische entzündliche Erkrankung hat, wobei das Verfahren die Verabreichung einer wirksamen Menge der Zusammensetzung umfasst, wobei die Zusammensetzung Folgendes umfasst:

(i) ein oder mehrere siRNA-Moleküle, die die Expression von FKBP52 und CD 147 abschalten, oder
(ii) einen oder mehrere Antikörper oder funktionelle Fragmente davon, die die Aktivität von FKBP52 und CD147 inhibieren.

**2.** Zusammensetzung für die Verwendung nach Anspruch 1, wobei die Zusammensetzung einen bispezifischen Antikörper umfasst, der die Aktivität von FKBP52 und CD147 inhibiert.

**3.** Zusammensetzung für die Verwendung nach Anspruch 1 oder 2, wobei die Zusammensetzung die Produktion von TNF$\alpha$, IL-1, IL-32$\gamma$ oder einer Kombination davon inhibiert.

**4.** Zusammensetzung, die gleichzeitig Aktivität und/oder Expression von FKBP52 und CD 147 inhibiert, für die Verwendung in einem Verfahren für die selektive Inhibierung einer adaptiven Immunantwort ohne wesentliche Beeinflussung einer angeborenen Immunantwort bei einem Subjekt, das eine chronische entzündliche Erkrankung hat, wobei die Zusammensetzung Folgendes umfasst:

(i) ein oder mehrere siRNA-Moleküle, die die Expression von FKBP52 und CD 147 abschalten, oder
(ii) einen oder mehrere Antikörper oder funktionelle Fragmente davon, die die Aktivität von FKBP52 und CD147 inhibieren.

**5.** Pharmazeutische Zusammensetzung, die gleichzeitig Aktivität und/oder Expression von FKBP52 und CD147 inhibiert, für die Verwendung in einem Verfahren zur Behandlung einer chronischen entzündlichen Erkrankung, wobei die pharmazeutische Zusammensetzung Folgendes umfasst:

sowohl einen CD147-Inhibitor als auch einen FKBP52-Inhibitor, wobei die FKBP52- und CD147-Inhibitoren Folgendes umfassen: (i) ein oder mehrere siRNA-Moleküle, um die FKBP52- und CD147-Expression abzuschalten, oder (ii) einen oder mehrere Antikörper oder funktionelle Fragmente davon, die die FKBP52- und CD147-Aktivität blockieren,
und
einen pharmazeutisch verträglichen Träger.

**6.** Pharmazeutische Zusammensetzung für die Verwendung nach Anspruch 5, wobei die chronische entzündliche Erkrankung eine psoriatische oder Autoimmunerkrankung ist.

**7.** Pharmazeutische Zusammensetzung für die Verwendung nach Anspruch 6, wobei die Erkrankung Psoriasis ist.

**8.** Pharmazeutische Zusammensetzung für die Verwendung nach den Ansprüchen 4 oder 5, wobei die FKBP52- und CD147-Inhibitoren einen bispezifischen Antikörper umfassen, der FKBP52- und CD147-Aktivität blockiert.

**Revendications**

**1.** Composition qui inhibe simultanément l'activité et/ou l'expression de FKBP52 et CD 147, pour une utilisation dans une méthode d'inhibition de la production de cytokines pro-inflammatoires chez un être humain souffrant d'une maladie inflammatoire chronique, ladite méthode comprenant l'administration d'une quantité efficace de ladite composition, où la composition comprend

(i) une ou plusieurs molécules d'ARNsi qui mettent sous silence l'expression de FKBP52 et CD 147, ou

(ii) un ou plusieurs anticorps ou fragments fonctionnels de ceux-ci qui inhibent l'activité de FKBP52 et CD147.

2. Composition pour une utilisation selon la revendication 1, où la composition comprend un anticorps bispécifique qui inhibe l'activité de FKBP52 et CD147.

3. Composition pour une utilisation selon la revendication 1 ou 2, où la composition inhibe la production de TNF$\alpha$, IL-1, IL-32$\gamma$ ou une combinaison de ceux-ci.

4. Composition qui inhibe simultanément l'activité et/ou l'expression de FKBP52 et CD147, pour une utilisation dans une méthode d'inhibition sélective d'une réponse immunitaire adaptative sans affecter de manière significative une réponse immunitaire innée chez un sujet souffrant d'une maladie inflammatoire chronique, où la composition comprend

(i) une ou plusieurs molécules d'ARNsi qui mettent sous silence l'expression de FKBP52 et CD 147, ou
(ii) un ou plusieurs anticorps ou fragments fonctionnels de ceux-ci qui inhibent l'activité de FKBP52 et CD147.

5. Composition pharmaceutique qui inhibe simultanément l'activité et/ou l'expression de FKBP52 et CD147, pour une utilisation dans une méthode de traitement d'une maladie inflammatoire chronique, où la composition pharmaceutique comprend un inhibiteur de CD147 ainsi qu'un inhibiteur de FKBP52, où les inhibiteurs de FKBP52 et CD147 comprennent (i) une ou plusieurs molécules d'ARNsi pour mettre sous silence l'expression de FKBP52 et CD147, ou (ii) un ou plusieurs anticorps ou fragments fonctionnels de ceux-ci qui inhibent l'activité de FKBP52 et CD147, et un véhicule pharmaceutiquement acceptable.

6. Composition pharmaceutique pour une utilisation selon la revendication 5, où la maladie inflammatoire chronique est une maladie psoriasique ou auto-immune.

7. Composition pharmaceutique pour une utilisation selon la revendication 6, où la maladie est le psoriasis.

8. Composition pharmaceutique pour une utilisation selon les revendications 4 ou 5, où les inhibiteurs de FKBP52 et CD147 comprennent un inhibiteur bispécifique qui bloque l'activité de FKBP52 et CD147.

**Figure 1**

**Figure 2**

**Figure 3**

**A.** **FKBP52 Transmembrane site**

1. mAb here

2. Intrabody
3. siRNA
4. Small Molecule

**B.** **Full-Length Cellular EMMPRIN**

Pro211

Pro180

mAb or BiAb Binding

*to Block*
*Cyclophilin A/B*
*(or FKBP's)*

**C.** **Activated Cleared EMMPRIN = Soluble EMMPRIN Ligand**

Pro211

Pro180

mAb or BiAb Binding

**D.** **Soluble EMMPRIN Receptor**

microvesicle    Pro211

Pro180

*mAb or BiAb*
*to Block*
*Cyclophilin A/B*
*(or FKBP's)*

ptor

# Figure 4

Figure 5

**Figure 6**

**Figure 7**

Figure 8

Figure 9

**Figure 10**

Figure 11

**Figure 12**

**Figure 13**

**Figure 14**

L Q|A|F\S\A|A|E|I|G|S|C|N K

**Figure 15**

**Figure 16**

**Figure 17**

**Figure 18**

**Figure 19**

CD147 Cell Surface Expression

**Figure 20**

Figure 21

Figure 22

Figure 23

Figure 24

Figure 25

Figure 26

Figure 27

Figure 28

Figure 29

Figure 30

Dual Inhibition of Psoriasis Th22 T cell TCISM#1 and TCISM#2 Targets Using Specific mAb's: TNF Production

**Figure 31**

Figure 32

**Figure 33**

**Figure 34**

**Figure 35**

Figure 36

Figure 37

A. Untreated Human Primary Skin Keratinocytes (Hka)
B. Hka Stimulated with IL-1β (1 ng/ml)
C. Hka stimulated with TNFα (10 ng/ml)
D. Stimulated with IL-32 (10 pg/ml)

Figure 38

Figure 39

Figure 40

Figure 41

Figure 42

Figure 43

Figure 44

Figure 45

Figure 46

Figure 47

Figure 48

Figure 49

Figure 50

Figure 51

Figure 52

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008065992 W

### Non-patent literature cited in the description

- **DAMSKER et al.** *Immunology,* 2009, vol. 126 (1), 55-62
- **GWINN et al.** *The Journal of Immunology,* 2006, vol. 177 (7), 4870-4879
- **JONSCHER et al.** *The Journal of Investigative Dermatology,* 2009, vol. 129 (1), S117
- **MARTIN E W.** Remington's Pharmaceutical Sciences. Mack Publishing Company, 1995
- **GUO, H. M. ; MAJMUDAR, G. ; JENSEN, T. C. ; BISWAS, C. ; TOOLE, B. P. ; GORDON, M. K.** Characterization of the gene for human EMMPRIN, a tumor cell surface inducer of matrix metalloproteinases. *Gene,* 1998, vol. 220, 99-108
- **SCHMIDT, R. ; BÜLTMANN, A. ; FISCHEL, S. ; GIL-LITZER, A. ; CULLEN, P. ; WALCH, A. et al.** Extracellular matrix metalloproteinase inducer (CD147) is a novel receptor on platelets, activates platelets, and augments nuclear factor {kappa}B dependent inflammation inmonocytes. *Circ. Res.,* 2008, vol. 107
- **RUIZ, S. ; CASTRO-CASTRO, A. ; BUSTELO, X. R.** CD147 inhibits the nuclear factor of activated T-cells by impairing vav1 and rac1 downstream signaling. *J. Biol. Chem.,* 2008, vol. 283, 5554-5566
- **GABISON, E. E. ; HOANG-XUAN, T. ; MAUVIEL, A. ; MENASHI, S.** MMPRIN/CD147, an MMP modulator in cancer, development and tissue repair. *Biochimie,* 2005, vol. 87, 361-368
- **ZHENG, H. C. ; TAKAHASHI, H. ; MURAI, Y. ; CUI, Z. G. ; NOMOTO, K. ; MIWA, S. et al.** Upregulated EMMPRIN/CD147 might contribute to growth and angiogenesis of gastric carcinoma: a good marker for local invasion and prognosis. *Br. J. Cancer,* 2006, vol. 95, 1371-1378
- **TANG, Y. ; NAKADA, M. T. ; KESAVAN, P. ; MC-CABE, F. ; MILLAR, H. ; RAFFERTY, P. et al.** Extracellular matrix metalloproteinase inducer stimulates tumor angiogenesis by elevating vascular endothelial cell growth factor and matrix metal loproteinases. *Cancer Res.,* 2005, vol. 65, 3193-3199
- **VELEZ, W. ; KYPRIANOU, N.** EMMPRIN is a biomarker of prostate cancer progression in TRAMP mice. *FASEB J.,* 2007, vol. 21, A621

- **XU, H. Y. ; QIAN, A. R. ; SHANG, P. ; XU, J. ; KONG, L. M. ; BIAN, H. J. ; CHEN, Z. N.** siRNA targeted against HAb18G/CD147 inhibits MMP-2 secretion, actin and FAK expression in hepatocellular carcinoma cell line via ERK1/2 pathway. *Cancer Lett.,* 2007, vol. 247, 336-344
- **MILLIMAGGI, D. ; MARI, M. ; D'ASCENZO, S. ; CA-ROSA, E. ; JANNINI, E. A. ; ZUCKER, S. et al.** Tumor vesicleassociated CD147 modulates the angiogenic capability of endothelial cells. *Neoplasia,* 2007, vol. 9, 349-357
- **SIDHU, S. S. ; MENGISTAB, A. T. ; TAUSCHER, A. N. ; LAVAIL, J. ; BASBAUM, C.** The microvesicle as a vehicle for EMMPRIN in tumor-stromal interactions. *Oncogene,* 2004, vol. 23, 956-963
- **TAYLOR, P. M. ; WOODFIELD, R. J. ; HODGKIN, M. N. ; PETTITT, T. R. ; MARTIN, A. ; KERR, D. J. ; WAKELAM, M. J. O.** Breast cancer cell-derived EMMPRIN stimulates fibroblast MMP2 release through a phospholipase A (2) and 5-lipoxygenase catalyzed pathway. *Oncogene,* 2002, vol. 21, 5765-5772
- **ZHU, P. ; DING, J. ; ZHOU, J. ; DONG, W. J. ; FAN, C. M. ; CHEN, Z. N.** Expression of CD147 on monocytes/macrophages in rheumatoid arthritis: its potential role in monocyte accumulation and matrix metalloproteinase production. *Arthritis Res. Ther.,* 2005, vol. 7, R1023-R1033
- **ZHU, P. ; LU, N. ; SHI, Z. G. ; ZHOU, J. ; WU, Z. B. ; YANG, Y. et al.** CD147 overexpression on synoviocytes in rheumatoid arthritis enhances matrix metalloproteinase production and invasiveness of synoviocytes. *Arthritis Res. Ther.,* 2006, vol. 8, 1-12
- **BELTON, R. J. ; CHEN, L. ; MESQUITA, F. S. ; NOWAK, R. A.** Basigin-2 is a cell surface receptor for soluble basigin ligand. *J. Biol. Chem.,* 2008, vol. 283, 17805-17814
- **YOSHIDA, S. ; SHIBATA, M. ; YAMAMOTO, S. ; HAGIHARA, M. ; ASAI, N. ; TAKAHASHI, M. et al.** Homo-oligomer formation by basigin, an immunoglobulin superfamily member, via its N-terminal immunoglobulin domain. *Eur. J. Biochem.,* 2000, vol. 267, 4372-4380

- **MELCHIOR, A. ; DENYS, A. ; DELIGNY, A. ; MAZURIER, J. ; ALLAIN, F.** Cyclophilin B induces integrinmediated cell adhesion by a mechanism involving CD98-dependent activation of protein kinase C-delta and p44/42 mitogen-activated protein kinases. *Exp. Cell Res,* 2008, vol. 314, 616-628
- **PAKULA, R. ; MELCHIOR, A. ; DENYS, A. ; VANPOUILLE, C. ; MAZURIER, J. ; ALLAIN, F.** Syndecan-1/CD147 association is essential for cyclophilin B-induced activation of p44/42 mitogen-activated protein kinases and promotion of cell adhesion and chemotaxis. *Glycobiology,* 2007, vol. 17, 492-503
- **YURCHENKO, V. ; CONSTANT, S. ; BUKRINSKY, M.** Dealing with the family: CD147 interactions with cyclophilins. *Immunology,* 2006, vol. 117, 301-309
- **ANDREOTTI, A. H.** Native state proline isomerization: an intrinsic molecular switch. *Biochemistry,* 2003, vol. 42, 9515-9524
- **YAO, Q. Z. ; LI, M. ; YANG, H., CHAI ; H., FISHER, W. ; CHEN, C. Y.** Roles of cyclophilins in cancers and other organ systems. *World J. Surg.,* 2005, vol. 29, 276-280
- **PAP, T.** Cyclophilins in rheumatoid arthritis-stepping into an undiscovered country?. *Clin. Immunol.,* 2005, vol. 116, 199-201
- **BILLICH, A. ; WINKLER, G. ; ASCHAUER, H. ; ROT, A. ; PEICHL, P.** Presence of cyclophilin A in synovial fluids of patients with rheumatoid arthritis. *J. Exp. Med.,* 1997, vol. 185, 975-980
- **YURCHENKO, V. ; O'CONNOR, M. ; DAI, W. W. ; GUO, H. M. ; TOOLE, B. ; SHERRY, B. ; BUKRINSKY,M.** CD147 is a signaling receptor for cyclophilin B. *Biochem. Biophys. Res. Commun.,* 2001, vol. 288, 786-788
- **YURCHENKO, V. ; ZYBARTH, G. ; O'CONNOR, M. ; DAI, W. W. ; FRANCHIN, G. ; HAO, T. et al.** Active site residues of cyclophilin A are crucial for its signaling activity via CD147. *J. Biol. Chem.,* 2002, vol. 277, 22959-22965
- **JIN, Z. G. ; LUNGU, A. O. ; XIE, L. ; WANG, M. ; WONG, C. ; BERK, B. C.** Cyclophilin A is a proinflammatory cytokine that activates endothelial cells. Arterioscler. *Thromb. Vasc. Biol.,* 2004, vol. 24, 1186-1191
- **ARORA, K. ; GWINN, W. M. ; BOWER, M. A. ; WATSON, A. ; OKWUMABUA, I. ; MACDONALD, H. R. et al.** Extracellular cyclophilins contribute to the regulation of inflammatory responses. *J. Immunol.,* 2005, vol. 175, 517-522
- **GWINN, W. M. ; DAMSKER, J. M. ; FALAHATI, R. ; OKWUMABUA, I. ; KELLY-WELCH, A. ; KEEGAN, A. D. et al.** Novel approach to inhibit asthma-mediated lung inflammation using anti-CD147 intervention. *J. Immunol.,* 2006, vol. 177, 4870-4879
- **DAMSKER, J. M. ; OKWUMABUA, I. ; BUKRINSKY, M. I. ; CONSTANT, S. L.** Contribution of cyclophilin-CD147 interactions in rheumatoid arthritis. *J. Immunol.,* 2006, vol. 176, 47
- **BOSE, S. ; MATHUR, M. ; BATES, P. ; JOSHI, N. ; BANERJEE, A. K.** Requirement for cyclophilin A for the replication of vesicular stomatitis virus New Jersey serotype. *J. Gen. Virol.,* 2003, vol. 84, 1687-1699
- **CASTRO, A. P. V. ; CARVALHO, T. M. U. ; MOUSSATCHE, N. ; DAMASO, C. R. A.** Redistribution of cyclophilin A to viral factories during vaccinia virus infection and its incorporation into mature particles. *J. Virol.,* 2003, vol. 77, 9052-9068
- **SORIN, M. ; KALPANA, G. V.** Dynamics of virus-host interplay in HIV-1 replication. *Curr. HIV Res.,* 2006, vol. 4, 117-130
- **CHEN, Z. N. ; MI, L. ; XU, J. ; YU, J. Y. ; WANG, X. H. ; JIANG, J. L. et al.** Function of HAb18G/CD147 in invasion of host cells by severe acute respiratory syndrome coronavirus. *J. Infect. Dis.,* 2005, vol. 191, 755-760
- **PUSHKARSKY, T. ; ZYBARTH, G. ; DUBROVSKY, L. ; YURCHENKO, V. ; TANG, H. ; GUO, H. M. et al.** CD147 facilitates HIV-1 infection by interacting with virus-associated cyclophilin A. *Proc. Natl Acad. Sci. USA,* 2001, vol. 98, 6360-6365
- **YU ; X. L. ; HU, T. ; DU, J. M. ; DING, J. P. ; YANG, X. M. ; ZHANG, J. et al.** Crystal structure of HAb18g/CD147-implications for immunoglobulin superfamily homophilic adhesion. *J. Biol. Chem.,* 2008, vol. 283, 18056-18065
- **YANG, H. ; CHEN, J. ; YANG, J. ; QIAO, S. ; ZHAO, S. ; YU, L.** Cyclophilin A is upregulated in small cell lung cancer and activates ERK1/2 signal. *Biochem. Biophys. Res. Commun.,* 2007, vol. 361, 763-767
- **BOSCO, D. A. ; EISENMESSER, E. Z. ; POCHAPSKY, S. ; SUNDQUIST, W. I. ; KERN, D.** Catalysis of cis/trans isomerization in native HIV-1 capsid by human cyclophilin A. *Proc. Natl Acad. Sci. USA,* 2002, vol. 99, 5247-5252
- **CAMPOS-OLIVAS, R. ; SUMMERS, M. F.** Backbone dynamics of the N-terminal domain of the HIV-1 capsid protein and comparison with the G94D mutant conferring cyclosporin resistance/dependence. *Biochemistry,* 1999, vol. 38, 10262-10271
- **SUN, J. X. ; HEMLER,M. E.** Regulation of MMP-1 and MMP-2 production through CD147/extracellular matrix metalloproteinase inducer interactions. *Cancer Res.,* 2001, vol. 61, 2276-2281
- **JIA, L. ; WANG, H. J. ; ZHOU, H. M. ; CAO, J. ; HU, Y. C. ; ZHANG, J. N.** Caveolin-1 up-regulates CD 147 glycosylation and the invasive capability of murine hepatocarcinoma cell lines. *Int. J. Biochem. Cell Biol.,* 2006, vol. 38, 1584-1593

- **JIA, L. ; ZHOU, H. M. ; WANG, S. J. ; CAO, J. ; WEI, W. ; ZHANG, J. N.** Deglycosylation of CD147 downregulates matrix metalloproteinase-11 expression and the adhesive capability of murine hepatocarcinoma cell HcaF in vitro. *IUBMB Life,* 2006, vol. 58, 209-216
- **MARKOVIC, I. ; STANTCHEV, T. S. ; FIELDS, K. H. ; TIFFANY, L. J. ; TOMIC, M. ; WEISS, C. D. et al.** Thiol/disulfide exchange is a prerequisite for CXCR4-tropic HIV-1 envelope-mediated T-cell fusion during viral entry. *Blood,* 2004, vol. 103, 1586-1594
- **IACONO, K. T. ; BROWN, A. L. ; GREENE, M. I. ; SAOUAF, S. J.** CD147 immunoglobulin superfamily receptor function and role in pathology. *Exp. Mol. Pathol.,* 2007, vol. 83, 283-295
- **CORNILESCU, G. ; DELAGLIO, F. ; BAX, A.** Protein backbone angle restraints from searching a database for chemical shift and sequence homology. *J. Biomol. NMR,* 1999, vol. 13, 289-302
- **LARSSON, G. ; MARTINEZ, G. ; SCHLEUCHER, J. ; WIJMENGA, S. S.** Detection of nano-second internal motion and determination of the overall tumbling times independent of the time scale of internal motion in proteins from NMR relaxation data. *J. Biomol. NMR,* 2003, vol. 27, 291-312
- **COLE, R. ; LORIA, J. P.** FAST-Modelfree: a program for rapid automated analysis of solution NMR spin-relaxation data. *J. Biomol. NMR,* 2003, vol. 26, 203-213
- **YURCHENKO, V. ; PUSHKARSKY, T. ; LI, J. H. ; DAI, W. W. ; SHERRY, B. ; BUKRINSKY, M.** Regulation of CD147 cell surface expression-involvement of the proline residue in the CD147 transmembrane domain. *J. Biol. Chem.,* 2005, vol. 280, 17013-17019
- **HANOULLE, X. ; MELCHIOR, A. ; SIBILLE, N. ; PARENT, B. ; DENYS, A. ; WIERUSZESKI, J. M. et al.** Structural and functional characterization of the interaction between cyclophilin B and a heparin-derived oligosaccharide. *J. Biol. Chem.,* 2007, vol. 282, 34148-34158
- **OTTIGER, M. ; ZERBE, O. ; GUNTERT, P. ; WUTHRICH, K.** The NMR solution conformation of unligated human cyclophilin A. *J. Mol. Biol.,* 1997, vol. 272, 64-81
- **EISENMESSER, E. Z. ; BOSCO, D. A. ; AKKE, M. ; KERN, D.** Enzyme dynamics during catalysis. *Science,* 2002, vol. 295, 1520-1523
- **PIOTUKH, K. ; GU, W. ; KOFLER, M. ; LABUDDE, D. ; HELMS, V. ; FREUND, C.** Cyclophilin a binds to linear peptide motifs containing a consensus that is present in many human proteins. *J. Biol. Chem.,* 2005, vol. 280, 23668-23674

- **BRAZIN, K. N. ; MALLIS, R. J. ; FULTON, D. B. ; ANDREOTTI, A. H.** Regulation of the tyrosine kinase Itk by the peptidyl-prolyl isomerase cyclophilin A. *Proc. Natl Acad. Sci. USA,* 2002, vol. 99, 1899-1904
- **GRATHWOHL, C. ; WUTHRICH, K.** NMR studies of the rates of proline cis-trans isomerization in oligopeptides. *Biopolymers,* 1981, vol. 20, 2623-2633
- **LIU, J. ; CHEN, C. M. ; WALSH, C. T.** Human and Escherichia coli cyclophilins-sensitivity to inhibition by the immunosuppressant cyclosporin A correlates with a specific tryptophan residue. *Biochemistry,* 1991, vol. 30, 2306-2310
- **HANNA, S. M. ; KIRK, P. ; HOLT, O. J. ; PUKLAVEC, M. J. ; BROWN, M. H. ; BARCLAY, A. N.** A novel form of the membrane protein CD147 that contains an extra Ig-like domain and interacts homophilically. *BMC Biochem.,* 2003, vol. 4, 17
- **KERN, D. ; KERN, G. ; SCHERER, G. ; FISCHER, G. ; DRAKENBERG, T.** Kinetic analysis of cyclophilincatalyzed prolyl cis/trans isomerization by dynamic NMR spectroscopy. *Biochemistry,* 1995, vol. 34, 13594-13602
- **YAP, A. S. ; CRAMPTON, M. S. ; HARDIN, J.** Making and breaking contacts: the cellular biology of cadherin regulation. *Curr. Opin. Cell Biol.,* 2007, vol. 19, 508-514
- **GUERRINI, M. ; HRICOVINI, M. ; TORRI, G.** Interaction of heparins with fibroblast growth factors: conformational aspects. *Curr. Pharm. Des.,* 2007, vol. 13, 2045-2056
- **WILSON, M. C. ; MEREDITH, D. ; FOX, J. E. M. ; MANOHARAN, C. ; DAVIES, A. J. ; HALESTRAP, A. P.** Basigin (CD147) is the target for organomercurial inhibition of monocarboxylate transporter isoforms 1 and 4-the ancillary protein for the insensitive MCT2 is embigin (gp70). *J. Biol. Chem.,* 2005, vol. 280, 27213-27221
- **BARCLAY, A. N. ; BROWN, M. H.** The SIRP family of receptors and immune regulation. *Nat. Rev. Immunol.,* 2006, vol. 6, 457-464
- **LANDS, W. E. M.** A review of alcohol clearance in humans. *Alcohol,* 1998, vol. 15, 147-160
- **KE, H. ; MAYROSE, D. ; CAO, W.** Crystal structure of cyclophilin A complexed with substrate Ala-Pro suggests a solvent-assisted mechanism of cis-trans isomerization. *Proc. Natl Acad. Sci. USA,* 1993, vol. 90, 3324-3328
- **ZHAO, Y. ; KE, H.** Crystal structure implies that cyclophilin predominantly catalyzes the trans to cis isomerization. *Biochemistry,* 1996, vol. 35, 7356-7361

- **EISENMESSER, E. Z. ; MILLET, O. ; LABEIKO-VSKY, W. ; KORZHNEV, D. M. ; WOLF-WATZ, M. ; BOSCO, D. A. et al.** Intrinsic dynamics of an enzyme underlies catalysis. *Nature,* 2005, vol. 438, 117-121
- **PUSHKARSKY,T. ; YURCHENKO, V. ; LABORI-CO, A. ; BUKRINSKY, M.** CD147 stimulates HIV-1 infection in a signalindependent fashion. *Biochem. Biophys. Res. Commun.,* 2007, vol. 363, 495-499
- **DELAGLIO, F. ; GRZESIEK, S. ; VUISTER, G. W. ; ZHU, G. ; PFEIFER, J. ; BAX, A.** NMRPipe-a multidimensional spectral processing system based on UNIX pipes. *J. Biomol. NMR,* 1995, vol. 6, 277-293
- **VRANKEN,W. F. ; BOUCHER,W. ; STEVENS, T. J. ; FOGH, R. H. ; PAJON, A. ; LLINAS, P. et al.** The CCPN data model for NMR spectroscopy: development of a software pipeline. *Proteins: Struct., Funct., Bioinform.,* 2005, vol. 59, 687-696
- **SATTLER, M. ; SCHLEUCHER, J. ; GRIESINGER, C.** Heteronuclear multidimensional NMR experiments for the structure determination of proteins in solution employing pulsed field gradients. *Prog. Nucl. Magn. Reson. Spectrosc.,* 1999, vol. 34, 93-158
- **D'AUVERGNE, E. J. ; GOOLEY, P. R.** Optimisation of NMR dynamic models. I. Minimisation algorithms and their performance within the model-free and Brownian rotational diffusion spaces. *J. Biomol. NMR,* 2008, vol. 40, 107-119
- **FARROW, N. A. ; ZHANG, O. W. ; FORMANKAY, J. D. ; KAY, L. E.** A heteronuclear correlation experiment for simultaneous determination of N-15 longitudinal decay and chemical-exchange rates of systems in slow equilibrium. *J. Biomol. NMR,* 1994, vol. 4, 727-734
- **FARROW, N. A. ; ZHANG, O. W. ; FORMANKAY, J. D. ; KAY, L. E.** Comparison of the backbone dynamics of a folded and an unfolded Sh3 domain existing in equilibrium in aqueous buffer. *Biochemistry,* 1995, vol. 34, 868-878
- **SARKAR, P. ; REICHMAN, C. ; SALEH, T. ; BIRGE, R. B. ; KALODIMOS, C. G.** Proline cis-trans isomerization controls autoinhibition of a signaling protein. *Mol. Cell,* 2007, vol. 25, 413-426
- **SCHON,M.P. ; BOEHNCKE,W.H.** Psoriasis. *N. Engl J Med.,* 2005, vol. 352, 1899-1912
- **STERN,R.S. ; NIJSTEN,T. ; FELDMAN,S.R. ; MARGOLIS,D.J. ; ROLSTAD,T.** Psoriasis is common, carries a substantial burden even when not extensive, and is associated with widespread treatment dissatisfaction. *J Investig. Dermatol. Symp. Proc.,* 2004, 136-139
- **ALAMANOS,Y. ; VOULGARI,P.V. ; DROSOS,A.A.** *Incidence and prevalence of psoriatic arthritis: A systematic review,* 2008
- **STEINHOFF,M. ; BRZOSKA,T. ; LUGER,T.A.** *Keratinocytes in epidermal immune responses,* 2001, 469-476
- **LOWES,M.A. ; BOWCOCK,A.M. ; KRUEGER,J.G.** *Pathogenesis and therapy of psoriasis,* 2007, 866-873
- **BARKER,J.N.** *The pathophysiology of psoriasis,* 1991, 227-230
- **BUSKE-KIRSCHBAUM,A. ; KERN,S. ; EBRECHT,M. ; HELLHAMMER,D.H.** *Altered distribution of leukocyte subsets and cytokine production in response to acute psychosocial stress in patients with psoriasis vulgaris,* 2007, 92-99
- **VAN BEELEN,A.J. ; TEUNISSEN,M.B.M. ; KAPSENBERG,M.L. ; DE JONG,E.C.** *Interleukin-17 in inflammatory skin disorders,* 2007, 374-381
- **LOWES,M.A. ; KIKUCHI,T. ; FUENTES-DUCULAN,J. ; CARDINALE,I. ; ZABA,L.C. ; HAIDER,A.S. ; BOWMAN,E.P. ; KRUEGER,J.G.** *Psoriasis vulgaris lesions contain discrete populations of Th1 and Th17 T cells,* 2008, 1207-1211
- **TESMER LA ; LUNDY,S. ; SARKAR S ; FOX DA.** *Th17 cells in human disease,* 2008, 87-113
- **MA,H.-L. ; LIANG,S. ; LI,J., NAPIERATA,L. ; BROWN,T. ; BENOIT,S. ; SENICES,M. ; GILL,D. ; DUNUSSI-JOANNOPOULOS,K. ; COLLINS,M. et al.** *IL-22 is required for Th17 cell-mediated pathology in a mouse model of psoriasis-like skin inflammation,* 2008, 597-607
- **NICKOLOFF,B.J. ; QIN,J.Z. ; NESTLE,F.O.** *Immunopathogenesis of psoriasis,* 2007, 45-56
- **TOICHI,E. ; TORRES,G. ; MCCORMICK,T.S. ; CHANG,T. ; MASCELLI,M.A. ; KAUFFMAN,C.L. ; ARIA,N. ; GOTTLIEB,A.B. ; EVERITT,D.E. ; FREDERICK,B. et al.** *An anti-IL-12p40 antibody down-regulates type I cytokines, chemokines, and IL-12/IL-23 in psoriasis,* 2006, 4917-4926
- **LEONARDI CL ; KIMBALL AB ; PAPP KA ; YEILDING N ; GUZZO C ; WANG Y ; LI S ; DOOLEY LT ; GORDON KB.** Efficacy and safety of ustekinumab, a human interleukin-12/23 monoclonal antibody, in patients with psoriasis: 76-week results from a randomized, double-blind, placebo-controlled trial (PHOENIX 1). *PHOENIX 1 study investigators,* 2008, 1665-1674
- **PAPP KA ; LANGLEY RG ; LEBWOHL M ; KRUEGER GG ; SZAPARY P ; YEILDING N ; GUZZO C ; HSU MC ; WANG Y ; LI S et al.** Efficacy and safety of usetekinumab, a human interleukin-12/23 monoclonal antibody, in patients with psoriasis: 52-week results from a randomized, double-blind, placebo-controlled trial. *PHOENIX,* 2008, vol. 2, 1639-1684
- **DINARELLO,C.A.** *IL-32, a novel cytokine with a possible role in disease,* 2007, iii61-iii64

- **KIM,S.H. ; HAN,S.Y. ; AZAM,T. ; YOON,D.Y. ; DIN-ARELLO,C.A.** *Interleukin-32: a cytokine and inducer pf TNF alpha,* 2005, 131-142
- **SHIOYA,M. ; NISHIDA,A. ; YAGI,Y. ; OGAWA,A. ; TSUJIKAWA,T. ; KIM-MITSUYAMA,S. ; TAKAYANAGI,A. ; SHIMIZU,N. ; FUJIYAMA,Y. ; ANDOH,A.** *Epithelial overexpression of interleukin-32 alpha in inflammatory bowel disease,* 2007, 480-486
- **JOOSTEN,L.A. ; NETEA,M.G. ; KIM,S.H. ; YOON,D.Y. ; OPPERS-WALGREEN,B. ; RADSTAKE,T.R. ; BARRERA,P. ; VAN DE LOO,A.A. ; DINARELLO,C.A. ; VAN DEN BERG,W.B.** *IL-32, a proinflammatory cytokine in rheumatoid arthritis,* 2006, 3298-3303
- **KIM,K.H. ; SHIM,J.H. ; SEO,E.H. ; CHO,M.C. ; KANG,J.W. ; KIM,S.H. ; YU,D.Y. ; SONG,E.Y. ; LEE,H.G. ; SOHN,J.H. et al.** *Interleukin-32 monoclonal antibodies for immunohistochemistry, western blotting, and ELISA,* 2008, 38-50
- **NETEA M.G. ; AZAM T. ; FERWERDA G ; GIRARDIN SE ; WALSH M ; PARK J-S ; ABRAHAM E ; KIM J-M ; YOON D-Y ; DINARELLO CA et al.** *IL-32 synergizes with nucleotide oligomerization domain (NOD) 1 and NOD2 ligands for IL-1b and IL-6 production through a caspase 1-dependent mechanism,* 2005, 16309-16314
- **EDWARDS III,C.K. ; VOLK,H. ; SCHIFF,M. ; SCHIFF,M.H. ; KOTZIN,B. ; MITSUYA,H. ; KAWAGUCHI,T. ; SAKATA,K.M. ; CHERONIS,J. ; TROLLINGER,D. et al.** *Anti-tumor necrosis factor-a therapy in rheumatoid arthritis patients reduces whole blood gene expression compared to DMARD therapy alone,* 2008
- **RASOOL ST ; TANG H ; WU J ; LI WEI ; MUKHTAR MM ; ZHANG J ; MU Y ; XING HX ; WU J ; ZHU Y.** *Increased level of IL-32 during human immunodeficiency virus infection suppresses HIV replication,* 2008, 161-167
- **KO,NY. ; CHANG,SH. ; LEE,J. ; KIM,N. ; KIM,Y. ; CHOI,W. ; CHOI,J. ; BAE,S. ; HONG,J. ; JAEKAL,J. et al.** *Unique expression of a small IL-32 protein in the Jurkat leukemic T cell line,* 2008, 121-127
- **EDWARDS III,C.K. ; BENDELE,A.M. ; REZNIKOV,L. ; FANTUZZI,G. ; CHLIPALA,E.S. ; LI L ; MOLDAWER,L.L. ; MOUNTZ,J.D. ; LI,Y. ; DINARELLO CA.** *Soluble human p55 and p75 tumor necrosis factor receptors reverse spontaneous arthritis in transgenic mice expressing transmembrane tumor necrosis factor a,* 2006, 2872-2885
- **BENDELE,A.M. ; CHLIPALA,E.S. ; SCHERRER,J. ; FRAZIER,J. ; SENNELLO,G. ; RICH,W.J. ; EDWARDS,I.C.** Combination benefit of treatment with the cytokine inhibitors interleukin-1 receptor antagonist and PEGylated soluble tumor necrosis factor receptor type I in animal models of rheumatoid arthritis. *Arthritis-Rheum.,* 2000, vol. 43, 2648-2659
- **HYKA,N. ; DAYER,J.M. ; MODOUX,C. ; KOHNO,T. ; EDWARDS-III,C.K. ; ROUX,L.P. ; BURGER, D.** Apolipoprotein A-I inhibits the production of interleukin-1 beta and tumor necrosis factor-alpha by blocking contact-mediated activation of monocytes by T lymphocytes. *Blood,* 2001, vol. 97, 2381-2389
- **LI L ; CALLAWAY,E. ; LIU,B. ; FITZPATRICK,D. ; HU,L.-J. ; DAYER,J.-M. ; NORRIS,D.A. ; EDWARDS III,C.K.** Dissociation of human T-cell mediated macrophage activation to produce proinflammatory cytokines by CD40 ligand (CD154). *IFN-g, or novel T-cell cytokine inducing surface molecules (TCISMs), in a cell-cell contact manner,* 2008
- **KUPPER,T.S. ; FUHLBRIGGE,R.C.** Immune surveillance in the skin: mechanisms and clinical consequences. *Nat. Rev. Immunol.,* 2004, vol. 4, 211-222
- **CONRAD,C. ; BOYMAN,O. ; TONEL,G. ; TUN-KYI,A. ; LAGGNER,U. ; DE FOUGERROLLES,A. ; KOTELIANSKI,V. ; GARDNER,H. ; NESTLE,F.O.** *a1b1 integrin is crucial for accumulation of epidermal T cells and the development of psoriasis,* 2007, 836-842
- **ELLIS,C.N. ; KRUEGER,G.G.** Treatment of chronic plaque psoriasis by selective targeting of memory effector T lymphocytes. *N. Engl J Med.,* 2001, vol. 345, 248-255
- **TAKADA,K. ; DANNING,C.L. ; KUROIWA,T. ; SCHLIMGEN,R. ; TASSIULAS,I.O. ; DAVIS,J.C., JR. ; YARBORO,C.H. ; FLEISHER,T.A. ; BOUMPAS,D.T. ; ILLEI,G.G.** *Lymphocyte depletion with fludarabine in patients with psoriatic arthritis: clinical and immunological effects,* 2003, 1112-1115
- **ROTTMAN,J.B. ; SMITH,T.L. ; GANLEY,K.G. ; KIKUCHI,T. ; KRUEGER,J.G.** the integrin alpha-e b-7in the pathogenesis of psoriasis vulgaris. *Potential role of the chemokine receptors CXCR3, CCR4,* 2001, 335-337
- **TERAKI,Y. ; MIYAKE,A. ; TAKLEBAYASHI,R. ; SHIOHARA,T.** *Homing receptor and chemokine receptor on intraepidermal T cells in psoriasis vulgaris,* 2004, 658-663
- **KUPPER,T.S.** Immunologic targets in psoriasis. *N. Engl J Med.,* 2003, vol. 349, 1987-1990
- **BERTH-JONES,J.** *The use of cyclosporin in psoriasis,* 2005, 257-260

- **KRUEGER,J.G. ; WOLFE,J.T. ; NABEYA,R.T. ; VALLAT,V.P. ; GILLEAUDEAU,P. ; HEFTLER,N.S. ; AUSTIN,L.M. ; GOTTLIEB,A.B.** *Successful ultraviolet B treatment of psoriasis is accompanied by a reversal of keratinocyte pathology and by selective depletion of intraepidermal T cells,* 1995, 2057-2068

- **GOTTLIEB,S.L. ; GILLEAUDEAU,P. ; JOHNSON,R. ; ESTES,L. ; WOODWORTH,T.G. ; GOTTLIEB,A.B. ; KRUEGER,J.G.** Response of psoriasis to a lymphocyte-selective toxin (DAB389IL-2) suggests a primary immune, but not keratinocyte, pathogenic basis. *Nat. Med.,* 1995, vol. 1, 442-447

- **ABRAMS,J.R. ; KELLEY,S.L. ; HAYES,E. ; KIKUCHI,T. ; BROWN,M.J. ; KANG,S. ; LEBWOHL,M.G. ; GUZZO,C.A. ; JEGASOTHY,B.V. ; LINSLEY,P.S. et al.** Blockade of T lymphocyte costimulation with cytotoxic T lymphocyte-associated antigen 4-immunoglobulin (CTLA4Ig) reverses the cellular pathology of psoriatic plaques, including the activation of keratinocytes, dendritic cells, and endothelial cells. *J Exp. Med.,* 2000, vol. 192, 681-694

- **GORDON,K.B. ; PAPP,K.A. ; HAMILTON,T.K. ; WALICKE,P.A. ; DUMMER,W., LI,N. ; BRESNAHNA,B.W. ; MENTER,A.** *Efalizumab for patients with moderate to severe plaque psoriasis: a randomized controlled trial,* 2003, 3073-3080

- **NICKOLOFF,B.J. ; WRONE-SMITH,T. ; BONISH,B.K. ; PORCELLI,S.A.** *Response of murine and normal human skin to injection of allogeneic blood-derived psoriatic immunocytes,* 1999, 546-552

- **NICKOLOFF,B.J. ; WRONE-SMITH,T.** *Injection of pre-psoriatic skin with CD4+ T cells induces psoriasis,* 1999, 145-158

- **PRINZ,J.C. ; GROB,B. et al.** *T cell clones from psoriasis skin lesions can promote keratinocyte proliferation in vitro via secreted products,* 1994, 593-598

- **VOLLMER,S. ; MENSSEN,A. ; TROMMLER,P. ; SCHENDEL,D. ; PRINZ,J.C.** *T-lymphocytes derived from skin lesiona of patients with psoriasis vulgaris express a novel cytokine pattern that is distinct from that of T helper type I and T helper type 2 cells,* 1994, 2377-2382

- **BATA-CSORGO,Z. ; HAMMERBERG,C. ; VOORHEES,J.J. ; COOPER,K.D.** Kinetics and regulation of human keratinocyte stem cell growth factors in short-term primary ex vivo culture. *Cooperative growth factors from psoriatic lesional T lymphocytes stimulate proliferation among psoriatic uninvolved, but not normal stem keratinocytes,* 1995, 317-327

- **LEBWOHL,M.** Psoriasis. *The Lancet,* 2003, vol. 361, 1197-1204

- **BAIER,G. ; ASADULLAH,K.A. ; ZUGEL,U.** *The immunological synapse: Kinases in T cell signalling as potential drug targets,* 2006, 3-5

- **OUYANG,W. ; KOLLS,J.K. ; ZHENG,Y.** *The biological functions of T helper 17 cell effector cytokines in inflammation,* 2008, 454-467

- **DONG,C.** *Th17 cells in development: an updated view of their molecular identity and genetic programming,* 2008, 337-348

- **MCGEACHY,M.J. ; BAK-JENSEN,K.S. ; CHEN,Y. ; TATO,C.M. ; BLUMENSCHEIN,W. ; MCCLANAHAN,T. ; CUA,D.J.** *TGF-b and IL-6 drive the production of IL-17 and IL-10 by T cells and restrain Th17 cell-mediated pathology,* 2007, 1390-1397

- **CHEN,Z. ; O'SHEA,J.J.** *Regulation of IL-17 production in human lymphocytes,* 2008, 71-78

- **CHEN,Z. ; TATO,C.M. ; MUUL,L. ; LAURENCE,A. ; O'SHEA,J.J.** *Distinct regualtion of interleukin-17 in human T helper lymphocytes,* 2007, 2936-2946

- **NETEA,M.G. ; LEWIS,M. ; AZAM,T. ; JOOSTEN,L.A. ; JACKAL,J. ; BAE,S.Y. ; DINARELLO CA ; KIM,S.-H.** *Interleukin-32 induces the differentiation of monocytes into macrophage-like cells,* 2008, 3515-3520

- **FRISHMAN,J.I. ; EDWARDS III,C.K. ; SONNENBERG,M.G. ; KOHNO,T. ; COHEN,A.M. ; DINARELLO,C.A.** Tumor necrosis factor (TNF)-alpha-induced interleukin-8 in human blood cultures discriminates neutralization by the p55 and p75 TNF soluble receptors. *J-Infect-Dis.,* 2000, vol. 182, 1722-1730

- **MUGLER,K.C. ; SINGH,M. ; TRINGLER,B. ; TORKKO,K.C. ; LIU,W. ; PAPKOFF,J. ; SHROYER,K.R.** *B7-h4 expression in a range of breast pathology: correlation with tumor T-cell infiltration,* 2007, 363-370

- **SWICK;B.L. ; RAVDEL,L. ; FITZPATRICK,J.E. ; ROBINSON,W.A.** *Platelet-derived growth factor receptor alpha mutational status and immunohistochemical expression in Merkel cell carcinoma: implications for treatment with imatinib mesylate,* 2008, 197-202

- **HERRMANN et al.** Construction of optimized bispecific antibodies for selective activation of the death receptor CD95. *Cancer Research,* 2008, vol. 68 (4), 1221-128

- **MANIKANDAN J ; PUSHPARAJ PN ; MELENDEZ AJ.** Protein i: interference at protein level by intrabodies. *Front Biosci.,* 2007, vol. 1 (12), 1344-52